# EUROPEAN PATENT SPECIFICATION

(11) **EP 2 089 029 B1**
(45) Date of publication and mention of the grant of the patent: **03.10.2012**
(21) Application number: 07871432.6
(22) Date of filing: 09.11.2007
(51) Int. Cl.: A61K 31/713, A61K 31/454, A61K 31/395, A61K 39/395, A61K 38/55, A61P 25/00

(54) **PAK INHIBITORS FOR USE IN TREATING NEURODEVELOPMENTAL DISORDERS**
PAK INHIBITOREN ZUR BEHANDLUNG VON NEUROLOGISCHEN ENTWICKLUNGSSTOERUNGEN
INHIBITEURS DE PAK POUR LE TRAITEMENT DES TROUBLES NEURODEVELOPEMENTAUX

(30) Priority: 10.11.2006 US 858108 P
(43) Date of publication of application: 19.08.2009
(60) Divisional application: 11185523.5
(73) Proprietor: Massachusetts Institute of Technology, Cambridge, MA 02139-4307 (US)
(72) Inventor: TONEGAWA, Susumu, Chestnut Hill, MA 02467 (US); HAYASHI, Mansuo, Winchester, MA 01890 (US); DOLAN, Bridget, Boston, MA 02114 (US)
(74) Representative: Hollywood, Jane Constance
(86) International application number: PCT/US2007/084325
(87) International publication number: WO 2008/063933

(56) References cited:
- WO-A-2004/083376
- WO-A-2005/095976
- US-B1- 6 451 837
- DATABASE WPI Week 200163 Thomson Scientific, London, GB; AN 2001-558197 XP002497531 & CN 1 302 610 A (null) 11 July 2001 (2001-07-11)

## Description

### Government Support

### Background of the Invention

Fragile X syndrome (FXS) is the most commonly inherited form of mental retardation, with symptoms of hyperactivity, stereotypy, anxiety, seizure, impaired social behavior, and/or cognitive delay. FXS results from the loss of expression of the *fragile X mental retardation 1 (FMRI)* gene, which encodes the fragile X mental retardation protein (FMRP). *FMR1* knockout *(FMR1* KO) mice and FXS patients show similar behavioral phenotypes, as well as similar abnormalities in synaptic morphology in the brain (O'Donnell et al., 2002, Annu. Rev. Neurosci., 25:315). Their brains have more dendritic spines and/or a higher proportion of longer and/or thinner spines compared to normal individuals (Hinton et al., 1991, Am. J. Med. Genet., 41:289; Comery et al., 1997, Proc. Natl. Acad Sci., USA, 94:5401; and Irwin et al., 2001, Am. J. Med. Genet., 98:161). Dendritic spines are the protrusions from dendritic shafts that serve as postsynaptic sites of the majority of excitatory synapses in mammals. Correlated with altered spine morphology, *FMR1* KO mice display abnormal synaptic function, including enhanced long-term depression (LTD) mediated by metabotropic glutamate receptor in the hippocampus and/or impaired long-term potentiation (LTP) in the cortex, compared to wild type mice (Huber et al., 2002, Neuropharmacology, 37:571; and Li et al., 2002, Mol. Cell Neurosci., 19:138*).*
Thus, these findings demonstrate that FMRP functions in regulating spine morphology, synaptic function, and/or animal behavior.

FMRP is a selective RNA-binding protein that associates with polyribosomes *(*Corbin et al., 1997, Hum. Mol. Genet., 6:1465; and Stefani et al., 2004, J. Neurosci., 24:7272) and with Argonaute2 (AGO2) and Dicer, two members of RISC, a complex that is required for RNAi-mediated gene silencing (Ishizuka et al., 2002, Genes Dev., 16:2497).
FMRP is thought to regulate synaptic morphology and/or function because of its ability to repress translation of its RNA binding partners (Laggerbauer et al., 2001, Hum. Mol. Genet., 10:329; Li et al., 2001, Nucleic Acids Res., 29:2276; and Mazroui et al., 2002, Hum. Mol. Genet., 11:3007), perhaps using an RNAi-mediated mechanism. Some of these RNAs encode proteins that are involved in synaptic morphology and/or function, such as Racl, microtubule-associated protein 1B, activity-regulated cytoskeleton-associaied protein, and alpha-calcium/calmodulin-dependent protein kinase II *(*Zhang et al., 2001, Cell, 107:591; Lee et al., 2003, Development, 130:5543; and Zalfa et al., 2003, Cell, 112:317)*.*

### Summary of the Invention

The invention is as set out in the claims.

The present invention encompasses the recognition that signaling pathways mediated by p21 -activated kinase (PAK) and FMRP may antagonize each other to regulate synaptic morphology and/or function. The present invention encompasses the recognition that abnormalities in cortical spine morphology of FXS patients and FNM1 knockout mice are opposite to those we found in transgenic mice in which PAK activity is inhibited by its dominant negative form (dnPAK). The present invention encompasses the recognition that FMRP binds to PAK.

The present invention provides inhibitors of PAK for use in treating fragile X syndrome (FXS) and/or other neurodevelopmental disorders.

Compositions comprising at least one PAK inhibitor and a pharmaceutically acceptable excipient are included. In certain embodiments, PAK modulators function by modulating the interaction between PAK and the fragile X mental retardation protein (FMRP).

The present invention provides methods for identifying PAK inhibitors useful for treating neurodevelopmental disorders. In one instance, such methods include high throughput screening methods. In another instance the methods include *in vitro, in cyto* assats.

The present invention provides specific PAK inhibitors. In some embodiments, specific PAK modulators include small molecule therapeutics. In some embodiments, small molecule modulators of PAK include Emodin, OSU-03012 and/or combinations thereof. The present invention includes pharmaceutical compositions comprising Emodin, OSU-03012 and/or combinations thereof. The present invention provides Emodin, OSU-03012 and/or combinations thereof for use in treating FXS and/or other neurodevelopmental disorder.

### Brief Description of the Drawing

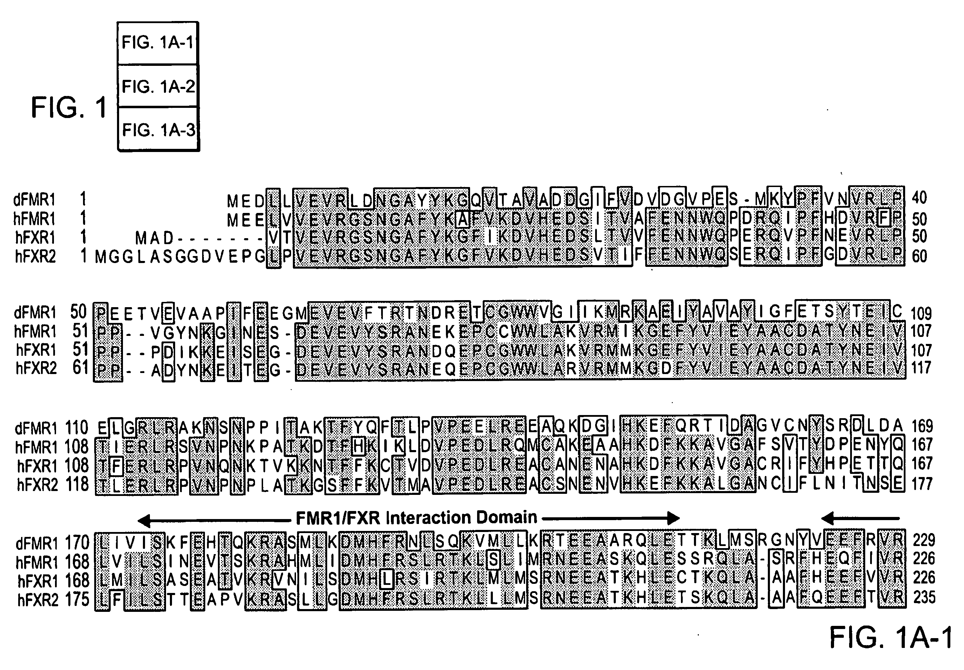

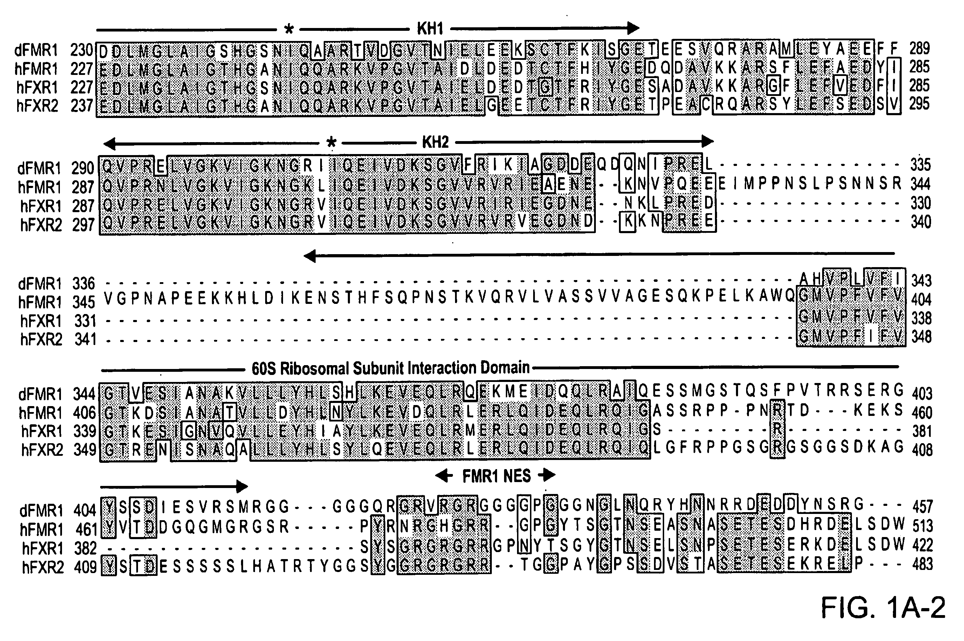

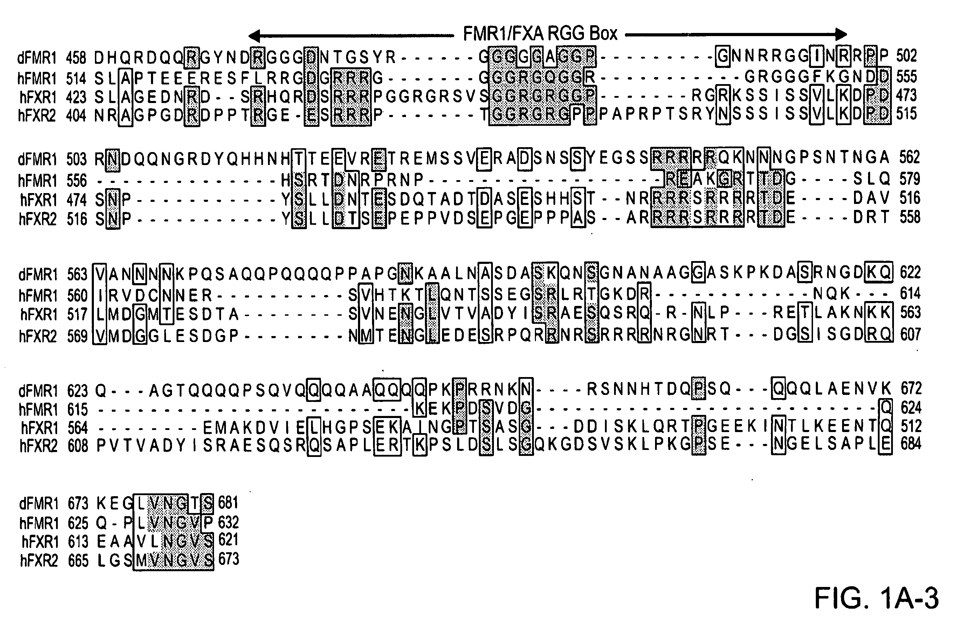

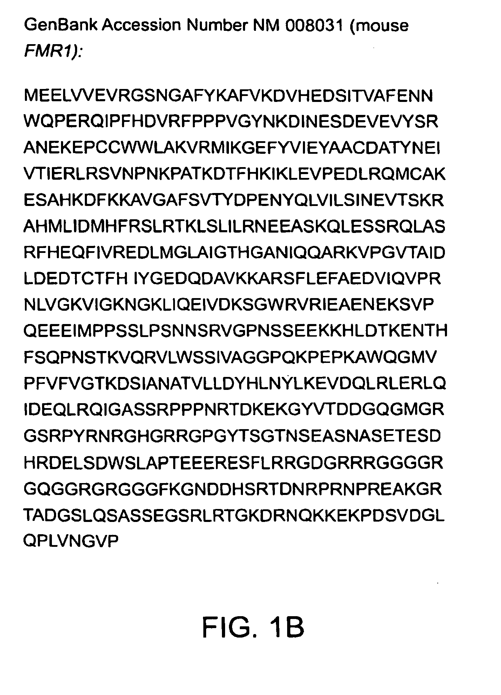

*Figure 1**. Representative FMR and FXR amino acid sequences.* Figure IA shows an alignment of representative examples of *Drosophila melanogaster* FMR1 (dFMR1; GenBank AAG22045), human FMR1 (hFMR1; GenBank AAB18829), human FXR1 (hFXR1; GenBank AAC50155), and human FXR2 (hFXR2; GenBank AAC50292) amino acid sequences. Dark gray boxes mark identical amino acids, and light gray boxes denote conservative amino acid substitutions. Insertions are denoted by a dash. The FMR1/FXR interaction domain is depicted. "KH1" and "KH2": KH domains; "NES": nuclear export signal; "RGG": domain that mediates protein-protein interactions, RNA- binding, and/or may be methylated. Figure modified from Wan et al., 2000, Mol. Cell. Biol., 20:8536. Figure IB shows an amino acid sequence for mouse *FMRl* (GenBank NM_008031).

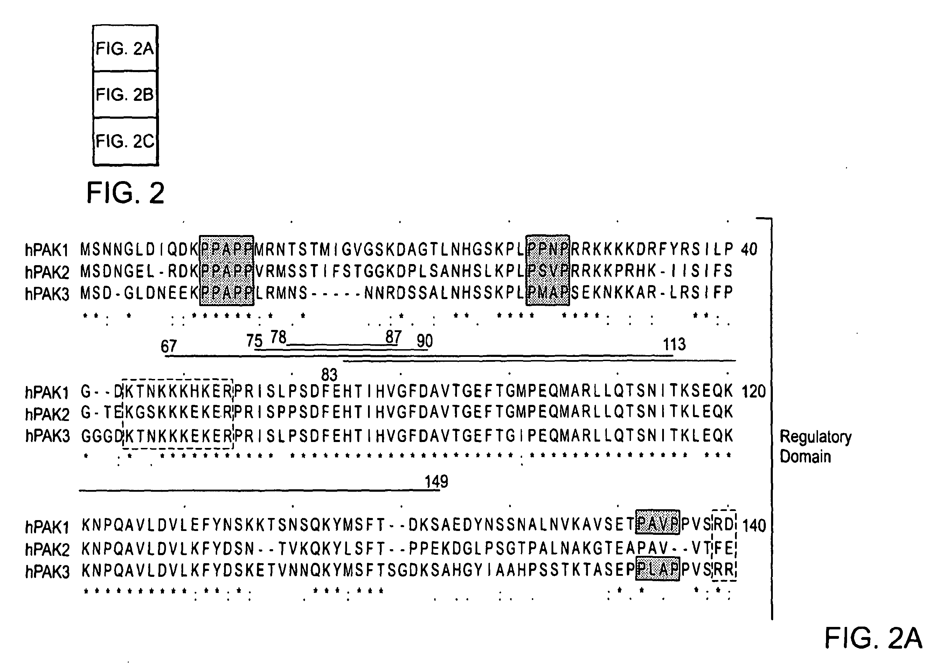

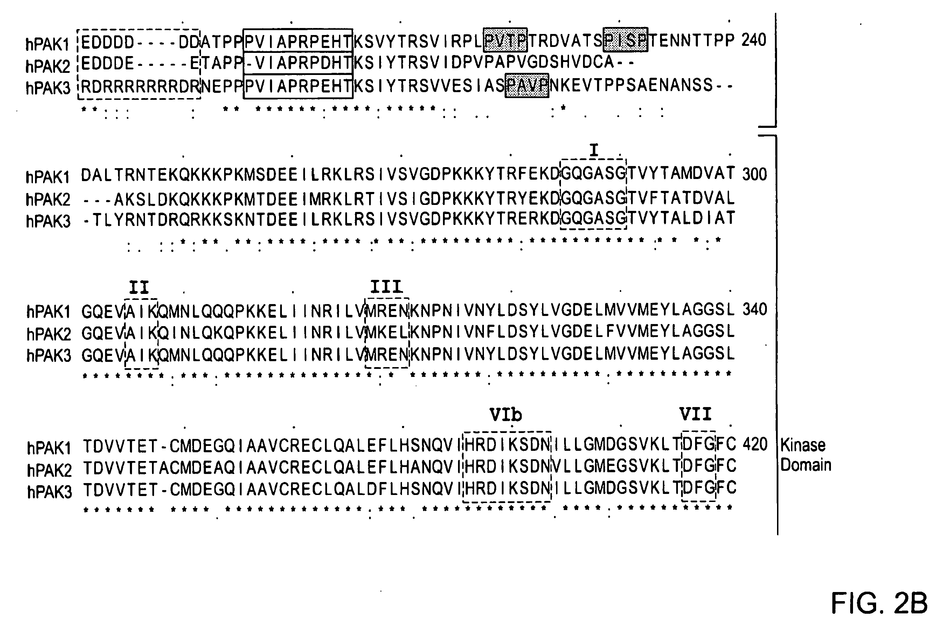

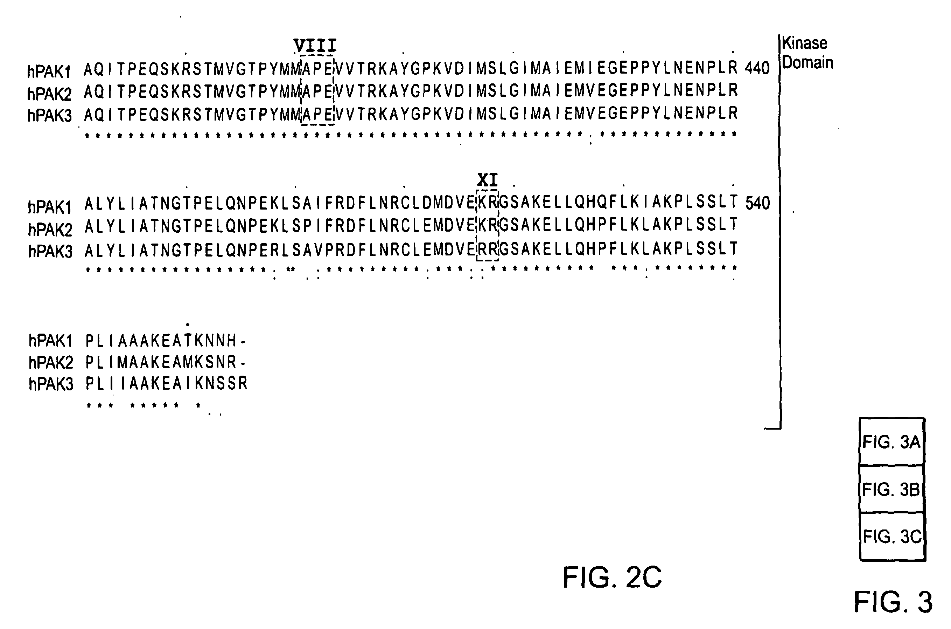

*Figure 2**. Representative human PAK1, PAK2, and PAK3 amino acid sequences.* Shown is an alignment of representative examples of human PAK 1 (GenBank AAA65441), human PAK2 (GenBank AAA65442), and human PAK3 (GenBank AAC36097) amino acid sequences. Asterisks below the sequences mark identical amino acids, and dots below the sequences denote conservative amino acid substitutions. Insertions are denoted by a dash. Black boxes mark proline-rich regions containing putative SH3-binding PXXP motifs. The open box marks the noncanonical P1X binding site. Gray boxes indicate highly charged basic or acidic tracts. The dark overhead lines indicate the homodimerization domain (amino acids 78 - 87), CRIB motif (amino acids 75 - 90), p21-binding domain (PBD; amino acids 67 - 113), and autoinhibitory switch domain (amino acids 83 - 139) for PAK1. Diagnostic kinase motifs in the catalytic domain are boxed and numbered per convention. Figure modified from Bokoch et al., 2003, Annu. Rev. Biochem., 72:743.

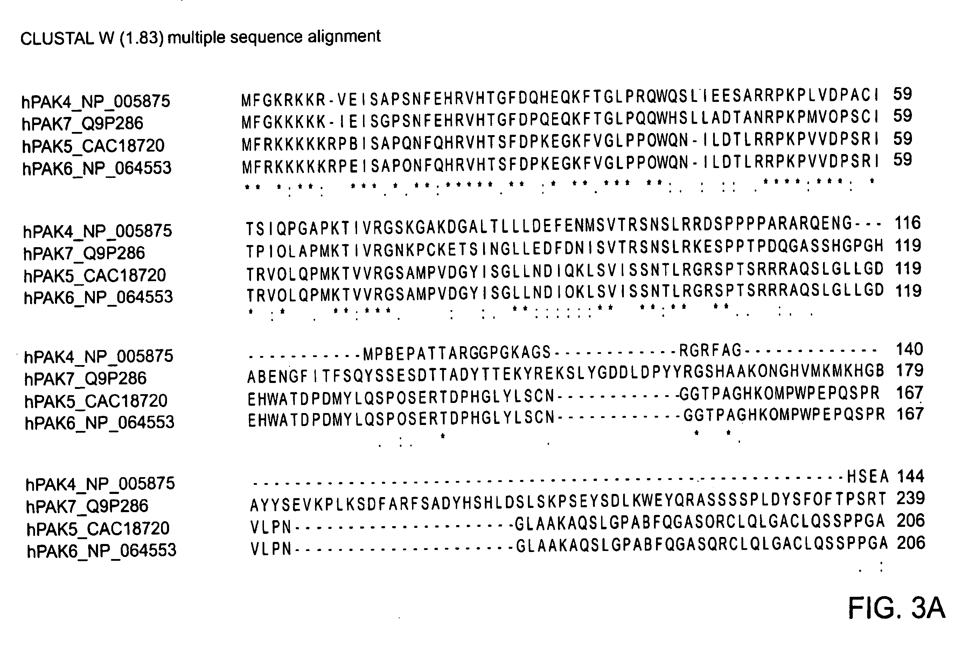

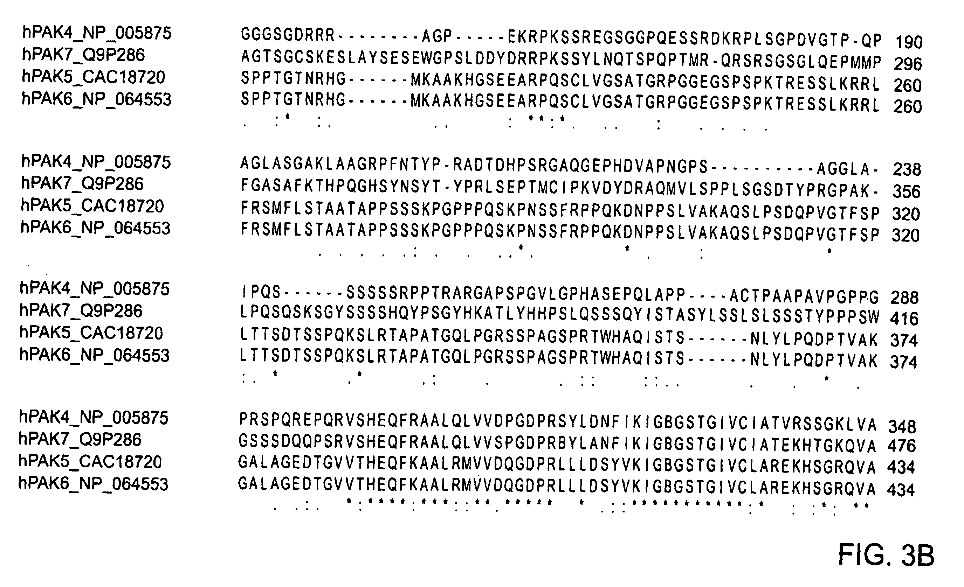

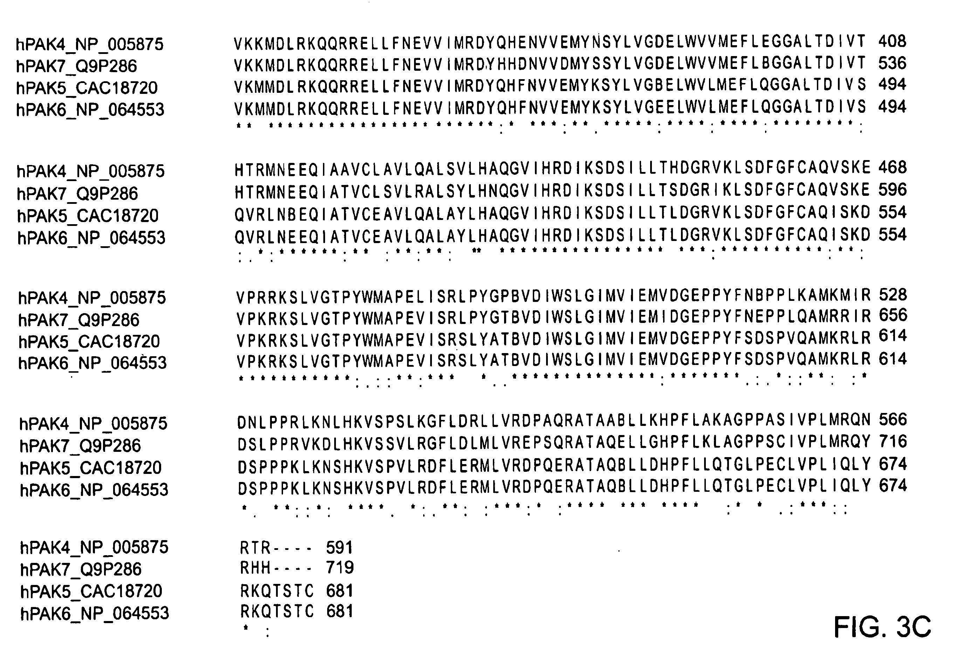

*Figure 3**. Representative human PAK4, PAK5, PAK6, and PAK7 amino acid sequences.* Shown is an alignment of representative examples of human PAK4 (GenBank NP_005875), human PAK5 (GenBank CAC 18720), human PAK6 (GenBank NP_064553), and human PAK7 (GenBank Q9P286) amino acid sequences. Asterisks below the sequences mark identical amino acids, and dots below the sequences denote conservative amino acid substitutions. Insertions are denoted by a dash.

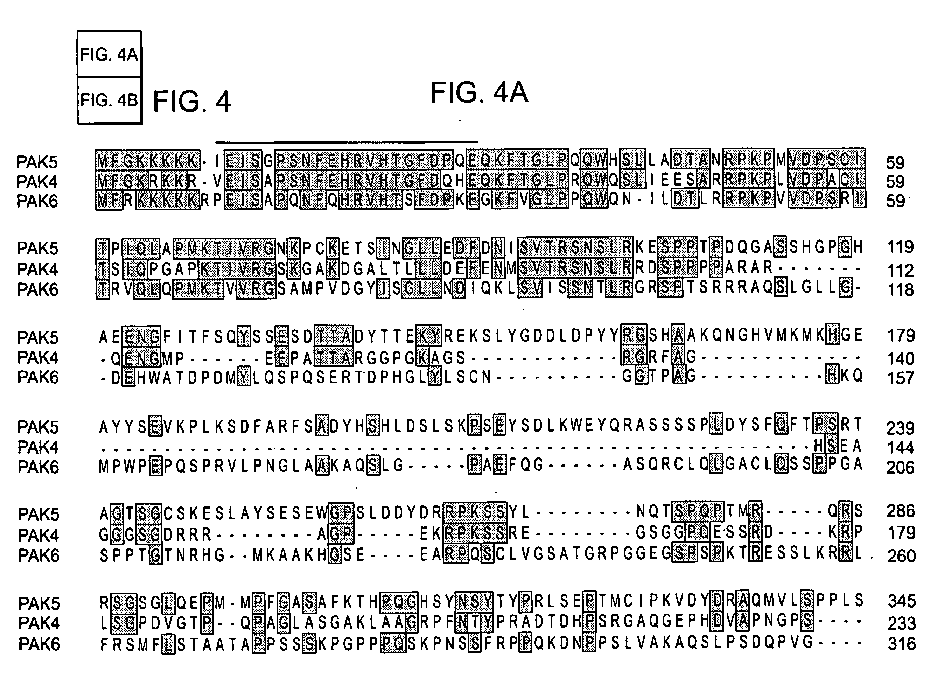

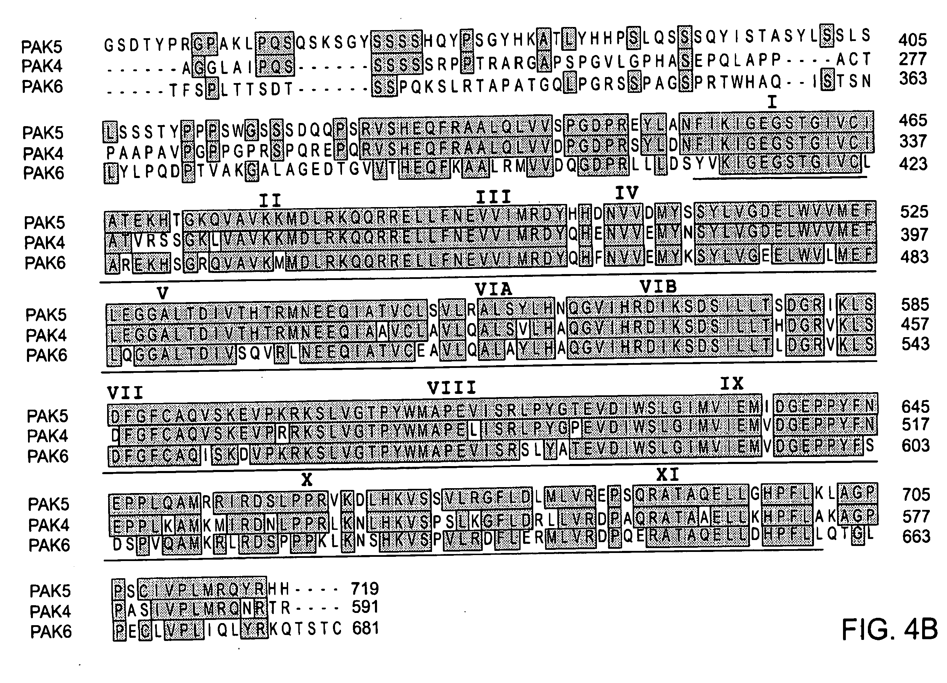

*Figure 4**. Representative human PAK4, PAK5, and PAK6 amino acid sequences.* Shown is an alignment of representative examples of human PAK4 (GenBank CAA09820), human PAK5 (GenBank BAA94194), and human PAK6 (GenBank AAF82800) amino acid sequences. Black boxes mark identical amino acids. Insertions are denoted by a dash. Diagnostic kinase motifs in the catalytic domain are boxed and numbered per convention. Figure modified from Dan et al., 2002, Mol. Cell. Biol., 22:567.

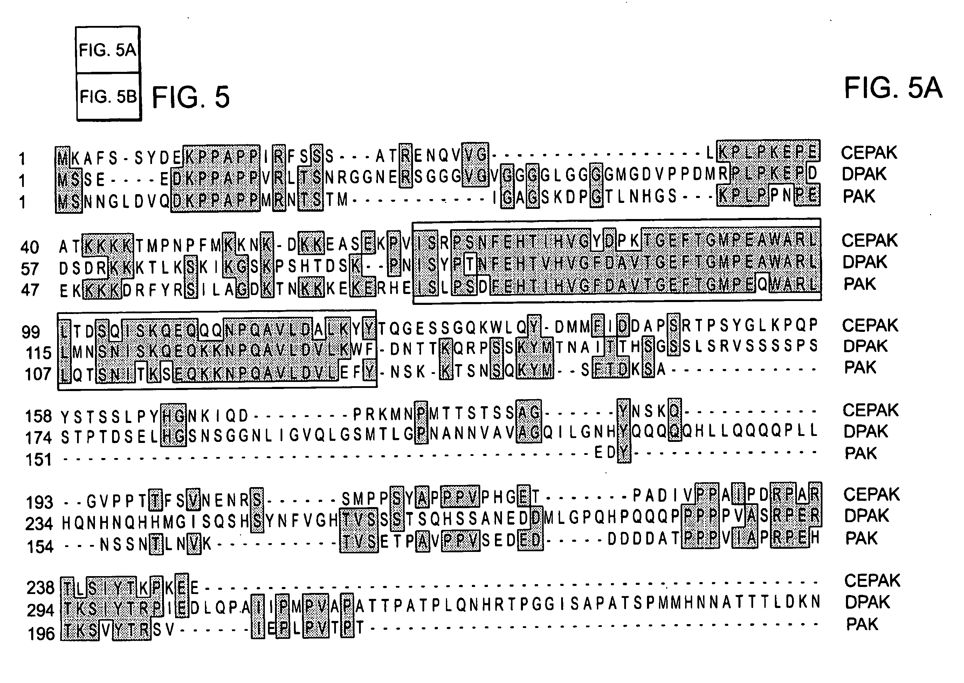

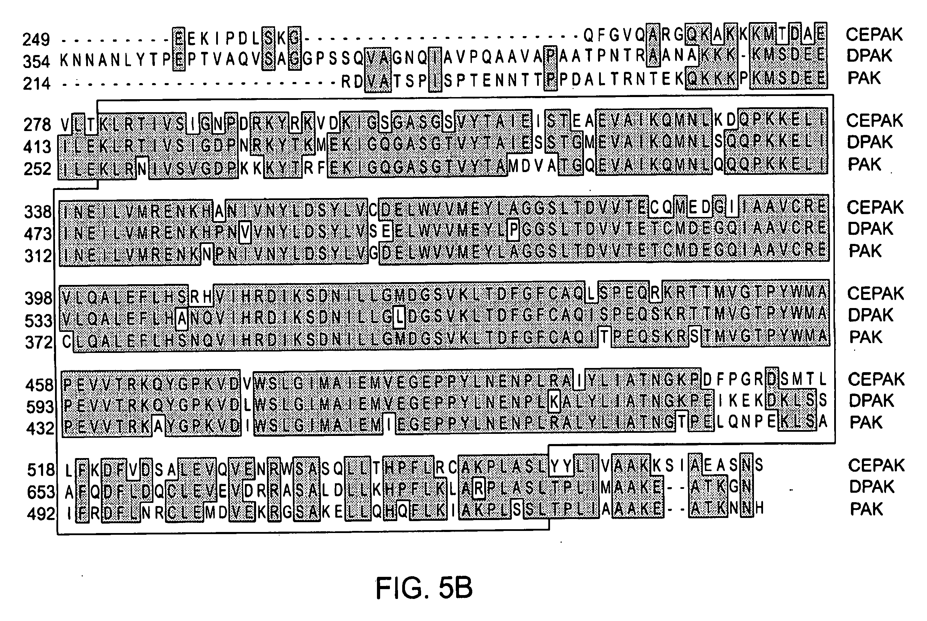

*Figure 5**. Representative* Caenorhabditis elegans, Drosophila melanogaster, *and rat PAK1 amino acid sequences.* Shown is an alignment of representative examples of PAK1 amino acid sequences from *C*. *elegans* (CEPAK; GenBank BAA11844), *D. melanogaster,* (DPAK; GenBank AAC47094), and rat (PAK; GenBank AAB95646). Black boxes mark identical amino acids. Insertions are denoted by a dash. The N-terminal box marks the p21-binding domain, and the C-terminal box denotes the C-terminal kinase domain. Figure modified from Chen et al., 1996, J. Biol. Chem., 271:26362,

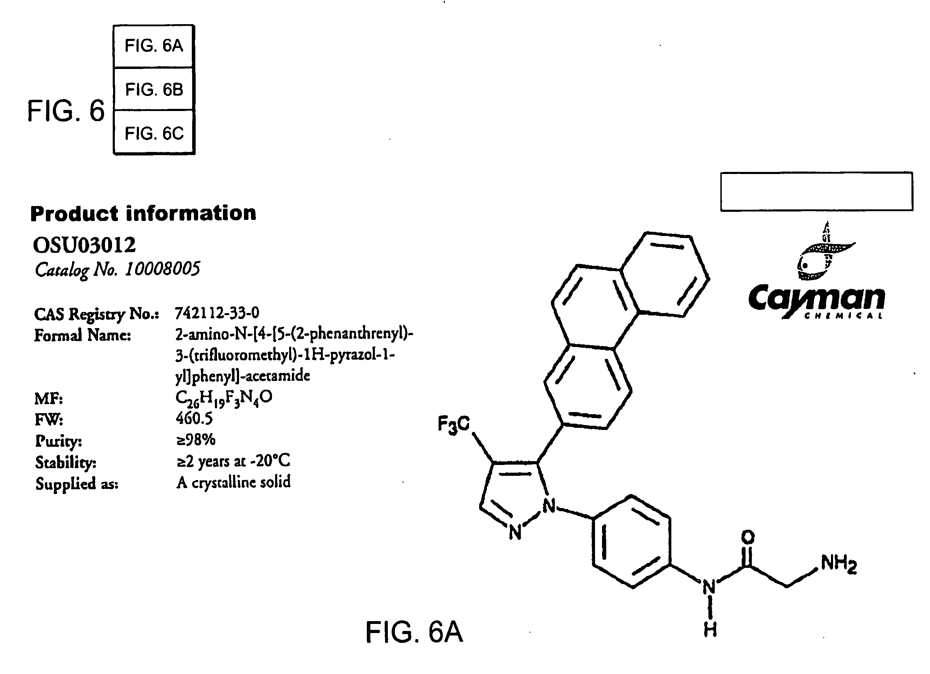

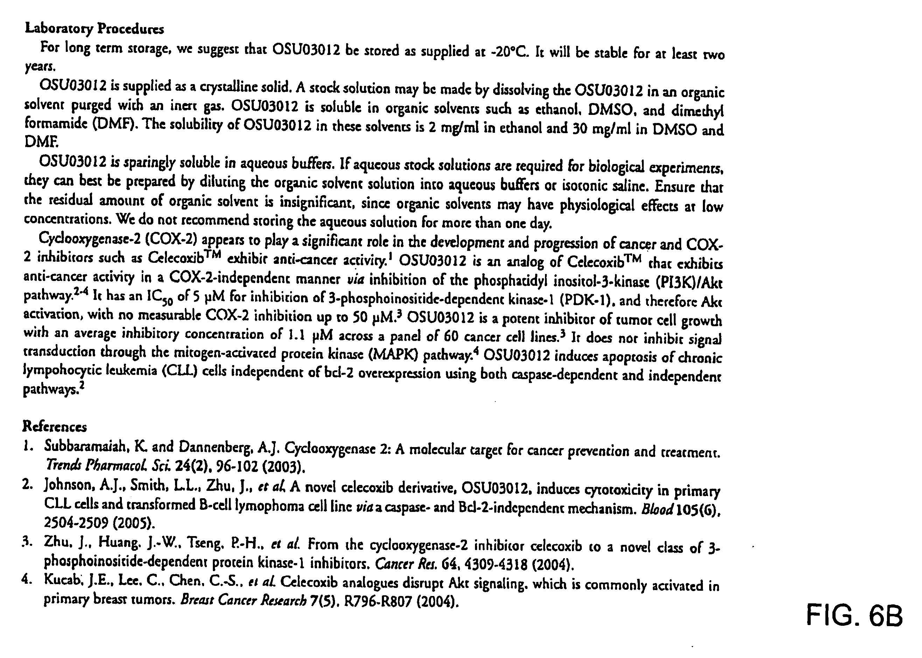

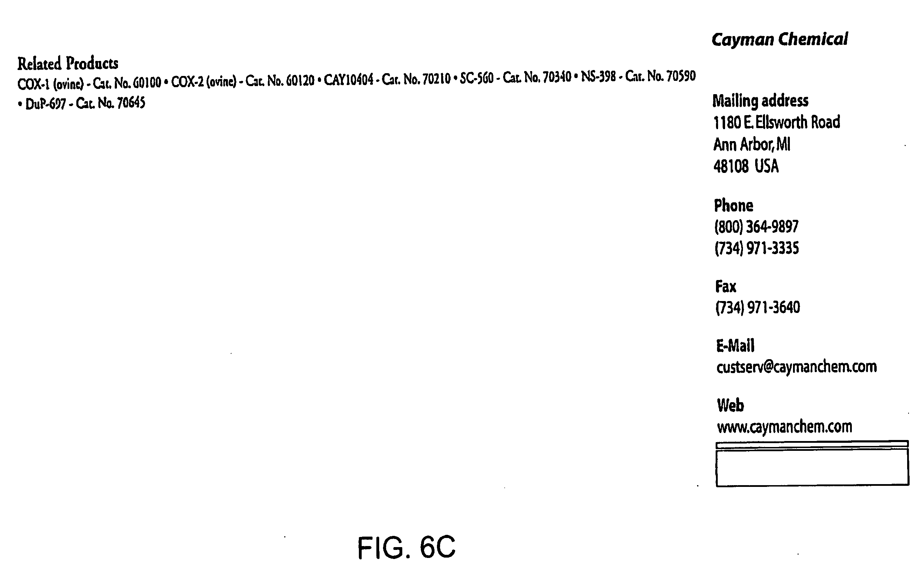

*Figure 6**.* OSU-03012 product information from Cayman Chemical catalog (May 2006).

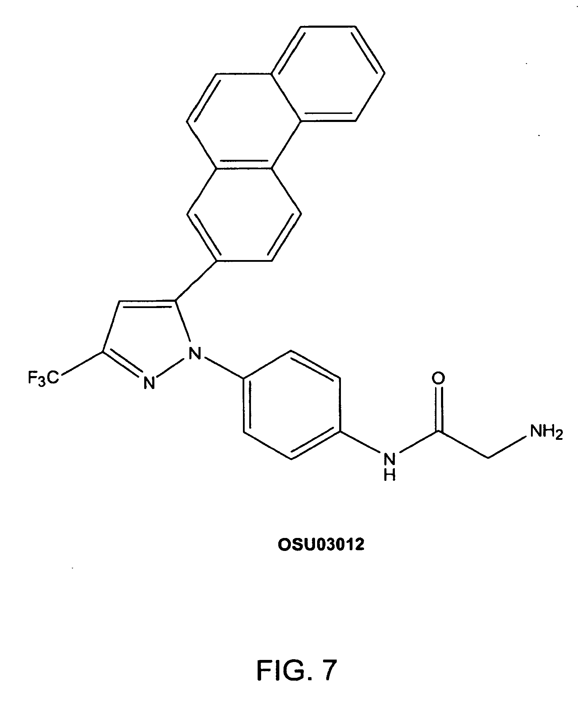

*Figure 7*. Chemical structure of OSU-03012.

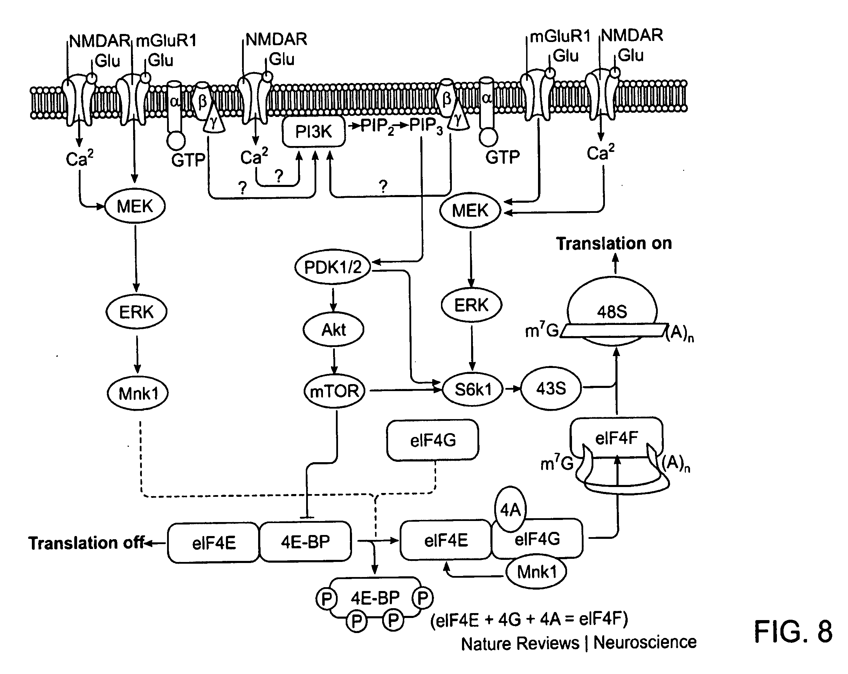

*Figure 8**. PAK is involved in multiple signaling pathways.* PAK participates upstream of Raf, mitogen-activated protein kinase kinase (MEK), extracellular signal-regulated kinase (ERK), and MAPK-interacting serine/threonine kinase 1 (Mnk1) in the ERK signaling pathway. PAK participates downstream of phosphoinositide-dependent kinase 1 (PDK1), PDK2, and phosphatidylinositol 3-kinase (PI3K) in the PI3K signaling pathway. Figure modified from Klann and Dever (2004, Nat. Rev. Neurosci., 5:931).

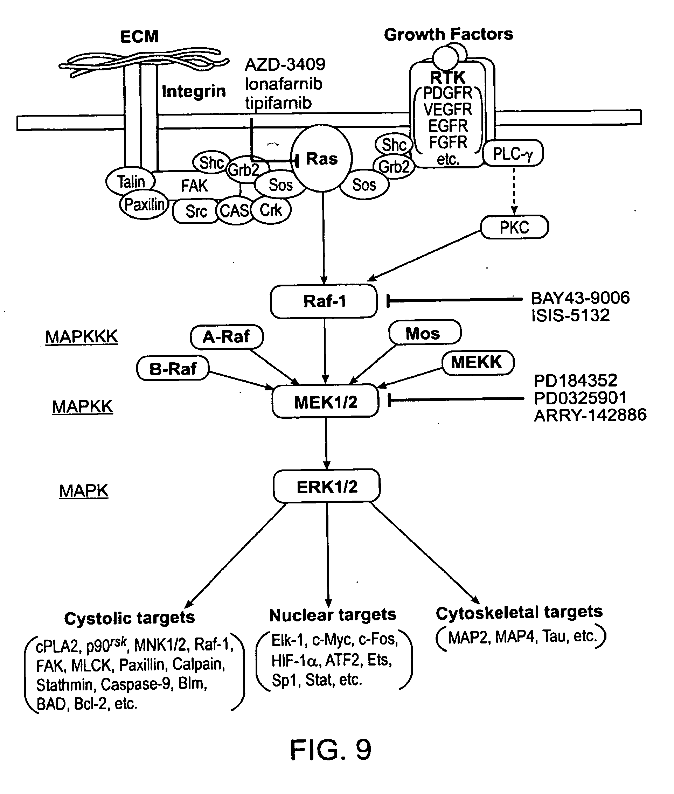

*Figure 9**. Schematic representation of the extracellular signal-regulated kinase (ERK) pathway.* The mitogen-activated protein (MAP) kinase cascade comprises three sequential kinases: MAP kinase kinase kinase (MAPKKK), MAP kinase kinase (MAPKK), and MAP kinase (MAPK). ERK1/2 phosphorylate a variety of nuclear, cytosolic and cytoskeletal targets. Integrin-focal adhesion kinase (FAK) pathway, which is activated by adhesion of integrins to specific extracellular matrix (ECM) molecules, is involved in activating the ERK pathway. Activation of the ERK pathway is often associated with cell proliferation, cell survival and cell migration. Exemplary inhibitors of the ERK pathway are shown. Several negative regulators of the ERK pathway exist, such as MAP kinase phosphatases (MKPs/DUSPs) and Sprouty proteins. Expression of MKPs and Sprouty proteins is induced in an ERK-dependent manner, and thus these proteins participate in the negative feedback regulatory loop of the ERK pathway.

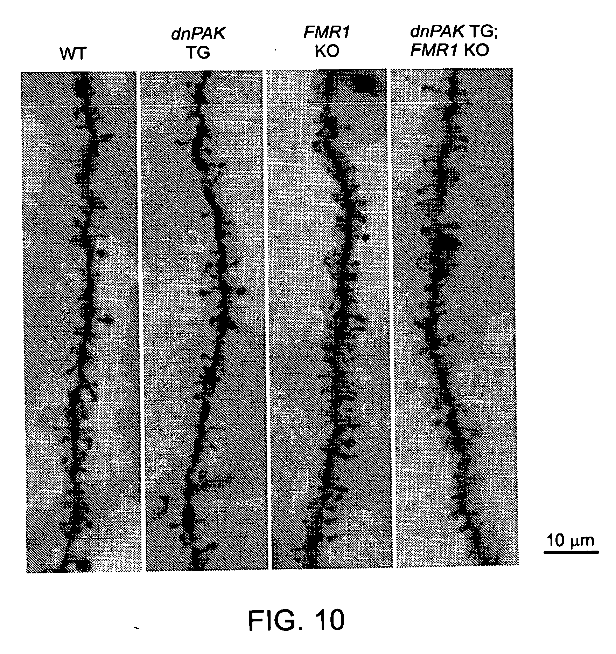

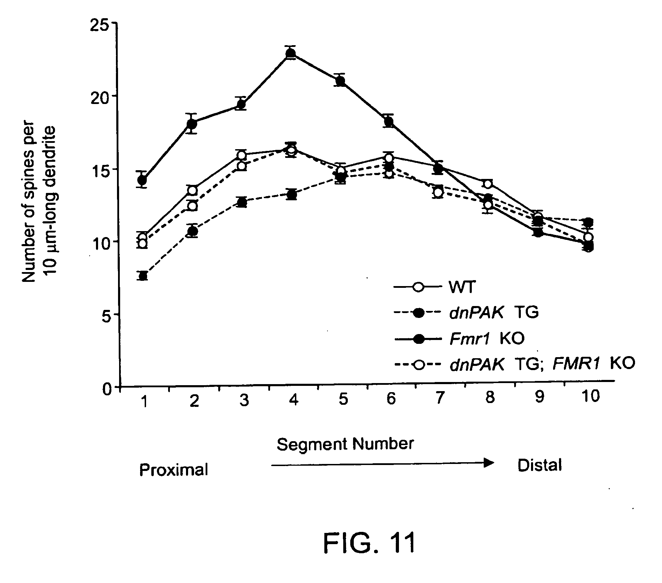

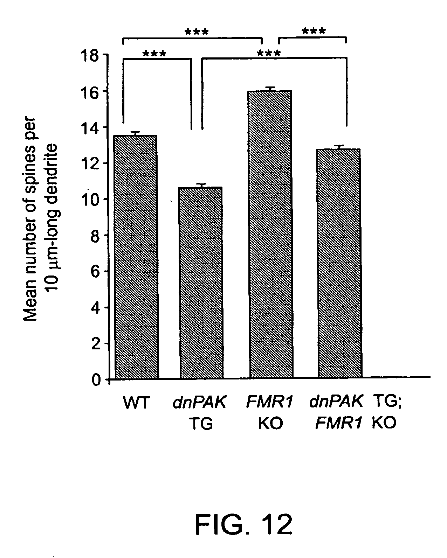

*Figure 10**. Golgi analysis.* Representative dendritic segments of layer II/)III pyramidal neurons from wild-type (WT; n = 20 neurons, 2 mice), *dnPAK* TG mice (n = 30 neurons, 3 mice), *FMR1* KO mice (n = 20 neurons, 2 mice), and double mutant *dnPAK TG; FMR1* KO mice (dMT; n = 40 neurons, 4 mice). On each primary apical dendritic branch, ten consecutive 10 µm-long dendritic segments were analyzed to quantify spine density per 10 µm-long dendritic segment (Figure 11) and mean spine density (Figure 12).

*Figure 11**. Quantification of spine density.* On each primary apical dendritic branch, ten consecutive 10 µm-long dendritic segments were analyzed to quantify spine density per 10 µm-long dendritic segment. Spine density in dMTs was comparable to wild-type controls in all dendritic segments except segment 7 and 8 (p > 0.05 in segments 1 - 6, 9, and 10; *p* < 0.01 in segments 7 and 8).

*Figure 12**. Quantification of mean spine density.* (A) On each primary apical dendritic branch, ten consecutive 10 µm-long dendritic segments were analyzed to quantify mean spine density per 10 µm-long dendritic segment. Mean spine density in dMTs (1.28 ± 0.02) was significantly lower than that in *FMR1* KO mice (1.60 ± 0.02; *p* < 0.001) and significantly higher than that in *dnPAK* TG mice (1.06 ± 0.01; *p* < 0.001). ANOVA, *p* < 0.0001. ***: *p* < 0.001.

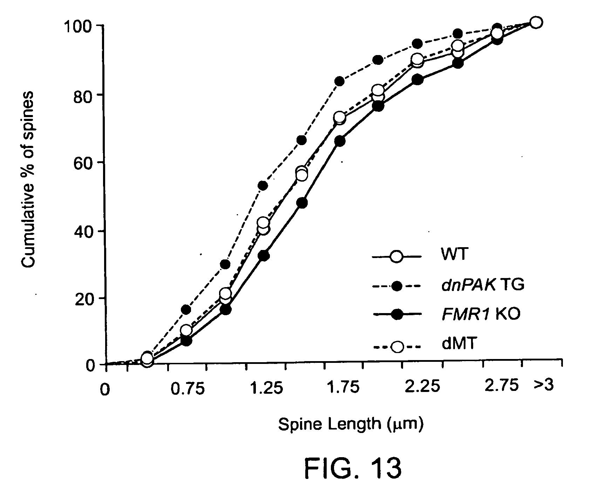

*Figure 13**. Quantificaition of spine length. FMR1* KO neurons (444 spines) exhibited a significant shift in the overall spine distribution towards spines of longer length compared to wild-type neurons (406 spines; Kolmogorov-Smirnov test: *p* < 0.05), while *dnPAK* TG neurons (630 spines) exhibited the opposite shift to shorter spines (*p* < 0.01). In contrast, spine length distribution in dMT neurons (785 spines) overlapped well with wild-type neurons and is significantly different from *FMR1* KO neurons (*p* < 0.01).

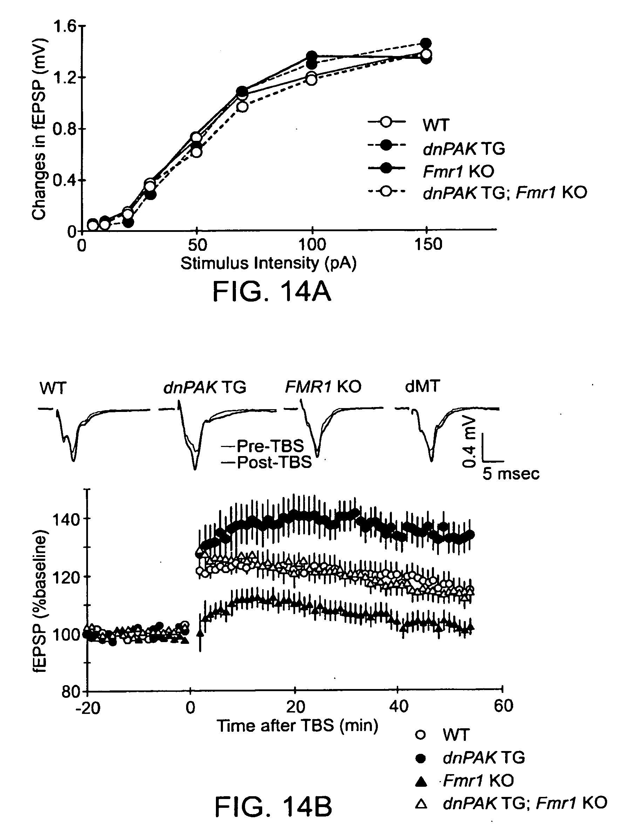

*Figure 14**. PAK inhibition rescues reduced cortical LTP in FMR1 KO mice.* (A) Input-output curves plotting the changes in field excitatory post-synaptic potential (fEPSP) amplitude and their corresponding presynaptic stimulus intensity in wild-type (n = 45 slices, 16 mice), *dnPAK* TG (n = 30 slices, 10 mice), *FMR1* KO (n = 57 slices, 19 mice) and dMT mice (n = 24 slices, 8 mice). (B) Cortical LTP induced by TBS was enhanced in *dnPAK* TG (n = 13 slices, 11 mice), but reduced in *FMR1* KO (n = 17 slices, 11 mice), relative to wild-type controls (n = 17 slices, 11 mice; for responses at 55 minutes post stimulation, ANOVA, *p* < 0.05; for both *dnPAK* TG versus WT and *FMR1* KO versus WT, *p* < 0.04). By contrast, the magnitude of LTP was indistinguishable between dMT slices (n = 13 slices, 9 mice) and wild-type controls (p > 0.05 for responses at 55 minutes post stimulation). An overlay of representative field potential traces taken during baseline of recording and at 55 minutes post stimulation is shown for each genotype.

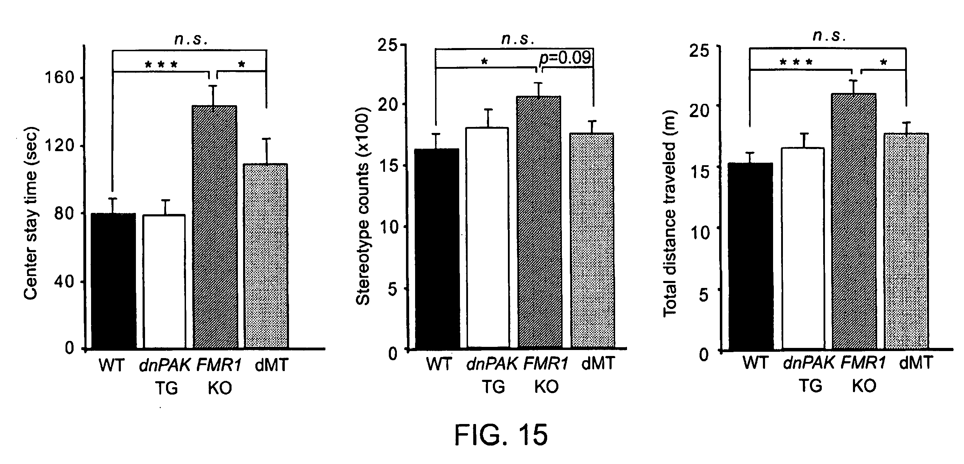

*Figure 15**. Open field test.* Mice of different genotypes were subjected to the open field test according to standard procedures. Each mouse ran for 10 minutes in an activity monitor chamber. Open field activity was detected by photobeam breaks and analyzed by VersaMax software. Activities measured were the amount of time the mouse spent in the center of the field, the number of times the mouse exhibited repetitive behaviors ("stereotypy,"), and the total distance traveled by the mouse. Genotypes are as follows: (1) wild-type (n = 10 mice); (2) *dnPAK* TG (n = 10 mice); (3) *FMR1* KO (n = 11 mice); and (4) *dnPAK* TG; *FMR1* KO *("dMT* mice" n = 11 mice). *"n.s.":* not statistically different. *: *p <* 0.05; ***: *p* < 0.001. *(A) FMR1* KO traveled a longer distance compared to wild-type mice (ANOVA, *p* < 0.01. WT: 15.29 ± 0.92 m; *FMR1* KO: 20.99 ± 1.10 m, *p* < 0.001 ). *(B) FMR1* KO exhibited a higher number of repetitive behaviors than wild-type mice *(stereotypy counts: ANOVA, p* < 0.05. WT: 1636 ± 119; *FMR1* KO: 2049 ± *125, p* < 0.05). *(C) FMR1* KO stayed a longer period of time in the center of the open field than wild-type mice (ANOVA, *p* < 0.001. WT: 79.8 ± 8.5 sec; *FMR1* KO: 143.1 ± 12.0 sec, *p* < 0.001). In all of these three behaviors, dMT mice exhibited comparable performance to wild-type controls (*p* > 0.05 for all of the following parameters: *distance traveled:* 17.76 ± 0.91 m; *stereotypy counts:* 1756 ± 102; *center time:* 108.8 ± 14.6 sec).

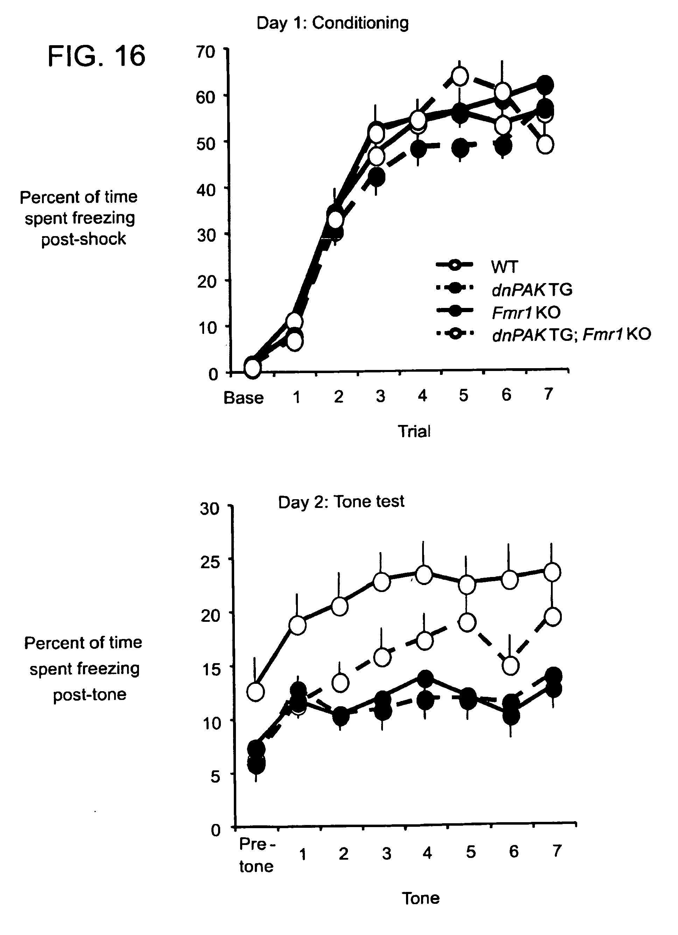

*Figure 16**. Trace fear conditioning task.* Mice of different genotypes (WT, n = 15 mice; *dnPAK TG,* n = 12 mice; *FMR1* KO, n = 15 mice; dMT, n = 9 mice) were subjected to the trace fear conditioning task according to standard procedures. On day 1 ("conditioning"), mice were placed into a training chamber for 60 seconds before the onset of a 15-second white noise tone. Another 30 seconds later, mice received a 1-second shock (0.7 mA intensity). Thus, one trial is composed of tone, 30 seconds blank time (also called "trace"), and then shock. Seven trials with an intertrial interval (ITI) of 210 seconds were performed. To examine whether mice remember this association, on day 2 ("tone test"), mice were placed into a new chamber with a different shape and smell from the first chamber. After 60 seconds, a 15-second tone was repeated for seven times with an ITI of 210 seconds. Video images were digitized and the percentage of freezing time during each ITI was analyzed by Image FZ program. Freezing was defined as the absence of all but respiratory movement for a 1-second period. On day 1 ("Conditioning"), the four genotypes of mice exhibited comparable amounts of freezing pre-conditioning ("Baseline") and post-conditioning in all trials. At the 24-hour tone test, the four genotypes exhibit comparable amounts of pre-tone freezing (ANOVA *p* > 0.05). However, for tone-dependent freezing, *FMR1* KO mice and *dnPAK* TG mice exhibited a significant reduction compared to wild-type controls (ANOVA for each tone session, *p* < 0.05; for *FMR1* KO versus WT, *p* < 0.05 for session I and *p* < 0.01 for sessions 2 to 7; for *dnPAK* TG versus WT, *p* > 0.05 for session 1 and *p* < 0.01 for sessions 2 to 7). dMT mice also showed freezing deficits during the first several tone sessions (sessions 1 to 4) compared to wild-type controls (*p* < 0.05). However, with additional tone sessions (sessions 5 to 7), freezing by dMT mice caught up to that of wild-type controls (*p* > 0.05). *"n.s.":* not statistically different. *: *p* < 0.05; **: *p* < 0.01; ***: *p* < 0.001.

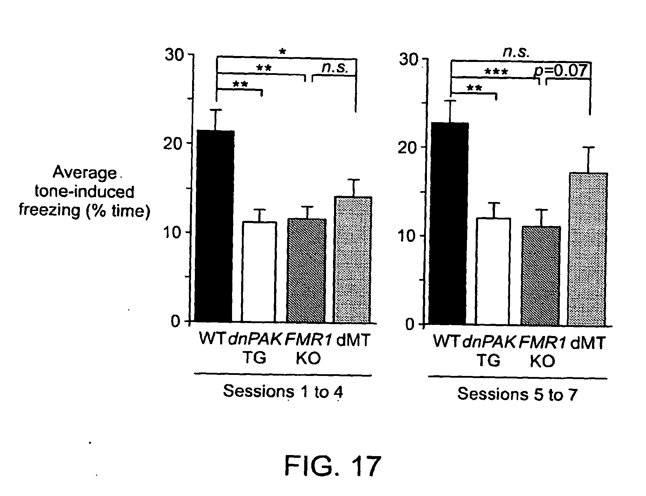

*Figure 17**. Quantification of mean tone-induced freezing.* Average freezing for tone sessions 1 to 4: ANOVA *p* < 0.05. dMT mice showed freezing deficits compared to wild-type controls (p < 0.05), but the deficits in dMT mice were less pronounced compared to *dnPAK TG* (*p* < 0.01) or *FMR1* KO mice (*p* < 0.01). Average freezing for tone sessions 5 to 7: ANOVA *p* < 0.05. Freezing level in dMT mice was not significantly different from wild-type controls (*p* > 0.05) and there were trends in its difference from *dnPAK* TG (*p* = 0.12) or *FMR1* KO mice *(p* = 0.07).

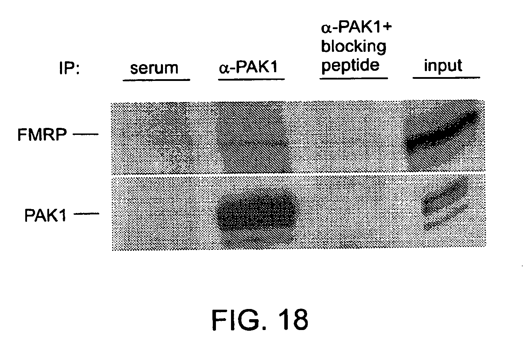

*Figure 18**. Immunoprecipitation.* Immunoprecipitations were performed using either rabbit serum (negative control), α-PAK1, or α-PAK1 plus a blocking peptide. Western blots were probed for either PAK1 or FMRP. α-PAK1, but not rabbit serum, immunoprecipitates FMRP in this assay, The specificity of the interaction was tested by including a blocking peptide specific for α-PAK1 in the immunoprecipitation reaction. Input: 2% of the extract used for a single immunoprecipitation was loaded on the gel.

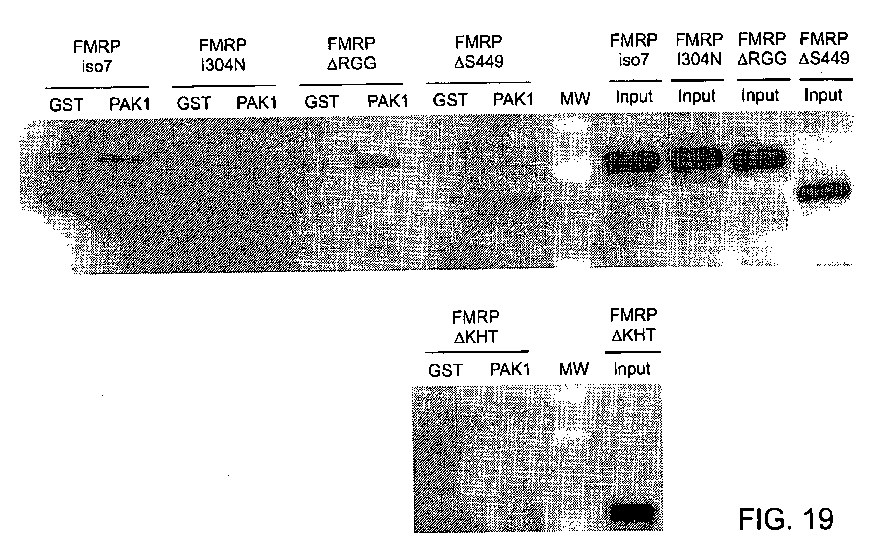

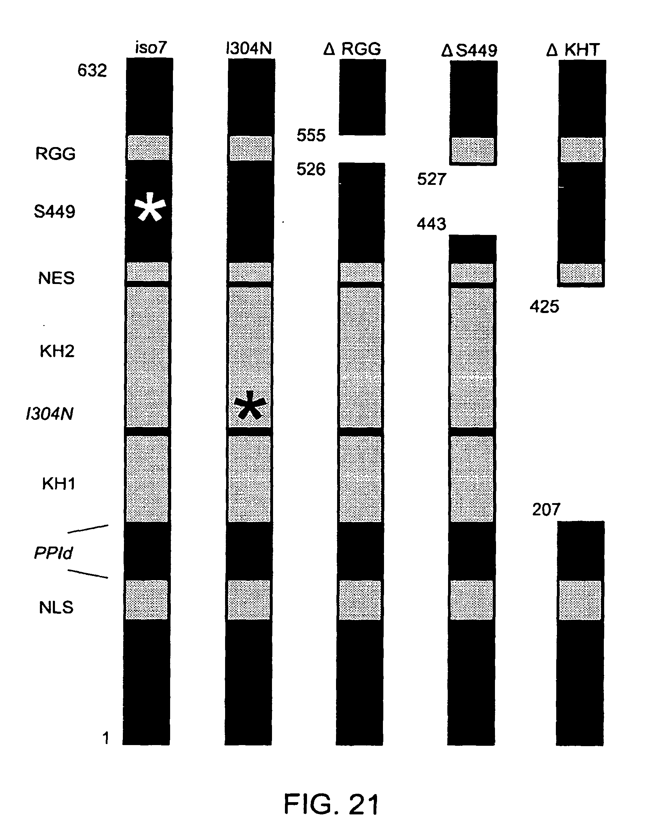

*Figure 19**. GST pull down.* FMRP was produced by *in vitro*-translation. GST and GST-tagged PAK1 were purified from a bacterial expression system. Wild type FMRP and the FMRP mutants depicted in Figure 21 were used in the GST-pull down assay. *"*Input*": in vitro* translated FMRP sample before the reaction was carried out; "MW": molecular weight standard.

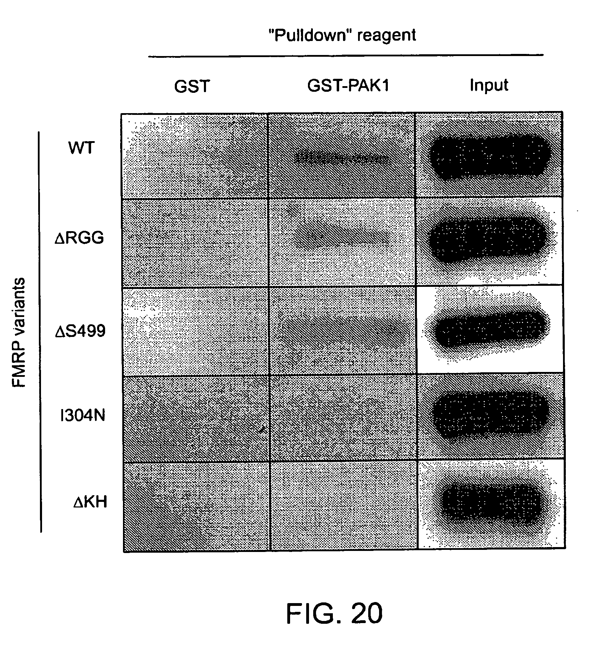

*Figure 20**. Characterization of the interaction between PAK1 and various FMRP variants* in vitro. *In vitro*-translated FMRP variants were incubated with GST or GST-PAK1 and glutathione sepharose beads. The complexes isolated by this method were subjected to SDS-PAGE and Western blotted for FMRP. "Input": 10% of *in vitro*-translated FMRP sample before the binding reaction was carried out was loaded on the gel.

*Figure 21**. Wild type and mutant* FMRP *domain structure.* Figure 21 (adapted from Mazroui *et al.,* 2003, *Hum. Mol. Genet.* 12:3087). shows a schematic structure of FMRP, highlighting various functional domains including three RNA-binding motifs (RGG, KH1, and KH2) and the phosphorylation site (S499, represented by a white asterisk). The constructs used for *in vitro* binding included full length (WT), truncated (ΔRGG, ΔS499 and ΔKH), or mutated (I304N) FMRP. ΔRGG refers to the FMRP variant with a deletion of the RGG box at amino acids 526 - 555. The deleted area in ΔS499 spans amino acids 443 - 527 and includes the phosphorylation site, S499, as well as putative phosphorylation sites. The isoleucine to asparagine missense mutation in the KH2 domain mimics that previously reported in a human FXS patient (I304N, represented by a black asterisk). The ΔKH deletion mutant lacks both KH domains in tandem corresponding to amino acids 207 - 425. Adapted from Mazroui et al., 2003, Hum. Mol. Genet., 12:3087; numbering refers to the amino acid positions designated by the SwissProt Q06787 entry.

### Definitions

*Amino acid:* As used herein, term "amino acid," in its broadest sense, refers to any compound and/or substance that can be incorporated into a polypeptide chain. In some embodiments, an amino acid has the general structure H₂N-C(H)(R)-COOH. In some embodiments, an amino acid is a naturally-occurring amino acid. In some embodiments, an amino acid is a synthetic amino acid; in some embodiments, an amino acid is a D-amino acid; in some embodiments, an amino acid is an L-amino acid. "Standard amino acid" refers to any of the twenty standard L-amino acids commonly found in naturally occurring peptides. "Nonstandard amino acid" refers to any amino acid, other than the standard amino acids, regardless of whether it is prepared synthetically or obtained from a natural source. As used herein, "synthetic amino acid" encompasses chemically modified amino acids, including but not limited to salts, amino acid derivatives (such as amides), and/or substitutions. Amino acids, including carboxy- and/or amino-terminal amino acids in peptides, can be modified by methylation, amidation, acetylation, and/or substitution with other chemical groups that can change the peptide's circulating half-life without adversely affecting their activity. Amino acids may participate in a disulfide bond. The term "amino acid" is used interchangeably with "amino acid residue," and may refer to a free amino acid and/or to an amino acid residue of a peptide. It will be apparent from the context in which the term is used whether it refers to a free amino acid or a residue of a peptide.

*Animal:* As used herein, the term "animals" refers to any member of the animal kingdom In some embodiments, "animal" refers to humans, at any stage of development. In some embodiments, "animal" refers to non-human animals, at any stage of development. In certain embodiments, the non-human animal is a mammal (*e.g*., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically-engineered animal, and/or a clone.

*Antibody:* As used herein, the term "antibody" refers to any immunoglobulin, whether natural or wholly or partially synthetically produced. All derivatives thereof which maintain specific binding ability are also included in the term. The term also covers any protein having a binding domain which is homologous or largely homologous to an immunoglobulin binding domain. Such proteins may be derived from natural sources, or partly or wholly synthetically produced. An antibody may be monoclonal or polyclonal. An antibody may be a member of any immunoglobulin class, including any of the human classes: IgG, IgM, IgA, IgD, and IgE. As used herein, the terms "antibody fragment" or "characteristic portion of an antibody" are used interchangeably and refer to any derivative of an antibody which is less than full-length. In general, an antibody fragment retains at least a significant portion of the full-length antibody's specific binding ability. Examples of antibody fragments include, but are not limited to, Fab, Fab', F(ab')2, scFv, Fv, dsFv diabody, and Fd fragments. An antibody fragment may be produced by any means. For example, an antibody fragment may be enzymatically or chemically produced by fragmentation of an intact antibody and/or it may be recombinantly produced from a gene encoding the partial antibody sequence. Alternatively or additionally, an antibody fragment may be wholly or partially synthetically produced. An antibody fragment may optionally comprise a single chain antibody fragment. Alternatively or additionally, an antibody fragment may comprise multiple chains which are linked together, for example, by disulfide linkages. An antibody fragment may optionally comprise a multimolecular complex. A functional antibody fragment typically comprises at least about 50 amino acids and more typically comprises at least about 200 amino acids.

*Approximately:* As used herein, the term "approximately" or "about," as applied to one or more values of interest, refers to a value that is similar to a stated reference value. In certain embodiment, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Biologically active:* As used herein, the phrase "biologically active" refers to a characteristic of any substance that has activity in a biological system and/or organism. For instance, a substance that, when administered to an organism, has a biological effect on that organism, is considered to be biologically active. In particular embodiments, where a protein or polypeptide is biologically active, a portion of that protein or polypeptide that shares at least one biological activity of the protein or polypeptide is typically referred to as a "biologically active" portion.

*Candidate substance:* As used herein, the term "candidate substance" refers to any substance that may potentially inhibit FXS and/or modulate PAK activity. A candidate substance may be a protein, a nucleic acid, a small molecule, a lipid, a carbohydrate, a glycoprotein, a proteoglycan, combinations thereof, and/or characteristic portions thereof. It may prove to be the case that the most useful candidate substances will be substances that are structurally related to FMRP, PAK, their binding partners, their upstream effectors, and/or their downstream effectors (*e.g*., mimics). However, it may be the case that the most useful candidate substances will have little or no structural relationship to FMRP, PAK, their binding partners, their upstream effectors, and/or their downstream effectors. In some embodiments, candidate substances are provided as individual substances. In some embodiments, candidate substances are provided in the form of a library, collection, and/or set of candidate substances. In some embodiments, candidate substances can be screened for their ability to modulate PAK.

*Characteristic portion:* As used herein, the term a "characteristic portion" of a substance, in the broadest sense, is one that shares some degree of sequence and/or structural identify and/or at least one functional characteristic with the relevant intact substance. For example, a "characteristic portion" of a protein or polypeptide is one that contains a continuous stretch of amino acids, or a collection of continuous stretches of amino acids, that together are characteristic of a protein or polypeptide. In some embodiments, each such continuous stretch generally will contain at least 2, 5, 10, 15, 20, 50, or more amino acids. A "characteristic portion" of a nucleic acid is one that contains a continuous stretch of nucleotides, or a collection of continuous stretches of nucleotides, that together are characteristic of a nucleic acid. In some embodiments, each such continuous stretch generally will contain at least 2, 5, 10, 15, 20, 50, or more nucleotides. In general, a characteristic portion of a substance (*e.g*. of a protein, nucleic acid, small molecule, *etc*.) is one that, in addition to the sequence and/or structural identity specified above, shares at least one functional characteristic with the relevant intact substance. In some embodiments, a characteristic portion may be biologically active.

*Expression:* As used herein, "expression" of a nucleic acid sequence refers to one or more of the following events: (1) production of an RNA template from a DNA sequence (*e.g.*, by transcription); (2) processing of an RNA transcript (*e.g*., by splicing, editing, 5' cap formation, and/or 3' end formation); (3) translation of an RNA into a polypeptide or protein; (4) post-translational modification of a polypeptide or protein.

*FMR1:* As used herein, the *"FMR1"* gene refers to any nucleotide sequence that encodes the fragile X mental retardation protein (FMRP) and/or a characteristic portion thereof. Representative examples of *FMR1* nucleotide sequences depicted in Figure 1 include GenBank Accession Number AF305881 and GenBank Accession Number L29074. The *"FXR"* gene is homologous to *FMR1* and may compensate for loss of *FMR1* function. Representative examples *of FXR* nucleotide sequences include, but are not limited to, *FXR1* (GenBank Accession Number U25165) and *FXR2* (GenBank Accession Number U31501). *FXRI and FXR2* genes depicted in Figure 1 are homologous to *FMR1* and may compensate for loss of *FMR1* function. In some embodiments, an *FMR1* gene comprises any nucleotide sequence that shares about 70%, about 80%, about 90%, about 95%, or greater than 95% sequence identity with the sequences of GenBank Accession Numbers AF305881, L29074, U25165, and/or U3150 1.

*Fragile X mental retardation protein (FMRP):* As used herein, "fragile X mental retardation protein," or "FMRP," refers to any protein product of the *fragile X mental retardation (FMR1)* nucleotide sequence, and/or a characteristic portion thereof. Representative examples of FMRP amino acid sequences depicted in Figure 1 include, but are not limited to, GenBank Accession Number AAG22045 and GenBank Accession Number AAB18829. The "FXR" protein is homologous to FMRP and may compensate for loss of FMRP function. Representative examples of FXR amino acid sequences include, but are not limited to, FXR (GenBank Accession Number AAC50155) and FXR2 (GenBank Accession Number AAC50292). In some embodiments, FMRP comprises any amino acid sequence that shares about 60%, about 70%, about 80%, about 90%, about 95%, or greater than 95% sequence identity with the sequences of GenBank Accession Numbers AAG22045, AAB 18829, AAC50155, and/or AAC50292.

*Fragile X Syndrome (FXS):* As used herein, in some embodiments "fragile X syndrome," or "FXS," refers to a disease, disorder, and/or condition characterized by one or more of the following symptoms: (1) behavioral symptoms, including but not limited to hyperactivity, stereotypy, anxiety, seizure, impaired social behavior, and/or cognitive delay;
(2) defective synaptic morphology, such as an abnormal number, length, and/or width of dendritic spines; and/or (3) defective synaptic function, such as enhanced long-term depression (LTD) and/or reduced long-term potentiation (LTP). In some embodiments, FXS refers to a disease, disorder, and/or condition caused by and/or associated with one or more of the following: (1) a mutation in *FMR1* (the nucleotide sequence encoding FMRP); (2) defective *FMR1* expression; (3) increased and/or decreased expression of FMRP; (4) defective FMRP function; (5) increased and/or decreased expression of FMRP's natural binding partners; (6) an increased and/or decreased ability of FMRP to bind to its natural binding partners; (7) decreased or absent arginine methylation of FMRP; (8) the increased methylation *of FMR1* CpG repeats in the 5' UTR of exon 1; (9) the mislocalization or misexpression of FMRP within the cell or within the organism; (10) the modulation of function *of FMR1* transcription factors Sp 1 and/or NRF 1; (11) an increased and/or decreased ability of FMRP to associate with polysomes; (12) the loss of function *of FXR1* and/or *FXR2,* which are homologous to *FMR1* and may compensate for loss of *FMR1* function; (13) the decreased ability of FMRP to recognize RNA secondary structures that FMRP normally recognizes (such as intramolecular G quartet and/or FMRP "kissing complex"); (14) an increased and/or decreased ability of FMRP to interact with miRNAs and/or members of the miRNA pathway; (15) an increased and/or decreased ability of FMRP to interact with its known target RNAs, such as RNAs encoding Racl, microtubule-associated protein 1B, activity-regulated cytoskeleton-associated protein, and/or alpha-calcium/calmodulin-dependent protein kinase II; (16) an increased and/or decreased ability of phosphatase PP2A to act on FMRP (PP2A is thought to bring about translational repression activity of FMRP); (17) the increased and/or decreased activity of mGluRS, which is known to decrease activity of phosphatase PP2A (18) exaggerated signaling in mGluR pathways *(*Bear et al., 2004, Trends Neurosci., 27:370);
(19) disruption of a KH domain of FMRP, which decreases the ability of FMRP to interact with PAK; and/or (20) an I304N mutation in FMRP, which decreases the ability of FMRP to interact with PAK. Those of ordinary skill in the art will appreciate that the teachings of the present invention described herein with respect to FXS are in fact applicable to other neurodevelopmental disorders including, for example, premature ovarian failure (POF)" fragile X-associated tremor ataxia (FXTAS), and/or other neurodevelopmental disorders, including, but not limited to, various forms of mental retardation and/or autism spectrum disorders (ASD). Furthermore, those of ordinary skill in the art will appreciate that the teachings of the present invention described herein with respect to FXS are applicable to any disease, disorder, and/or condition caused by and/or associated with one or more of the following: (1) a mutation in *FMR1* (the nucleotide sequence encoding FMRP);
(2) *defective FMR1* expression; (3) increased and/or decreased expression of FMRP; (4) defective FMRP function; (5) increased and/or decreased expression of FMRP's natural binding partners; (6) an increased and/or decreased ability of FMRP to bind to its natural binding partners; (7) decreased or absent arginine methylation of FMRP; (8) the increased methylation of *FMR1* CpG repeats in the 5' UTR of exon 1; (9) the mislocalization or misexpression of FMRP within the cell or within the organism; (10) the modulation of function of *FMR1* transcription factors Sp1 and/or NRF1; (11) an increased and/or decreased ability of FMRP to associate with polysomes; (12) the loss of function of *FXR1* and/or *FXR2,* which are homologous to *FMR1* and may compensate for loss of *FMR1* function; (13) the decreased ability of FMRP to recognize RNA secondary structures that FMRP normally recognizes (such as intramolecular G quartet and/or FMRP "kissing complex"); (14) an increased and/or decreased ability of FMRP to interact with miRNAs and/or members of the miRNA pathway; (15) an increased and/or decreased ability of FMRP to interact with its known target RNAs, such as RNAs encoding Racl, microtubule-associated protein 1B, activity-regulated cytoskeleton-associated protein, and/or alpha-calcium/calmodulin-dependent protein kinase U; (16) an increased and/or decreased ability of phosphatase PP2A to act on FMRP (PP2A is thought to bring about translational repression activity of FMRP); (17) the increased and/or decreased activity of mGluRS, which is known to decrease activity of phosphatase PP2A; (18) exaggerated signaling in mGluR pathways (Bear et al., 2004, Trends Neurosci., 27:370);
(19) disruption of a KH domain of FMRP, which decreases the ability of FMRP to interact with PAK; and/or (20) an I304N mutation in FMRP, which decreases the ability of FMRP to interact with PAK.

*Functional:* As used herein, a "functional" biological molecule is a biological molecule in a form in which it exhibits a property and/or activity by which it is characterized.

*Gene:* As used herein, the term "gene" has its meaning as understood in the art It will be appreciated by those of ordinary skill in the art that the term "gene" may include gene regulatory sequences *(e.g.,* promoters, enhancers, *etc.)* and/or intron sequences. It will further be appreciated that definitions of gene include references to nucleic acids that do not encode proteins but rather encode functional RNA molecules such as tRNAs, RNAi-inducing agents, *etc.* For the purpose of clarity we note that, as used in the present application, the term "gene" generally refers to a portion of a nucleic acid that encodes a protein; the term may optionally encompass regulatory sequences, as will be clear from context to those of ordinary skill in the art. This definition is not intended to exclude application of the term "gene" to non-protein-coding expression units but rather to clarify that, in most cases, the term as used in this document refers to a protein-coding nucleic acid.

*Gene product* or *expression product:* As used herein, the term "gene product" or "expression product" generally refers to an RNA transcribed from the gene (pre-and/or post-processing) or a polypeptide (pre- and/or post-modification) encoded by an RNA transcribed from the gene.

*Homology:* As used herein, the term "homology" refers to the overall relatedness between polymeric molecules, *e.g.* between nucleic acid molecules (*e.g*. DNA molecules and/or RNA molecules) and/or between polypeptide molecules. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% identical. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% similar.

*Identity:* As used herein, the term "identity" refers to the overall relatedness between polymeric molecules, *e.g*. between nucleic acid molecules (*e.g*. DNA molecules and/or RNA molecules) and/or between polypeptide molecules. Calculation of the percent identity of two nucleic acid sequences, for example, can be performed by aligning the two sequences for optimal comparison purposes (*e.g*., gaps can be introduced in one or both of a first and a second nucleic acid sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or substantially 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using the algorithm of Meyers and Miller (CABIOS, 1989, 4:11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix.

*Isolated:* As used herein, the term "isolated" refers to a substance and/or entity that has been (1) separated from at least some of the components with which it was associated when initially produced (whether in nature and/or in an experimental setting), and/or (2) produced, prepared, and/or manufactured by the hand of man. Isolated substances and/or entities may be separated from at least about 10%, about 20%, about 30%, about 40%, about 50%, about 60%, about 70%, about 80%, about 90%, or more of the other components with which they were initially associated. In some embodiments, isolated agents are more than about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components.

*Natural binding partner:* As used herein, the term "natural binding partner" refers to any substance that binds to PAK and/or FMRP. In some embodiments, the substance binds directly, and in some embodiments, the substance binds indirectly. A natural binding partner substance may be a protein, nucleic acid, lipid, carbohydrate, glycoprotein, proteoglycan, and/or small molecule that binds to either PAK and/or FMRP. A change in the interaction between PAK and/or FMRP and a natural binding partner may manifest itself as an increased and/or decreased probability that the interaction forms. A change in the interaction between PAK and/or FMRP and a natural binding partner may manifest itself as an increased and/or decreased concentration of PAK and/or FMRP/natural binding partner complex within the cell. This can result in an increased and/or decreased activity of PAK and/or FMRP. The present invention identifies FMRP as a novel natural binding partner of PAK. Other natural binding partners of PAK include PAK substrates and/or other substances that interact with PAK. Examples of natural binding partners of PAK include; but are not limited to; Myosin light chain kinase (MLCK); regulatory Myosin light chain (R-MLC); Myosin I heavy chain; Myosin II heavy chain; Myosin VI; Caldesmon; Desmin; Op18/stathmin; Merlin; Filamin A; LIM kinase (LIMK); Ras; Raf; Mek; p47^{phox}; BAD; caspase 3; estrogen and/or progesterone receptors; RhoGEF; GEF-H1; NET1; Gaz; phosphoglycerate mutase-B; RhoGDI; prolactin; p41^{Arc}; Aurora-A; Rac/Cdc42; CIB; sphingolipids; G-protein β and/or γ subunits; PIX/COOL; GIT/PKL; Paxillin; Nef; NESH; SH3-containing proteins *(e.g.* Nck and/or Grb2); kinases (e.g. Akt, PDK1, PI 3-kinase/p85, Cdk5, Cdc2, Src kinases, Abl, and/or protein kinase A (PKA)); and/or phosphatases *(e.g.* phosphatase PP2A, POPX1, and/or POPX2) (Bokoch et al., 2003,Annu. Rev. Biochem., 72:743; and Hofmann et al., 2004, J. Cell Sci., 117:4343).
Prominent upstream effectors of PAK include, but are not limited to, PDK1, PDK2, PI3K, and/or NMDARs.

*Nucleic acid:* As used herein, the term "nucleic acid," in its broadest sense, refers to any compound and/or substance that is or can be incorporated into an oligonucleotide chain. In some embodiments, a nucleic acid is a compound and/or substance that is or can be incorporated into an oligonucleotide chain via a phosphodiester linkage. In some embodiments, "nucleic acid" refers to individual nucleic acid residues (*e.g*. nucleotides and/or nucleosides). In some embodiments, "nucleic acid" refers to an oligonucleotide chain comprising individual nucleic acid residues. As used herein, the terms "oligonucleotide" and "polynucleotide" can be used interchangeably. In some embodiments, "nucleic acid" encompasses RNA as well as single and/or double-stranded DNA and/or cDNA. Furthermore, the terms "nucleic acid," "DNA," "RNA," and/or similar terms include nucleic acid analogs, *i.e.* analogs having other than a phosphodiester backbone. For example, the so-called "peptide nucleic acids," which are known in the art and have peptide bonds instead of phosphodiester bonds in the backbone, are considered within the scope of the present invention. The term "nucleotide sequence encoding an amino acid sequence" includes all nucleotide sequences that are degenerate versions of each other and/or encode the same amino acid sequence. Nucleotide sequences that encode proteins and/or RNA may include introns. Nucleic acids can be purified from natural sources, produced using recombinant expression systems and optionally purified, chemically synthesized, *etc.* Where appropriate, *e.g*., in the case of chemically synthesized molecules, nucleic acids can comprise nucleoside analogs such as analogs having chemically modified bases or sugars, backbone modifications, *etc.* A nucleic acid sequence is presented in the 5' to 3' direction unless otherwise indicated. The term "nucleic acid segment" is used herein to refer to a nucleic acid sequence that is a portion of a longer nucleic acid sequence. In many embodiments, a nucleic acid segment comprises at least 3, 4, 5, 6, 7, 8, 9, 10, or more residues. In some embodiments, a nucleic acid is or comprises natural nucleosides (*e.g*. adenosine, thymidine, guanosine, cytidine, uridine, deoxyadenosine, deoxythymidine, deoxyguanosine, and deoxycytidine); nucleoside analogs (*e.g.*, 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (*e.g*., methylated bases); intercalated bases; modified sugars (*e.g*., 2'-fluororibose, ribose, 2'-deoxyribose, arabinose, and hexose); and/or modified phosphate groups (*e.g*., phosphorothioates and 5'-N-phosphoramidite linkages). In some embodiments, the present invention is specifically directed to "unmodified nucleic acids," meaning nucleic acids *(e.g.* polynucleotides and residues, including nucleotides and/or nucleosides) that have not been chemically modified in order to facilitate or achieve delivery.

*PAK activity:* As used herein, the term "PAK activity" refers to any activity and/or function of p21-activated kinase. Examples of "PAK activity" include, but are not limited to, PAK binding to other substances, PAK kinase activity, autophosphorylation, translocation, *etc.* "PAK activity" is used interchangeably with "PAK function."

*PAK Modulator*: As used herein, the term "PAK modulator" refers to any substance that directly and/or indirectly changes, affects, alters, increases, and/or decreases the activity and/or levels of PAK. PAK modulators may modulate the level of PAK mRNA and/or protein; an activity of PAK; the half-life of PAK mRNA and/or protein; and/or the interaction between PAK and its natural binding partners (*e.g*., a substrate for a PAK kinase, a Rac protein, a cdc42 protein, and/or FMRP), as measured using standard methods. "PAK inhibitors" may inhibit, decrease, and/or abolish the level of PAK mRNA and/or protein; an activity of PARK; the half-life of PAK mRNA and/or protein; and/or the interaction between PAK and its natural binding partners (*e.g*., a substrate for a PAK kinase, a Rac protein, a cdc42 protein, and/or FMRP), as measured using standard methods. "PAK activators" may activate and/or increase the level of PAK mRNA and/or protein; an activity of PAK; the half-life of PAK mRNA and/or protein; and/or the interaction between PAK and its natural binding partners (*e.g*., a substrate for a PAK kinase, a Rac protein, a cdc42 protein, and/or FMRP), as measured using standard methods. mRNA expression levels may be determined using standard RNase protection assays and/or *in situ* hybridization assays, and/or the level of protein may be determined using standard Western and/or immunohistochenistry analysis. The phosphorylation levels of signal transduction proteins downstream of PAK activity may be measured using standard assays. PAK modulators may modulate binding between PAK and FMRP- "PAK inhibitors" may reduce, abolish, and/or remove the binding between these two entities, and "PAK activators" may increase and/or strengthen the binding between these two entities. Thus, binding between PAK and FMRP is stronger in the absence of the inhibitor than in its presence and is weaker in the absence of the activator than in its presence. Put another way, a PAK inhibitor increases the Kₘ of binding between PAK and FMRP, and a PAK activator decreases the Kₘ of binding between PAK and FMRP. Alternatively or additionally, PAK modulators may inhibit and/or activate the kinase activity of PAK. In particular, PAK inhibitors and/or activators may modulate the ability of PAK to phosphorylate FMRP. PAK modulators may be inorganic and/or organic. PAK modulators may comprise one or more of the following: proteins, peptides, antibodies, nucleic acids, RNAi-inducing entities, antisense oligonucleotides, ribozymes, small molecules, glycoproteins, proteoglycans, viruses, lipids, and/or carbohydrates. PAK modulators may be in the form of monomers, dimers, oligomers, and/or in a complex. In some embodiments, as used herein, a "PAK modulator" is a substance that modulates all isoforms of PAK. In one instance, as used herein, a "PAK modulator" is a substance that modulates a single isoform of PAK. In another instance, as used herein, a "PAK modulator" is a substance that modulates two or more isoforms of PAK. In some embodiments, as used herein, a "PAK modulator" is a substance that modulates a subset of PAK isoforms. In yet another instance, as used herein, a "PAK modulator" is a substance that modulates any of the isoforms of PAK.

*PAK protein:* As used herein the term "PAK protein" refers to a protein that belongs in the family of PAK serine/threonine protein kinases. These include mammalian isoform identified, *e.g.,* PAK1, PAK2, PAK3, PAK4, PAK5, PAK6, and/or PAK7; and/ or lower eukaryotic isoforms, such as the yeast Ste20 (Leberter et al., 1992, EMBO J., 11:4805) and/or the Dictyostelium single-headed myosin I heavy chain kinases (Wu et al., 1996, J. Biol. Chem., 271:31787)
Representative examples of PAK amino acid sequences depicted in Figures 2, 3, 4, and 5 include, but are not limited to, human PAK1 (GenBank Accession Number AAA65441), human PAK2 (GenBank Accession Number AAA65442), human PAK3 (GenBank Accession Number AAC36097), human PAK 4 (GenBank Accession Numbers NP_005875 and CAA09820), human PAK5 (GenBank Accession Numbers CAC18720 and BAA94194), human PAK6 (GenBank Accession Numbers NP-064553 and AAF82800), human PAK7 (GenBank Accession Number Q9P286), *C. elegans* PAK (GenBank Accession Number BAA 11844), *D*. *melanogaster* PAK (GenBank Accession Number AAC47094), and rat PAK1 (GenBank Accession Number AAB95646). Representative examples of *PAK* genes encoding PAK proteins include, but are not limited to, human *PAK1* (GenBank Accession Number U24152), human *PAK2* (GenBank Accession Number U24153), human *PAK3* (GenBank Accession Number AF068864), human *PAK4* (GenBank Accession Number AJ011855), human *PAK5* (GenBank Accession Number AB040812), and human *PAK6* (GenBank Accession Number AF276893). In some embodiments, a PAK protein comprises any amino acid sequence that shares about 60%, about 70%, about 80%, about 90%, about 95%, or greater than 95% sequence identity with the sequences of GenBank Accession Numbers AAA65441, AAA65442, AAC36097, NP_005875, CAA09820, CAC 18720, BAA94194, NP_064553, AAF82800, Q9P286, BAA 11844, AAC47094, and/or AAB95646. In some embodiments, a *PAK* gene comprises any nucleotide sequence that shares about 60%, about 70%, about 80%, about 90%, about 95%, or greater than 95% sequence identity with the sequences of GenBank Accession Numbers U24152, U24153, AF068864, AJ011855, AB040812, and/or AF276893. In some embodiments, as used herein, "PAK"-refers to all isoforms of PAK. In some embodiments, as used herein, "PAK" refers to a single isoform of PAK. In some embodiments, as used herein, "PAK" refers to two or more isoforms of PAK. In some embodiments, as used herein, "PAK" refers to a subset of PAK isoforms. In some embodiments, as used herein, "PAK" refers to any of the isoforms of PAK.

*Protein:* As used herein, the term "protein" refers to a polypeptide (*i.e.,* a string of at least two amino acids linked to one another by peptide bonds). Proteins may include moieties other than amino acids (*e.g.*, may be glycoproteins, proteoglycans, *etc*.) and/or may be otherwise processed or modified. Those of ordinary skill in the art will appreciate that a "protein" can be a complete polypeptide chain as produced by a cell (with or without a signal sequence), or can be a characteristic portion thereof Those of ordinary skill will appreciate that a protein can sometimes include more than one polypeptide chain, for example linked by one or more disulfide bonds or associated by other means. Polypeptides may contain L-amino acids, D-amino acids, or both and may contain any of a variety of amino acid modifications or analogs known in the art. Useful modifications include, *e.g*., terminal acetylation, amidation, *etc.* In some embodiments, proteins may comprise natural amino acids, non-natural amino acids, synthetic amino acids, and combinations thereof. The term "peptide" is generally used to refer to a polypeptide having a length of less than about 100 amino acids.

*Small Molecule:* In general, a "small molecule" is understood in the art to be an organic molecule that is less than about 5 kilodaltons (Kd) in size. In some embodiments, the small molecule is less than about 4 Kd, 3 Kd, about 2 Kd, or about 1 Kd. In some embodiments, the small molecule is less than about 800 daltons (D), about 600 D, about 500 D, about 400 D, about 300 D, about 200 D, or about 100 D. In some embodiments, a small molecule is less than about 2000 g/mol, less than about 1500 g/mol, less than about 1000 g/mol, less than about 800 g/mol, or less than about 500 g/mol. In some embodiments, small molecules are non-polymeric. In some embodiments, small molecules are not proteins, peptides, or amino acids. In some embodiments, small molecules are not nucleic acids or nucleotides. In some embodiments, small molecules are not saccharides or polysaccharides.

*Subject:* As used herein, the term "subject" or "patient" refers to any organism to which a composition of this invention may be administered, *e.g*., for experimental, diagnostic, prophylactic, and/or therapeutic purposes. Typical subjects include animals (*e.g*., mammals such as mice, rats, rabbits, non-human primates, and humans; insects; worms; *etc*.).

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Suffering from:* An individual who is "suffering from" FXS and/or other neurodevelopmental disorders has been diagnosed with or displays one or more symptoms of FXS and/or other neurodevelopmental disorders.

*Susceptible to:* An individual who is "susceptible to" FXS has not been diagnosed with FXS and/or may not exhibit symptoms of FXS but may be characterized by one or more of the following: (1) a mutation in *FMR1* (the nucleotide sequence encoding FMRP); (2) defective *FMR1* expression; (3) increased and/or decreased expression of FMRP; (4) defective FMRP function; (5) increased and/or decreased expression of FMRP's natural binding partners; (6) an increased and/or decreased ability of FMRP to bind to its natural binding partners; (7) decreased or absent arginine methylation of FMRP; (8) the increased methylation of *FMR1* CpG repeats in the 5' UTR of exon 1; (9) the mislocalization or misexpression of FMRP within the cell or within the organism; (10) the modulation of function of *FMR1* transcription factors Sp1 and/or NRF1; (11) an increased and/or decreased ability of FMRP to associate with polysomes; (12) the loss of function of *FXR1* and/or *FXR2,* which are homologous to *FMR1* and may compensate for loss of *FMR1* function; (13) the decreased ability of FMRP to recognize RNA secondary structures that FMRP normally recognizes (such as intramolecular G quartet and/or FMRP "kissing complex"); (14) an increased and/or decreased ability of FMRP to interact with miRNAs and/or members of the miRNA pathway; (15) an increased and/or decreased ability of FMRP to interact with its known target RNAs, such as RNAs encoding Rac1, microtubule-associated protein 1B, activity-regulated cytoskeleton-associated protein, and/or alpha-calcium/calmodulin-dependent protein kinase II; (16) an increased and/or decreased ability of phosphatase PP2A to act on FMRP (PP2A is thought to bring about translational repression activity of FMRP); (17) the increased and/or decreased activity of mGluR5, which is known to decrease activity of phosphatase PP2A; (18) exaggerated signaling in mGluR pathways (Bear et al., 2004, Trends Neuroscci., 27:370);
(19) disruption of a KH domain of FMRP, which decreases the ability of FMRP to interact with PAK; and/or (20) an I304N mutation in FMRP, which decreases the ability of FMRP to interact with PAK. In some embodiments, an individual who is susceptible to FXS will develop FXS and/or other neurodevelopmental disorder. In some embodiments, an individual who is susceptible to FXS will not develop FXS and/or other neurodevelopmental disorder.

*Test substance:* As used herein, the phrase "test substance" refers to any substance that may be utilized in the systems, methods, assays, and/or compositions described herein. A "test substance" may refer to one or more of the following: (1) a PAK protein, a nucleic acid encoding PAK, and/or homolog, portion, variant, mutant, and/or derivative thereof; (2) a natural binding partner of PAK, a nucleic acid encoding a natural binding partner of PAK, and/or a homolog, portion, variant, mutant, and/or derivative thereof; and/or (3) an FMRP protein, a nucleic acid encoding FMRP, and/or a homolog, portion, variant, mutant, and/or derivative thereof; (4) a substrate of PAK kinase, a nucleic acid encoding a substrate of PAK, and/or a homolog, portion, variant, mutant, and/or derivative thereof; and/or (5) a substance related to PAK signal transduction, and/or a homolog, portion, variant, mutant, and/or derivative thereof. In some embodiments, a test substance is a protein; nucleic acid; small molecule; carbohydrate; lipid; library, collection, and/or set of any of these; and/or combination of any of these.

*Therapeutically effective amount:* As used herein, the term "therapeutically effective amount" means an amount of inventive PAK modulator that is sufficient, when administered to a subject suffering from or susceptible to FXS and/or other neurodevelopmental disorders, to treat, diagnose, prevent, and/or delay the onset of the FXS and/or other neurodevelopmental disorders symptom(s) and/or condition(s).

*Therapeutic agent:* As used herein, the phrase "therapeutic agent" refers to any agent that, when administered to a subject, has a therapeutic effect and/or elicits a desired biological and/or pharmacological effect.

*Treating:* As used herein, the term "treat," "treatment," or "treating" refers to any method used to partially or completely alleviate, ameliorate, relieve, inhibit, prevent, delay onset of, reduce severity of and/or reduce incidence of one or more symptoms or features of a particular disease, disorder, and/or condition (*e.g*., FXS and/or other neurodevelopmental disorders). Treatment may be administered to a subject who does not exhibit signs of a disease and/or exhibits only early signs of the disease for the purpose of decreasing the risk of developing pathology associated with the disease.

*Vector:* As used herein, "vector" refers to a nucleic acid molecule capable of transporting another nucleic acid to which it has been linked. In some embodiment, vectors are capable of extra-chromosomal replication and/or expression of nucleic acids to which they are linked in a host cell such as a eukaryotic and/or prokaryotic cell. Vectors capable of directing the expression of operatively linked genes are referred to herein as "expression vectors".

### Detailed Description of Certain Preferred Embodiments of the Invention

In some embodiments, the present invention provides inhibitors of PAK for use in treating fragile X syndrome (FXS) and/or other neurodevelopmental disorders.

Compositions are included comprising at least one PAK inhibitor and a pharmaceutically acceptable excipient. In certain embodiments, PAK modulators function by inhibiting the interaction between PAK and the fragile X mental retardation protein (FMRP).

In some embodiments, the present invention provides methods for identifying PAK inhibitors useful for treating neurodevelopmebtal disorder such methods include high throughput screening methods. In another instance, the methods include *in vitro, in cyto,* assays.

### p21-activated kinase (PAK)

PAK, a family of serine-threonine kinases that is composed of at least three members, PAK1, PAK2 and/or PAK3, functions downstream of the small GTPases Rac and/or Cdc42 to regulate multiple cellular functions, including motility, morphogenesis, angiogenesis, and/or apoptosis (Bokoch et al., 2003, Annu. Rev. Biochem., 72:743; and Hofmann et al., 2004, J. Cell Sci., 117:4343). GTP-bound Rac and/or Cdc42 bind to inactive PAK, releasing steric constraints imposed by a PAK autoinhibitory domain and/or permitting PAK auto-phosphorylation and/or activation. Numerous autophosphorylation sites have been identified that serve as markers for activated PAK

As used herein, "PAK" refers to all isoforms of PAK (e.g. PAK1, PAK2, PAK3, PAK4, PAK5, PAK6, PAK7, etc.). As used herein, "PAK" can refer to a single isoform of PAK. As used herein, "PAK" can refer to two or more isoforms of PAK. As used herein, "PAK" can refer to a subset of PAK isoforms. As used herein, "PAK" can refer to any of the isoforms of PAK. As used herein, "PAK" can be a substance that is at least 60%, at least 70%, at least 80%, at least 90%, at least 95%, or at least 98% identical to any and/or all of PAK1, PAK2, PAK3, PAK4, PAK5, PAK6, and/or PAK7.

Prominent downstream targets of mammalian PAK include, but are not limited to, substrates of PAK kinase, such as Myosin light chain kinase (MLCK), regulatory Myosin light chain (R-MLC), Myosins I heavy chain, myosin II heavy chain, Myosin VI, Caldesmon, Desmin, Op18/stathmin, Merlin, Filamin A, LIM kinase (LIMK), Ras, Raf, Mek, p47^{Phox}, BAD, caspase 3, estrogen and/or progesterone receptors, RhoGEF, GEF-H1, NET1, Gaz, phosphoglycerate mutase-B, RhoGDI, prolactin, p41^{Arc}, and/or Aurora-A (Bokoch et al., 2003, Annu. Rev. Biochem., 72:743; and Hofmann et al., 2004, J. Cell Sci., 117:4343). Other substances known to bind to PAK in cells include CIB; sphingolipids; G-protein β and/or y subunits; PIX/COOL; GIT/PKL; Nef; Paxillin; NESH; SH3-containing proteins (*e.g*. Nck and/or Grb2); kinases (*e.g*. Akt, PDK1, PI 3-kinase/p85, Cdk5, Cdc2, Src kinases, Abl, and/or protein kinase A (PKA)); and/or phosphatases *(e.g.* phosphatase PP2A, POPX1, and/or POPX2)

Prominent upstream effectors of PAK include, but are not limited to, PDK1, PDK2, PI3K, and/or NMDARs.

The present invention encompasses the recognition that the abnormalities in cortical spine morphology of FXS patients and/or *FMR1* KO mice are substantially opposite of those found in transgenic mice in which activity of p21-activated kinase (PAK) is inhibited by its dominant negative form (*dnPAK TG* mice), for example, in the postnatal forebrain.

### PAK inhibitors

PAK inhibitors have been described in the art as possible substances for use in the treatment of cancer, endometriosis, urogenital disorders, macropinocytosis, viral infection, vascular permeability, joint disease, lymphocyte activation, muscle contraction, and/or diabetes (see, for example, U.S. Patents 5,863,532, 6,191,169, and 6,248,549; U.S. Patent Applications 2002/0045564, 2002/086390, 2002/106690, 2002/142325, 2003/124107, 2003/166623, 2004/091992, 2004/102623,2004/208880,2005/0203114, 2005/037965, 2005/080002, and 2005/233965; EP Patent Publication 1492871; Kumar et al., 2006, Nat. Rev. Cancer, 6:459; Eswaren et al., 2007, Structure, 15:201).

PAK inhibitors have also been described in the art as possible substances for use in the treatment of neurological disorders characterized by nerve damage and/or neurodegeneration. Such disorders include Parkinson's disease, Alzheimer's disease, prion-related diseases, neurofibromatosis, stroke-induced nerve damage, and/or diseases associated with nerve injury due to ischaemia and/or anoxia (see, for example, U.S. Patents 5,952,217 and 6,046,224; U.S. Patent Application 2006/088897; and PCT applications WO 99/02701 and WO 04/07507).

However, PAK inhibitors have not previously been described in the art as possible substances for use in the treatment of neurodevelopmental disorders, including but not limited to FXS, premature ovarian formation (POF), fragile X-associated tremor ataxia (FXTAS), various forms of mental retardation, and/or autism spectrum disorders (ASD). The present invention encompasses the use of PAK inhibitors in the treatment of FXS, POF, FXTAS, and/or other neurodevelopmental disorders.

A PAK inhibitor can refer to a substance, wherein the substance may be inorganic and/or organic; a biological effector and/or a nucleic acid encoding an agent such as a biological effector; a protein, polypeptide, or peptide including, but not limited to, a structural protein, an enzyme, a cytokine (such as an interferon and/or an interleukin), an antibiotic, a polyclonal or monoclonal antibody and/or an effective part thereof, such as an Fv fragment, which antibody or part thereof may be natural, synthetic, or humanized, a peptide hormone, a receptor, a signaling molecule and/or other protein; a nucleic acid, as defined below, including, but not limited to, an oligonucleotide and/or modified oligonucleotide, an RNAi-inducing entity, an antisense oligonucleotide and/or modified antisense oligonucleotide, cDNA, genomic DNA, an artificial and/or natural chromosome (*e.g*. a yeast artificial chromosome, bacterial artificial chromosome, *etc.)* and/or portion thereof, RNA, including mRNA, tRNA, rRNA and/or a ribozyme, and/or a peptide nucleic acid (PNA); a virus or virus-like particle; a nucleotide, ribonucleotide, and/or synthetic analogue thereof, which may be modified or unmodified; an amino acid and/or analogue thereof, which may be modified or unmodified; a non-peptide (*e.g*., steroid) hormone; a glycoprotein; a proteoglycan; a lipid; and/or a carbohydrate. Small molecules, including inorganic and/or organic chemicals, which bind to and/or occupy the active site of the polypeptide thereby making the catalytic site inaccessible to substrate such that normal biological activity is prevented, are included. A PAK inhibitor may be in solution and/or in suspension (e.g., in crystalline, colloidal, or other particulate form). A PAK inhibitor may be in the form of a monomer, dimer, oligomer, *etc*., and/or in a complex.

As used herein, a "PAK inhibitor" can be a substance that inhibits all isoforms of PAK (e.g. PAK1. PAK2, PAK3, PAK4, PAK5, PAK6, PAK7, etc.). As used herein, a "PAK inhibitor" can be a substance that inhibits a single isoform of PAK. As used herein, a "PAK inhibitor" can be a substance that inhibits two or more isoforms of PAK. As used herein, a "PAK inhibitor" can be a substance that inhibits a subset of PAK isoforms. As used herein, a "PAK inhibitor" can be a substance that inhibits any of the isoforms of PAK. As used herein, a "PAK inhibitor" can be a substance that inhibits any of PAK1, PAK2, PAK3, PAK4, PAK5, PAK6, and/or PAK7. As used herein, a "PAK inhibitor" can be a substance that inhibits any subset of PAK1, PAK2, PAK3, PAK4, PAK5, PAK6, and/or PAK7. As used herein, a "PAK inhibitor" can be a substance that inhibits all of PAK1, PAK2, PAK3, PAK4, PAK5, PAK6, PAK7. As used herein, a "PAK inhibitor" can be a substance that inhibits any entity that is at least 60%, at least 70%, at least 80%, at least 90%, at least 95%, or at least 98% identical to any and/or all of PAK1, PAK2, PAK3, PAK4, PAK5, PAK6, and/or PAK7.

Candidate PAK inhibitors may be isolated from natural sources, such as animals, bacteria, fungi, plants, and/or marine samples. It will be understood that candidate PAK inhibitors can be derived and/or synthesized from chemical compositions and/or man-made substances.

### Rational Drug Design

As used herein the term "candidate substance" refers to any substance that may potentially inhibit FXS and/or inhibit PAK activity. A candidate substance may be a protein, a nucleic acid, a small molecule, a lipid, a carbohydrate, a glycoprotein, a proteoglycan, combinations thereof, and/or characteristic portions thereof. It may prove to be the case that the most useful candidate substances will be substances that are structurally related to FMRP, PAK, their binding partners, their upstream effectors, and/or their downstream effectors (e.g., mimics). Using lead compounds to help develop improved compounds is known as "rational drug design" and includes not only comparisons with known inhibitors and/or activators, but predictions relating to the structure of target substances.

Rational drug design may be used to predict and/or produce structural analogs of known biologically-active PAK modulators (*i.e.* "PAK modulator starting materials"). By creating such analogs, it is possible to fashion therapeutic agents which may be more active and/or stable than the PAK modulator starting material, may have different susceptibility to alteration, and/or may affect the function of various the PAK modulator starting material. In some embodiments, a three-dimensional structure for a known candidate substance and/or characteristic portion thereof could be generated. In some embodiments, this is accomplished by x-ray crystallography, computer modeling, and/or by a combination of these approaches.

Antibodies can be used to ascertain the structure of a PAK modulators starting material. In principle, this approach yields a pharmacore upon which subsequent drug design can be based. It is possible to bypass protein crystallography altogether by generating anti-idiotypic antibodies to a functional, pharmacologically active antibody. As a mirror image of a mirror image, the binding site of anti-idiotype would be expected to be an analog of the original antigen. The anti-idiotype could then be used to identify and/or isolate peptides from banks of chemically- and/or biologically-produced peptides. Selected peptides would then serve as pharmacores. Anti-idiotypes may be generated using the methods described herein for producing antibodies, using an antibody as the antigen.

Other sterically similar substances may be formulated to mimic the key portions of the structure of rationally-designed PAK modulators. Such substances may be used in the same manner as or in a different manner from the PAK modulator starting material.

### Libraries

Libraries of candidate substances may be employed in-the methods and/or compositions described herein. The phrase "library of candidate substances," as used herein, refers to a collection and/or set of multiple species of substances that consist of randomly- and/or systematically-selected subunits and/or members. Screening libraries of candidate substances is a rapid and/or efficient way to screen large number of related and/or unrelated substances for activity. Combinatorial approaches lend themselves to rapid evolution of potential drugs by the creation of second, third, fourth, etc. generation substances modeled of active, but otherwise undesirable substances.

Combinatorial libraries (also known as "combinatorial chemical libraries"), small molecule libraries, peptides and/or peptide mimetics, defined chemical entities, oligonucleotides, and/or natural product libraries can be screened for activity. In some embodiments, a library of candidate substances may comprise a synthetic combinatorial library (*e.g*., a combinatorial chemical library), a cellular extract, a bodily fluid (*e.g*., urine, blood, tears, sweat, and/or saliva), or other mixture of synthetic and/or natural substances (*e.g*., a library of small molecules and/or a fermentation mixture).

Libraries of candidate substances may include, for example, proteins (*e.g*. peptides, polypeptides, antibodies, amino acids, etc.), nucleic acids (*e.g*., DNA, RNA, oligonucleotides, RNAi-inducing entities, antisense nucleic acids, ribozymes, peptide nucleic acids, aptamers, etc.), carbohydrates (*e.g*. monosaccharides, disaccharides, polysaccharides, etc.), small molecules (*e.g*. organic small molecules, inorganic small molecules, etc.), combinations thereof, and/or characteristic portions thereof. Each member of a library may be singular and/or may be a part of a mixture (*e.g*., a "compressed library"). A library may comprise substantially purified substances and/or may be "dirty" (*i.e.,* containing a significant quantity of impurities).

Candidate substances may be used in an initial screen in batches (*e.g*. batches of types of substances). The substances of those batches which show enhancement and/or reduction of the activity being assayed may subsequently be tested individually.

Libraries can be acquired from various commercial sources in an effort to "brute force" the identification of useful substances. Commercially available libraries can be obtained from Affymetrix, ArQuIe, Neose Technologies, Sarco, Ciddco, Oxford Asymmetry, Maybridge, Aldrich, Panlabs, Pharmacopoeia, Sigma, and/or Tripose. A comprehensive review of combinatorial libraries, in particular their construction and/or uses is provided in Dolle et al. (1999, J. of Comb. Chem., 1:235). Reference is made to combinatorial peptide library protocols (Cabilly, ed., Methods in Molecular Biology, Humana Press, Totowa, NJ, 1998).

Further references describing combinatorial libraries, their production and/or use include those available from the URL http://www.netsci.org/Science/Combichem/, including The Chemical Generation of Molecular Diversity. Michael R. Pavia, Sphinx Pharmaceuticals, A Division of Eli Lilly (Published July, 1995); Combinatorial Chemistry: A Strategy for the Future—MDL Information Systems discusses the role its Project Library plays in managing diversity libraries (Published July, 1995); Solid Support Combinatorial Chemistry in Lead Discovery and SAR Optimization, Adnan M. M. Mjalli and Barry E. Toyonaga, Ontogen Corporation (Published July, 1995); Non-Peptidic Bradykinin Receptor Antagonists From a Structurally Directed Non-Peptide Library. Sarvajit Chakravarty, Babu J. Mavunkel, Robin Andy, Donald J. Kyle*, Scios Nova Inc. (Published July, 1995); Combinatorial Chemistry Library Design using Pharmacophore Diversity Keith Davies and Clive Briant, Chemical Design Ltd. (Published July, 1995); A Database System for Combinatorial Synthesis Experiments—Craig James and David Weininger, Daylight Chemical Information Systems, Inc. (Published July, 1995); An Information Management Architecture for Combinatorial Chemistry, Keith Davies and Catherine White, Chemical Design Ltd. (Published July, 1995); Novel Software Tools for Addressing Chemical Diversity, R. S. Pearlman, Laboratory for Molecular Graphics and Theoretical Modeling, College of Pharmacy, University of Texas (Published June/July, 1996); Opportunities for Computational Chemists Afforded by the New Strategies in Drug Discovery: An Opinion, Yvonne Connolly Martin, Computer Assisted Molecular Design Project, Abbott Laboratories (Published June/July, 1996); Combinatorial Chemistry and Molecular Diversity Course at the University of Louisville: A Description, Arno F. Spatola, Department of Chemistry, University of Louisville (Published June/July, 1996); Chemically Generated Screening Libraries: Present and Future. Michael R. Pavia, Sphinx Pharmaceuticals, A Division of Eli Lilly (Published June/July, 1996); Chemical Strategies For Introducing Carbohydrate Molecular Diversity Into The Drug Discovery Process. Michael J. Sofia, Transcell Technologies Inc. (Published June/July, 1996); Data Management for Combinatorial Chemistry. Maryjo Zaborowski, Chiron Corporation and Sheila H. DeWitt, Parke-Davis Pharmaceutical Research, Division of Wamer-Lambert Company (Published November, 1995); and/or The Impact of High Throughput Organic Synthesis on R&D in Bio-Based Industries, John P. Devlin (Published March, 1996)_{.}

Selection protocols for isolating desired members of large libraries are known in the art. Selection protocols can involve phage display techniques. Such systems, in which diverse peptide sequences are displayed on the surface of filamentous bacteriophage have proven useful for creating libraries of antibody fragments (and the nucleotide sequences that encoding them) for *in vitro* selection and/or amplification of specific antibody fragments that bind a target antigen. Nucleotide sequences encoding the V_{H} and/or V_{L} regions are linked to gene fragments which encode leader signals that direct them to the periplasmic space of *E*. *coli* and, as a result, the resultant antibody fragments are displayed on the surface of the bacteriophage, typically as fusions to bacteriophage coat proteins (*e.g*., pIII and/or pVIII). Alternatively or additionally, antibody fragments are displayed externally on lambda phage capsids (phagebodies). An advantage of phage-based display systems is that, because they are biological systems, selected library members may be amplified simply by growing the phage containing the selected library member in bacterial cells. Furthermore, since the nucleotide sequence that encodes the polypeptide library member is contained on a phage and/or phagemid vector, sequencing, expression, and/or subsequent genetic manipulation is relatively straightforward.

Methods for the construction of bacteriophage antibody display libraries and/or lambda phage expression libraries are well known in the art (Kang et a/., 1991. Proc. Natl. Acad. Sci. USA., 88: 4363; Clackson et al., 1991, Nature, 352: 624; Lowman et al., 1991, Biochemistry, 30: 10832; Burton et al., 1991, Proc. Natl. Acad. Sci., USA, 88: 10134; Hoogenboom et al., 1991, Nucleic Acids Res, 19: 4133; Chang et al., 1991, J. Immunol., 147: 3610; Breitling et al., 1991, Gene, 104: 147; Hawkins et al., 1992, J. Immunol., 22: 867; Marks et al., 1992, J. Biol. Chem., 267: 16007; and Lerner et al., 1992, Science, 258: 1313). Such techniques may be modified if necessary for the expression generally of polypeptide libraries.

One approach has been the use of scFv phage-libraries (Bird et al., 1988, Science, 242: 423; Huston et al., 1988, Proc. Natl. Acad. Sci., USA, 85:5879; Chaudhary et al., 1990, Proc. Natl. Acad. Sci, USA, 87:1066; and Chiswell et al., 1992, Trends Biotech., 10:80). Various embodiments of scFv libraries displayed on bacteriophage coat proteins have been described. Refinements of phage display approaches are known, for example as described in PCT Publications WO 92/01047, WO 96/06213, and WO 97/08320).

Alternative library selection technologies include bacteriophage lambda expression systems, which may be screened directly as bacteriophage plaques and/or as colonies of lysogens, as previously described (Huse et al., 1989, Science, 246:1275; Caton et al., 1990, Proc. Natl. Acad. Sci., USA, 87; Mullinax et al., 1990, Proc. Natl. Acad. Sci., USA, 87:8095; and Persson et al., 1991, Proc. Natl. Acad. Sci., USA, 88: 2432) and are of use. These expression systems may be used to screen a large number of different members of a library, in the order of about 10⁶ or more.

Screening systems can rely, for example, on direct chemical synthesis of library members. One method involves the synthesis of peptides on a set of pins and/or rods, such as described in PCT Publication WO 84/03564.
A similar method involving peptide synthesis on beads, which forms a peptide library in which each bead is an individual library member, is described in U.S. Patent 4,631,211 and a related method is described in PCT Publication WO 92/00091. A significant improvement of the bead-based methods involves tagging each bead with a unique identifier tag, such as an oligonucleotide, so as to facilitate identification of the amino acid sequence of each library member. These improved bead-based methods are described in PCT Publication WO 93/06121.

Another chemical synthesis method involves the synthesis of arrays of peptides (or peptidomimetics) on a surface in a manner that places each distinct library member *(e.g.,* unique peptide sequence) at a discrete, predefined location in the array. The identity of each library member is determined by its spatial location in the array. The locations in the array where binding interactions between a predetermined molecule (*e.g*., a receptor) and reactive library members occur is determined, thereby identifying the sequences of the reactive library members on the basis of spatial location. These methods are described in U.S. Patent 5,143,854; PCT Publications WO 90/15070 and WO 92/10092; Fodor et al., 1991, Science, 251: 767; and Dower et al., 1991, Ann. Rep. Med. Chem., 26: 271.

Other systems for generating libraries of polypeptides or nucleotides involve the use of cell-free enzymatic machinery for the *in vitro* synthesis of the library members. In one method, RNA molecules are selected by alternate rounds of selection against a target ligand and PCR amplification (Tuerk et al., 1990, Science, 249: 505; and Ellington et al., 1990, Nature, 346: 818). A similar technique may be used to identify DNA sequences which bind a predetermined human transcription factor (Thiesen et al., 1990, Nucleic Acids Res., 18: 3203; Beaudry et al., 1992 Science, 257:635; and PCT Publications WO 92/05258 and WO 92/14843).
In a similar way, *in vitro* translation may be used to synthesize polypeptides as a method for generating large libraries. These methods which generally comprise stabilized polysome complexes, are described further in PCT Publications WO 88/08453, WO 90/05785, WO 90/07003, WO 91/02076, WO 91/05058, and WO 92/02536. Alternative display systems which are not phage-based, such as those disclosed in PCT Publications WO 95/22625 and WO 95/11922, use the polysomes to display polypeptides for selection.

One combinatorial approach in use is based on a strategy involving the synthesis of libraries containing a different structure on each particle of the solid phase support, interaction of the library with a soluble test substance, identification of the "bead" which interacts with the test substance, and determination of the structure carried by the identified "bead" (Lam et al., 1991, Nature, 354:82). An alternative to this approach is the sequential release of defined aliquots of the structures from the solid support, with subsequent determination of activity in solution, identification of the particle from which the active structure was released, and elucidation of its structure by direct sequencing (Salmon et al., 1993, Proc. Natl. Acad. Sci., USA, 90:11708)
and/or by reading its code (Kerr et al., 1993, J. Am. Chem. Soc., 115:2529; Nikolaiev et al., 1993, Pept. Res., 6:161; and Ohlmeyer et al., 1993, Proc. Natl. Acad. Sci., USA, 90:109221).

Soluble random combinatorial libraries may be synthesized using a simple principle for the generation of equimolar mixtures of peptides which was first described by Furka et al. (1988, Xth International Symposium on Medicinal Chemistry, Budapest 1988; Furka et al., 1988, 14th International Congress of Biochemistry, Prague 1988; and Furka et al., 1991, Int. J. Peptide Protein Res., 37:487).
The construction of soluble libraries for iterative screening has been described (Houghten et al., 1991, Nature, 354:84).
Lam *et al.* disclosed the novel and unexpectedly powerful technique of using insoluble random combinatorial libraries. Lam synthesized random combinatorial libraries on solid phase supports, so that each support had a test compound of uniform molecular structure, and screened the libraries without prior removal of the test compounds from the support by solid phase binding protocols (Lam et al., 1991, Nature, 354:82).

Special libraries called "diversity files" may be used to assess the specificity, reliability, and/or reproducibility of the new methods. Diversity files contain a large number of compounds (*e.g*., 1000 or more small molecules) representative of many classes of compounds that could potentially result in nonspecific detection in an assay. Diversity files are commercially available and/or can be assembled from individual substances that are commercially available.

The present invention provides for screening of libraries of candidate substances. Such libraries may be exposed to a test substance (*e.g*. PAK protein, PAK gene, and/or characteristic portion thereof) with or without FMRP, and the relevant assay(s), described in detail below, carried out. Such libraries may be used in the *in vitro, in cyto,* and/or *in vivo* assay(s) described in detail below.

### Protein and Peptide PAK Inhibitors

PAK inhibitors may comprise proteins and/or peptides. Peptides can be proteins having fewer than about 100 amino acids. Peptides can range from about 5 to about 100, from about 5 to about 50, from about 5 to about 40, from about 5 to about 35, from about 5 to about 30, from about 5 to about 25, or from about 20 to about 25 amino acids in length. A peptide sequence can be based on the sequence of a protein. A peptide sequence can be a random arrangement of amino acids. Peptides from panels of peptides comprising random sequences and/or sequences which have been varied consistently to provide a maximally diverse panel of peptides may be used.

Peptide inhibitors of protein kinases, such as myosin light chain kinase (MLCK) and/or CAM kinase, are well known in the art (Kemp et al., 1991, Methods in Enzymology, 201:287; Saitoh et al., 1987, J. Biol. Chem., 262:7796; Nakanishi et al., 1992, J. Biol. Chem., 267:2157; and Strauss et al., 1992, Am. J. Physiol, 262:C1437). Many protein kinases regulate themselves through intermolecular autoinhibition (e.g., Johnson et al., 1996, Cell, 85: 149; and Kemp et al., 1994, Trends in Biochem. Sci, 19:440), in which inhibition is achieved via the interaction of amino acid sequences within the kinase which act as pseudosubstrates which block the catalytic domain of the kinase. The kinase remains inhibited until a specific Ser, Thr, and/or Tyr residue(s) located in the pseudosubstrate is phosphorylated (autophosphorylation), and/or until interaction with an effector eliminates binding of the pseudosubstrate. In the case of PAK, autophosphorylation occurs following binding of Cdc42/Rac1 and the subsequent conformation changes due to this interaction. Regions around these phosphorylation sites are regulatory domains that bind at the substrate binding site, and autophosphorylation fully opens the catalytic site. Thus, peptide modulators of PAK may comprise peptides having at least one amino acid sequence corresponding to at least one of these regulatory domains, but with the phosphorylatable residue replaced by another amino acid that is not phosphorylatable, such that the peptide acts as a "dead-end" pseudosubstrate and modulates PAK activity. Amino acids used to replace the phosphorylatable residues may be Asp, Glu, and/or Ala. Since PAK cannot phosphorylate such peptides, they act as competitive inhibitors.

Candidate substances may comprise FMRP proteins and/or characteristic portions thereof. Accordingly, when applied to cells at high concentrations, *i.e.,* in excess of the amount of endogenous FMRP, the FMRP peptides may act as competitors and bind to endogenous PAK, thereby inhibiting binding, and/or phosphorylation of, endogenous FMRP. The skilled artisan would know how much of a competitive peptide inhibitor to add such that the peptide is added to cells and/or cell lysates "in excess."

Peptide modulators of PAK are synthesized based on PAK substrates, either exogenous *(e.g.* Myosin light chain kinase (MLCK), regulatory Myosin light chain (R-MLC), Myosins I heavy chain, Myosin II heavy chain, Myosin VI, Caldesmon, Desmin, Op 18/stathmin, Merlin, Filamin A, LIM kinase (LIMK), Ras, Raf, Mek, p47^{phox}, BAD, caspase 3, estrogen and/or progesterone receptors, RhoGEF, GEF-H1, NET 1, Gaz, phosphoglycerate mutase-B, RhoGDI, prolactin, p41^{Arc}, and/or Aurora-A), and/or intramolecular *(e.g.,* autophosphorylation and/or autoinhibitory sites). In certain embodiments, peptide modulators of PAK comprise peptides that compete with PAK's natural binding partners *(e.g.,* FMRP, Cdc42, and/or Rac1) for binding to PAK protein. In some embodiments, a peptide comprising the sequence Pro-Pro-Val-Ile-Ala-Pro-Arg-Pro-Glu-His-Thr-Lys-Ser-Val-Tyr-Thr-Arg-Ser (*i.e.* the Pro-rich PIX-interacting motif of PAK) disrupts the PIX-PAK interaction and reduces PAK autophosphorylation (Maruta et al., 2002, Methods Mol. Biol., 189:75). Peptide modulators of PAK may be non-phosphorylatable analogues and/or pseudosubstrates of PAK substrates.

Protein inhibitors of PAK comprise peptide mimetics. As used herein, the term "peptide mimetics" refers to structures which substitute for peptides in interactions with natural binding partners, receptors, and/or enzymes. The mimetic may possess affinity, efficacy, and/or substrate function. A peptide mimetic may exhibits function(s) of a particular peptide, without restriction of structure. Peptide mimetics may include amino acid residues and/or other chemical moieties which provide the desired functional characteristics. Peptide mimetics include analogs of structural motifs such as the PAK autophosphorylation site, the substrate binding site(s) of PAK, the binding site(s) of PAK's natural binding partners, and/or the FMRP binding site of PAK.

Protein inhibitors of PAK can comprise a FMRP protein, including mutants described herein, a peptide mimetic, a substance that binds to an FMRP protein, and/or characteristic portions thereof. A PAK inhibitor can comprise a PAK protein, including mutants described herein, a peptide mimetic, and/or an agent that binds to a PAK protein. Such agents can be identified by, for example, direct binding assays, such as two-hybrid assays, GST pulldowns, etc. described in detail below.

Inventive protein modulators of PAK (or characteristic portions thereof) may be produced, for example, by utilizing a host cell system engineered to express an inventive *PAK* modulator-encoding nucleic acid.

Any system may be used to produce protein inhibitors of PAK (and/or characteristic portions thereof), such as eggs, baculovirus, plant cells, fungal cells (e.g. yeast cells), insect cells (e.g. SFM cells, Sf21 cells, Sf9 cells, Schneider cells, S2 cells, etc.), avian cells (e.g. chicken embryo fibroblasts, etc.), mammalian cells (e.g. Chinese hamster ovary (CHO) cells, HeLa cells, Madin-Darby canine kidney (MDCK) cells, baby hamster kidney (BHK cells), NSO cells, MCF-7 cells, MDA-MB-438 cells, U87 cells, A172 cells, HL60 cells, A549 cells, SP10 cells, DOX cells, DG44 cells, HEK 293 cells, SHSY5Y, Jurkat cells, BCP-1 cells, COS cells, Vero cells (African green monkey kidney), GH3 cells, 9L cells, 3T3 cells, MC3T3 cells, C3H-10T1/2 cells, NIH-3T3 cells, C6/36 cells, etc.), and/or hybrid cells. Alternatively or additionally, protein inhibitors of PAK (and/or characteristic portions thereof) may be expressed in cells using recombinant techniques, such as through the use of an expression vector (Sambrook et al., Molecular Cloning: A Laboratory Manual, 3rd ed., Cold Spring Harbor Laboratory Press, Cold Spring Harbor, N.Y., 2001).

Protein inhibitors of PAK (and/or characteristic portions thereof) may be produced in the context of intact virus, whether otherwise wild type, attenuated, killed, etc. Protein inhibitors of PAK (and/or characteristic portions thereof) may be produced in the context of virus like particles. For example, virus may be grown in eggs, such as embryonated hen eggs, in which case the harvested material is typically allantoic fluid. Alternatively or additionally, virus may be derived from any method using tissue culture to grow the virus. Suitable cell substrates for growing virus include, for example, dog kidney cells such as MDCK and/or cells from a clone of MDCK, MDCK-like cells, monkey kidney cells such as AGMK cells including Vero cells, cultured epithelial cells as continuous cell lines, 293T cells, BK-21 cells, CV-1 cells, and/or any other cell type suitable for the production of virus *(e.g.,* cells available from ATCC, Rockville, Md.). Suitable cell substrates include human cells, such as MRC-5 cells. Suitable cell substrates are not limited to cell lines; for example primary cells such as chicken embryo fibroblasts are included.

Alternatively or additionally, protein inhibitors of PAK in accordance with the present invention may be readily prepared by standard, well-established techniques, such as solid-phase peptide synthesis (SPPS) as described by Stewart et al. (Solid Phase Peptide Synthesis, 2nd ed., Pierce Chemical Company, Rockford, IL, 1984); and as described by Bodanszky et al. (The Practice of Peptide Synthesis, Springer-Verlag, New York, NY, 1984).

It will be appreciated, of course, that protein inhibitors of PAK may incorporate amino acid residues which are modified without affecting activity. For example, the termini may be derivatized to include blocking groups, *i.e*. chemical substituents suitable to protect and/or stabilize the N- and/or C-termini from "undesirable degradation," a term meant to encompass any type of enzymatic, chemical, and/or biochemical breakdown of the protein at its termini which is likely to affect the function of the protein, *i.e*. sequential degradation of the compound at a terminal end thereof.

Blocking groups include protecting groups conventionally used in the art of protein chemistry which will not adversely affect the *in vivo* activities of the protein. For example, suitable N-terminal blocking groups may be introduced by alkylation and/or acylation of the N-terminus. Examples of suitable N-terminal blocking groups include C₁ -C₅ branched and/or unbranched alkyl groups, acyl groups such as formyl and/or acetyl groups, as well as substituted forms thereof, such as the acetamidomethyl (Acm) group. Desamino analogs of amino acids are useful N-terminal blocking groups may be coupled to the N-terminus of the protein and/or used in place of the N-terminal reside. Suitable C-terminal blocking groups, in which the carboxyl group of the C-terminus may or may not be incorporated, include esters, ketones and/or amides. Ester and/or ketone-forming alkyl groups (*e.g*. lower alkyl groups, such as methyl, ethyl and/or propyl) and/or amide-forming amino groups (*e.g.* primary amines (-NH₂), and/or mono- and/or di-alkylamino groups (*e.g*. methylamino, ethylamino, dimethylamino, diethylamino, methylethylamino, *etc*.) are examples of C-terminal blocking groups. Descarboxylated amino acid analogues such as agmatine are useful C-terminal blocking groups and may be either coupled to the protein's C-terminal residue and/or used in place of it. Further, it will be appreciated that the free amino and/or carboxyl groups at the termini may be removed altogether from the protein to yield desamino and/or descarboxylated forms thereof without adversely affecting protein activity.

Other modifications may be incorporated without adversely affecting the activity and these include, but are not limited to, substitution of one or more of the amino acids in the natural L-isomeric form with amino acids in the D-isomeric form. Thus, a protein may include one or more D-amino acid resides, and/or may comprise amino acids which are all in the D-form. Retro-inverso forms of proteins in accordance with the present invention are contemplated, for example, inverted proteins in which all amino acids are substituted with D-amino acid forms.

To ensure that a protein obtained from either chemical and/or biological synthetic techniques is the desired protein, analysis of the protein should be conducted. Such amino acid composition analysis may be conducted using high-resolution mass spectrometry to determine the molecular weight of the protein. Alternatively or additionally, the amino acid content of the protein may be confirmed by hydrolyzing the protein in aqueous acid and separating, identifying, and/or quantifying the components of the mixture using HPLC and/or an amino acid analyzer. Protein sequenators, which sequentially degrade the protein and identify the amino acids in order, may be used to determine definitely the sequence of the protein. Prior to its use, the protein is usually purified to remove contaminants. In this regard, it will be appreciated that the protein is often purified so as to meet the standards set out by the appropriate regulatory agencies. Any one of a number of a conventional purification procedures may be used to attain the required level of purity including, for example, reversed-phase high-pressure liquid chromatography (HPLC) using an alkylated silica column such as C₄-, C₈- and/or C₁₈- silica. A gradient mobile phase of increasing organic content is generally used to achieve purification, for example, acetonitrile in an aqueous buffer, usually containing a small amount of trifluoroacetic acid. Ion-exchange chromatography may be used to separate proteins based on charge.

Acid addition salts of peptides are described. Thus, a protein treated with an inorganic acid (e.g. hydrochloric, hydrobromic, sulfuric, nitric, phosphoric, etc.) and/or an organic acid (e.g. acetic, propionic, glycolic, pyruvic, oxalic, malic, malonic, succinic, maleic, fumaric, tataric, citric, benzoic, cinnamic, mandelic, methanesulfonic, ethanesulfonic, p-toluenesulfonic, salicyclic etc.) to provide a water-soluble salt of the protein, is suitable for use in the invention.

Sequence variants of protein inhibitors of PAK are included. Variants may differ from naturally occurring proteins by conservative amino acid sequence differences and/or by modifications which do not affect sequence. For example, conservative amino acid changes may be made, which although they alter the primary sequence of a protein, do not normally alter its function. In some embodiments, about 1 about 2, about 3, about 4, about 5, about 10, or more than about 10 conservative amino acid changes can be made without any adverse effect on protein function. In some embodiments, variants of protein modulators of PAK are approximately 60% identical, approximately 70% identical, approximately 80% identical, approximately 90% identical, approximately 95% identical, or greater than approximately 95% identical to a given protein modulator of PAK.

Modifications which do not normally alter primary amino acid sequence include *in vivo* and/or *in vitro* chemical derivatization of polypeptides, *e.g*., acetylation, and/or carboxylation. Modifications of glycosylation, *e.g*., those made by modifying the glycosylation patterns of a polypeptide during its synthesis, processing, and/or in further processing steps are contemplated, for example, by exposing the polypeptide to enzymes which affect glycosylation (*e.g*., mammalian glycosylating and/or deglycosylating enzymes). Sequences which have phosphorylated amino acid residues are contemplated (*e.g*., phosphotyrosine, phosphoserine, and/or phosphothreonine). In some embodiments, useful modifications include, *e.g*., terminal acetylation, amidation, lipidation, phosphorylation, glycosylation, acylation, farnesylation, sulfation, *etc.*

Proteins and/or characteristic portions thereof which have been modified using ordinary molecular biological techniques so as to improve their resistance to proteolytic degradation and/or to optimize solubility properties and/or to render them more suitable as a therapeutic agent are contemplated. Homologs of such proteins include those containing residues other than naturally occurring L-amino acids, *e.g.,* D-amino acids and/or non-naturally occurring synthetic amino acids. Proteins of the invention are not limited to products of any of the specific exemplary processes listed herein.

Substantially pure protein obtained as described herein may be purified by following known procedures for protein purification, wherein an immunological, enzymatic, and/or other assay is used to monitor purification at each stage in the procedure. Protein purification methods are well known in the art, and are described, for example in Deutscher et al. (ed., Guide to Protein Purification, Harcourt Brace Jovanovich, San Diego, CA, 1990).

In some embodiments, PAK inhibitors comprise antibodies (for example, monoclonal and/or polyclonal antibodies, single chain antibodies, chimeric antibodies, and/or CDR-grafted antibodies) which bind to PAK (*i.e.,* an anti-PAK antibody), a PAK modulator, a natural binding partner of PAK (*e.g*. FMRP), and/or an element upstream and/or downstream of PAK to inhibit and/or activate an interaction between PAK and/or a PAK modulator, natural binding partner, upstream element, and/or downstream element.

Antibodies generated in accordance with the present invention may include, but are not limited to, polyclonal, monoclonal, chimeric (*i.e*. "humanized"), and/or single chain (*e.g*. recombinant) antibodies. Antibodies of the present invention may include antibodies with reduced effector functions and/or bispecific molecules. Antibodies of the present invention may include Fab fragments and/or fragments produced by a Fab expression library. In the production of antibodies, screening for the desired antibody may be accomplished by techniques known in the art, *e.g*., ELISA (enzyme-linked immunosorbent assay).

Antibodies directed against proteins may be generated using methods that are well known in the art. U.S. Patent 5,436,157 discloses methods of raising antibodies to peptides. For the production of antibodies, various host animals, including but not limited to rabbits, mice, rats, sheep, goats, chickens, chicken eggs, and/or guinea pigs, can be immunized by injection with a polypeptide or peptide fragment thereof. To increase the immunological response, various adjuvants may be used depending on the host species, including but not limited to oil-emulsion and emulsifier-based adjuvants (*e.g*., complete Freund's Adjuvant, incomplete Freund's adjuvant, MF59 [Novartis], SAF, *etc.),* gel-type adjuvants *(e.g.,* aluminum hydroxide, aluminum phosphate, calcium phosphate, *etc.),* microbial adjuvants (*e.g*., immunomodulatory DNA sequences that include CpG motifs; endotoxins such as monophosphoryl lipid A; exotoxins such as cholera toxin, *E*. *coli* heat labile toxin, and pertussis toxin; muramyl dipeptide, BCG [bacille Calmette-Guerin], corynebacterium, *etc.*), particulate adjuvants (*e.g*., liposomes, biodegradable microspheres, saponins, *etc.*), synthetic adjuvants (*e.g*., nonionic block copolymers, muramyl peptide analogues, polyphosphazene, synthetic polynucleotides, *etc.*), polymers (*e.g*. polyphosphazenes, described in U.S. Patent 5,500,161, pluronic polyols; polyanions; *etc*.), QS21, squalene, tetrachlorodecaoxide, lysolecithin, peptides, keyhole limpet hemocyanins, dinitrophenol, *etc*., and/or combinations thereof.

The generation of polyclonal antibodies is accomplished by inoculating the desired animal with the antigen and/or isolating antibodies which specifically bind the antigen therefrom.

For the preparation of monoclonal antibodies, any technique which provides for the production of antibody molecules by continuous cell lines in culture may be utilized. For example, the hybridoma technique (Kohler et al., 1975, Nature, 256:495),
the triorna technique, the human B-cell hybridoma technique (Kozbor et al., 1983, Immunology Today, 4:72), and/or the EBV-hybridoma technique (Cole et al., 1985, in Monoclonal Antibodies and Cancer Therapy, Alan R. Liss, Inc.) may be employed to produce monoclonal antibodies. In some embodiments, monoclonal antibodies are produced in germ-free animals utilizing the technology described in PCT Publication WO 90/13678.

A nucleic acid encoding a monoclonal antibody obtained using the procedures described herein may be cloned and/or sequenced using technology which is available in the art (see, *e.g*., Wright et al., 1992, Critical Rev. Immunol., 12:125 and references therein).

The term "antibody" refers to any immunoglobulin, whether natural or wholly or partially synthetically produced and to derivatives thereof and characteristic portions thereof. An antibody may be monoclonal or polyclonal. An antibody may be a member of any immunoglobulin class, including any of the human classes: IgG, IgM, IgA, IgD, and IgE.

As used herein, an antibody fragment (*i.e*. characteristic portion of an antibody) refers to any derivative of an antibody which is less than full-length. In general, an antibody fragment retains at least a significant portion of the full-length antibody's specific binding ability. Examples of antibody fragments include, but are not limited to, Fab, Fab', F(ab')2, scFv, Fv, dsFv diabody, and Fd fragments.

An antibody fragment may be produced by any means. For example, an antibody fragment may be enzymatically or chemically produced by fragmentation of an intact antibody and/or it may be recombinantly produced from a gene encoding the partial antibody sequence. Alternatively or additionally, an antibody fragment may be wholly or partially synthetically produced. An antibody fragment may optionally comprise a single chain antibody fragment. Alternatively or additionally, an antibody fragment may comprise multiple chains which are linked together, for example, by disulfide linkages. An antibody fragment may optionally comprise a multimolecular complex. A functional antibody fragment will typically comprise at least about 50 amino acids and more typically will comprise at least about 200 amino acids.

Antibodies may include chimeric (e.g. "humanized") and single chain (recombinant) antibodies. Antibodies may have reduced effector functions and/or bispecific molecules. Antibodies may include fragments produced by a Fab expression library.

Single-chain Fvs (scFvs) are recombinant antibody fragments consisting of only the variable light chain (VL) and variable heavy chain (VH) covalently connected to one another by a polypeptide linker. Either VL or VH may comprise the NH2-terminal domain. The polypeptide linker may be of variable length and composition so long as the two variable domains are bridged without significant steric interference. Typically, linkers primarily comprise stretches of glycine and serine residues with some glutamic acid or lysine residues interspersed for solubility.

Diabodies are dimeric scFvs. Diabodies typically have shorter peptide linkers than most scFvs, and they often show a preference for associating as dimers.

An Fv fragment is an antibody fragment which consists of one VH and one VL domain held together by noncovalent interactions. The term "dsFv" as used herein refers to an Fv with an engineered intermolecular disulfide bond to stabilize the VH-VL pair.

A F(ab')2 fragment is an antibody fragment essentially equivalent to that obtained from immunoglobulins by digestion with an enzyme pepsin at pH 4.0-4.5. The fragment may be recombinantly produced.

A Fab' fragment is an antibody fragment essentially equivalent to that obtained by reduction of the disulfide bridge or bridges joining the two heavy chain pieces in the F(ab')2 fragment The Fab' fragment may be recombinantly produced.

A Fab fragment is an antibody fragment essentially equivalent to that obtained by digestion of immunoglobulins with an enzyme (*e.g*. papain). The Fab fragment may be recombinantly produced. The heavy chain segment of the Fab fragment is the Fd piece.

An antibody and/or characteristic portion thereof produced in a non-human subject may be recognized to varying degrees as foreign when the antibody is administered to a human subject and an immune response against the antibody may be generated in the subject One approach for minimizing and/or eliminating this problem is to produce chimeric and/or humanized antibody derivatives, *i.e.,* antibody molecules comprising portions which are derived from non-human antibodies and/or portions which are derived from human antibodies. Chimeric antibody molecules may include, for example, the variable region from an antibody of a mouse, rat, and/or other species, with human constant regions. A variety of approaches for making chimeric antibodies have been described (Morrison et al., 1985, Proc. Natl. Acad. Sci., USA, 81:6851; Takeda et al., 1985, Nature, 314:452; U.S. Patents 4,816,397 and 4,816,567; European publications EP 0173494 and EP 171496; and United Kingdom patent GB 2177096B). Humanized antibodies may have only the hypervariable domains of the variable region of non-human origin and have other parts of the variable region of the antibody, especially the conserved framework regions of the antigen-binding domain, of human origin. Such humanized antibodies may be made by any of several techniques known in the art, (e.g., Teng et al., 1983, Proc. Natl. Acad. Sci, USA, 80:7308; Kozbor et al., 1983, Immunology Today, 4:727; and Olsson et al., 1982, Meth. Enzymol, 92:3), and may be made according to the teachings of PCT Publication WO 92/06193 and/or European Publication EP 0239400. Humanized antibodies may be commercially produced by, for example, Scotgen Limited (Twickenham, Great Britain); Antitope (Cambridge, United Kingdom); Fusion Antibodies (Northern Ireland); and Genentech (South San Francisco, CA).

Techniques described for the production of single-chain (e.g. recombinant) antibodies (U.S. Patent 4,946,778) can be adapted to produce protein-specific single-chain antibodies. Techniques described for the construction of Fab expression libraries (e.g. Huse et al., 1989, Science, 246: 1275) can be utilized to allow rapid and/or easy identification of monoclonal Fab fragments possessing a desired specificity.

Antibody fragments which contain the idiotype of the antibody molecule can be generated by known techniques. For example, such fragments include but are not limited to a F(ab')₂ fragment which can be produced by pepsin digestion of the antibody molecule; Fab' fragments which can be generated by reducing the disulfide bridges of the F(ab')₂ fragment; Fab fragments which can be generated by treating the antibody molecule with papain and/or a reducing agent; and/or Fv fragments.

To generate a phage antibody library, a cDNA library is first obtained from mRNA which is isolated from cells (*e.g*., hybridomas) which express a desired protein (*e.g*., a desired antibody) to be expressed on the phage surface. cDNA copies of the mRNA are produced using reverse transcriptase. cDNA which specifies immunoglobulin fragments are obtained by PCR and the resulting DNA is cloned into a suitable bacteriophage vector to generate a bacteriophage DNA library comprising DNA specifying immunoglobulin genes. Procedures for making a bacteriophage library comprising heterologous DNA are well known in the art and are described in Sambrook et al. (Molecular Cloning: A Laboratory Manual, 3rd ed., Cold Spring Harbor Laboratory Press, Cold Spring Harbor, N.Y., 2001).

Bacteriophage encoding a desired antibody may be engineered such that the antibody is displayed on the surface thereof in such a manner that it is available for binding to its corresponding binding protein (*e.g*., the antigen against which the antibody is directed). Thus, when bacteriophage which express a specific antibody are incubated in the presence of a cell which expresses the corresponding antigen, the bacteriophage will bind to the cell. Bacteriophage which do not express the antibody will not bind to the cell. Such panning techniques are well known in the art.

Processes such as those described above, have been developed for the production of human antibodies using M13 bacteriophage display (Burton et al., 1994, Adv. Immunol., 57:191). Essentially, a cDNA library is generated from mRNA obtained from a population of antibody-producing cells. The mRNA encodes rearranged immunoglobulin genes and thus, the cDNA encodes the same. Amplified cDNA is cloned into M13 expression vectors creating a library of phage which express human Fab fragments on their surface. Phage which display the antibody of interest are selected by antigen binding and are propagated in bacteria to produce soluble human Fab immunoglobulin. Thus, in contrast to conventional monoclonal antibody synthesis, this procedure immortalizes DNA encoding human immunoglobulin rather than cells which express human immunoglobulin.

The procedures just presented describe the generation of phage which encode the Fab portion of an antibody molecule. However, the invention should not be construed to be limited solely to the generation of phage encoding Fab antibodies. Rather, phage which encode single chain antibodies (scFv/phage antibody libraries) are included in the invention. Fab molecules comprise the entire Ig light chain, *i.e*., they comprise the variable and constant region of the light chain, but include only the variable region and/or first constant region domain of the heavy chain. Single chain antibody molecules comprise a single chain of protein comprising the Ig Fv fragment. An Ig Fv fragment includes only the variable regions of the heavy and light chains of the antibody, having no constant region contained therein. Phage libraries comprising scFv DNA may be generated following the procedures described in Marks et al. (1991, J. Mol. Biol., 222:581).
Panning of phage so generated for the isolation of a desired antibody is conducted in a manner similar to that described for phage libraries comprising Fab DNA.

Synthetic phage display libraries in which the heavy and light chain variable regions may be synthesized such that they include nearly all possible specificities are included (Barbas, 1995, Nature Med., 1:837; and de Kruifet et ah, 1995, J. Mol. Biol, 248:97.

For use therapeutically, antibody preparations may be unable to fix complement and/or induce other effector functions. Complement fixation may be prevented by deletion of the Fc portion of the antibody, by using an antibody isotype which is not capable of fixing complement, and/or by using a complement fixing antibody in conjunction with a drug which inhibits complement fixation. Alternatively or additionally, amino acid residues within the Fc region which are involved in activating complement (see *e.g*., Tan et al., 1990, Proc. Natl. Acad. Sci., USA, 87:162; and Duncan et al., 1988, Nature, 332:738) may be mutated to reduce and/or eliminate the complement-activating ability of an intact antibody. Likewise, amino acids residues within the Fc region which are involved in binding of the Fc region to Fc receptors (see *e.g*., Canfield et al., 1991, J. Exp. Med., 173:1483; and Lund et al., 1991, J. Immunol., 147:2657) may be mutated to reduce and/or eliminate Fc receptor binding if an intact antibody is to be used.

Antibodies may be fragmented using conventional techniques and the fragments screened for utility in the same manner as for whole antibodies. For example, F(ab')₂ fragments can be generated by treating antibody with pepsin. The resulting F(ab')₂ fragment can be treated to reduce disulfide bridges to produce Fab' fragments.

### Nucleic Acid PAK Inhibitors

Nucleic acid inhibitors of PAK useful in the present invention include, but are not limited to, RNAi-inducing agents, for example, small interfering RNAs ("siRNAs"), short hairpin RNAs ("shRNAs"), etc.; antisense DNAs and/or RNAs; ribozymes; DNA for gene therapy; viral fragments, including viral DNA and/or RNA; DNA and/or RNA chimeras; mRNA; plasmids; cosmids; genomic DNA; cDNA; gene fragments; various structural forms of DNA including single-stranded DNA, double-stranded DNA, supercoiled DNA and/or triple-helical DNA; Z-DNA; etc.

The present invention includes nucleic acids which encode PAK inhibitor proteins and/or characteristic portions thereof. In some embodiments, the invention provides nucleic acids which are complementary to nucleic acids which encode PAK modulator protein and/or characteristic portions thereof.

In some embodiments, PAK inhibitors comprise nucleic acids, including but not limited to PAK-specific RNAi-inducing agents, antisense nucleic acids, and/or ribozymes.

The invention includes nucleic acids which hybridize to nucleic acids encoding PAK and/or PAK inhibitors and/or characteristic portions thereof. Such nucleic acids may be used, for example, as primers and/or as probes. To give but a few examples, such nucleic acids may be used as primers in polymerase chain reaction (PCR), as probes for hybridization (including *in situ* hybridization), and/or as primers for reverse transcription-PCR (RT-PCR).

RNA interference (RNAi) is an evolutionarily conserved process in which presence of an at least partly double-stranded RNA molecule in a eukaryotic cell leads to sequence-specific inhibition of gene expression. RNAi was originally described as a phenomenon in which the introduction of long dsRNA (typically hundreds of nucleotides) into a cell results in degradation of mRNA containing a region complementary to one strand of the dsRNA (U.S. Patent 6,506,559; and Fire et al., 1998, Nature, 391:806).
Subsequent studies in *D. melanogaster* showed that long dsRNAs are processed by an intracellular RNase III-like enzyme called Dicer into smaller dsRNAs primarily comprised of two approximately 21 nucleotide (nt) strands that form a 19 base pair duplex with 2 nt 3' overhangs at each end and 5'-phosphate and 3'-hydroxyl groups (see, *e.g*., PCT Publication WO 01/75164; U.S. Patent Application Publications 2002/0086356 and 2003/0108923; Zamore et al., 2000, Cell, 101:25; and Elbashir et al., 2001, Genes Dev., 15:188).

Short dsRNAs having structures such as this, referred to as siRNAs, silence expression of genes that include a region that is substantially complementary to one of the two strands. This strand is referred to as the "antisense" or "guide" strand, with the other strand often being referred to as the "sense" strand. The siRNA is incorporated into a ribonucleoprotein complex termed the RNA-induced silencing complex (RISC) that contains member(s) of the Argonaute protein family. Following association of the siRNA with RISC, a helicase activity unwinds the duplex, allowing an alternative duplex to form the guide strand and a target mRNA containing a portion substantially complementary to the guide strand. An endonuclease activity associated with the Argonaute protein(s) present in RISC is responsible for "slicing" the target mRNA, which is then further degraded by cellular machinery.

Considerable progress towards the practical application of RNAi was achieved with the discovery that exogenous introduction of siRNAs into mammalian cells can effectively reduce the expression of target genes in a sequence-specific manner via the mechanism described above. A typical siRNA structure includes an approximately 19 nucleotide double-stranded portion, comprising a guide strand and an antisense strand. Each strand has a 2 nt 3' overhang. Typically the guide strand of the siRNA is perfectly complementary to its target gene and mRNA transcript over at least 17 - 19 contiguous nucleotides, and typically the two strands of the siRNA are perfectly complementary to each other over the duplex portion. However, as will be appreciated by one of ordinary skill in the art, perfect complementarity is not required. Instead, one or more mismatches in the duplex formed by the guide strand and the target mRNA is often tolerated, particularly at certain positions, without reducing the silencing activity below useful levels. For example, there may be 1, 2, 3, or even more mismatches between the target mRNA and the guide strand (disregarding the overhangs). Thus, as used herein, two nucleic acid portions such as a guide strand (disregarding overhangs) and a portion of a target mRNA that are "substantially complementary" may be perfectly complementary (*i.e.,* they hybridize to one another to form a duplex in which each nucleotide is a member of a complementary base pair) or they may have a lesser degree of complementarity sufficient for hybridization to occur. One of ordinary skill in the art will appreciate that the two strands of the siRNA duplex need not be perfectly complementary. In some embodiments, at least 80%, at least 90%, or more of the nucleotides in the guide strand of an effective siRNA are complementary to the target mRNA over at least about 19 contiguous nucleotides. The effect of mismatches on silencing efficacy and the locations at which mismatches may most readily be tolerated are areas of active study (see, *e.g*., Reynolds et al., 2004, Nat. Biotechnol., 22:326).

It will be appreciated that molecules having the appropriate structure and degree of complementarity to a target gene will exhibit a range of different silencing efficiencies. A variety of additional design criteria have been developed to assist in the selection of effective siRNA sequences. Numerous software programs that can be used to choose siRNA sequences that are predicted to be particularly effective to silence a target gene of choice are available (see, *e.g.,* Yuan et al., 2004, Nucl. Acids. Res., 32:W130; and Santoyo et al., 2005, Bioinformatics, 21:1376).

As will be appreciated by one of ordinary skill in the art, RNAi may be effectively mediated by RNA molecules having a variety of structures that differ in one or more respects from that described above. For example, the length of the duplex can be varied (*e.g.*, from about 17-29 nucleotides); the overhangs need not be present and, if present, their length and the identity of the nucleotides in the overhangs can vary (though most commonly symmetric dTdT overhangs are employed in synthetic siRNAs).

Additional structures, referred to as short hairpin RNAs (shRNAs), are capable of mediating RNA interference. An shRNA is a single RNA strand that contains two complementary regions that hybridize to one another to form a double-stranded "stem," with the two complementary regions being connected by a single-stranded loop. shRNAs are processed intracellularly by Dicer to form an siRNA structure containing a guide strand and an antisense strand. While shRNAs can be delivered exogenously to cells, more typically intracellular synthesis of shRNA is achieved by introducing a plasmid or vector containing a promoter operably linked to a template for transcription of the shRNA into the cell, *e.g*., to create a stable cell line or transgenic organism.

While sequence-specific cleavage of target mRNA is currently the most widely used means of achieving gene silencing by exogenous delivery of RNAi-inducing entities to cells, additional mechanisms of sequence-specific silencing mediated by short RNA entities are known. For example, post-transcriptional gene silencing mediated by RNAi-inducing entities can occur by mechanisms involving translational repression. Certain endogenously expressed RNA molecules form hairpin structures containing an imperfect duplex portion in which the duplex is interrupted by one or more mismatches and/or bulges. These hairpin structures are processed intracellularly to yield single-stranded RNA species referred to as known as microRNAs (miRNAs), which mediate translational repression of a target transcript to which they hybridize with less than perfect complementarity. siRNA-like molecules designed to mimic the structure of miRNA precursors have been shown to result in translational repression of target genes when administered to mammalian cells.

Thus the exact mechanism by which an RNAi-inducing entity inhibits gene expression appears to depend, at least in part, on the structure of the duplex portion of the RNAi-inducing entity and/or the structure of the hybrid formed by one strand of the RNAi-inducing entity and a target transcript RNAi mechanisms and the structure of various RNA molecules known to mediate RNAi, *e.g*., siRNA, shRNA, miRNA and their precursors, have been extensively reviewed (see, *e.g*., Dykxhhorn et al., 2003, Nat. Rev. Mol. Cell Biol., 4:457; Hannon et al., 2004, Nature, 431:3761; and Meister et al., 2004, Nature, 431:343).
It is to be expected that future developments will reveal additional mechanisms by which RNAi may be achieved and will reveal additional effective short RNAi-inducing entities. Any currently known or subsequently discovered RNAi-inducing entities are within the scope of the present invention.

An RNAi-inducing entity that is delivered according to the methods of the invention and/or is present in a composition of the invention may be designed to silence any eukaryotic gene. The gene can be a mammalian gene, *e.g*., a human gene. The gene can be a wild type gene, a mutant gene, an allele of a polymorphic gene, *etc.* In certain embodiments, a PAK modulator is an RNAi-inducing entity that targets PAK expression. The following sequences may be used to design RNAi-inducing entities that target PAK expression in accordance with the guidelines described herein: GenBank Accession Numbers U24152, U24153, AF068864, AJ011855, AB040812, AF276893; and/or nucleic acids encoding the proteins of GenBank Accession Numbers AAA65441, AAA65442, AAC36097, NP_005875, CAA09820, CAC18720, BAA94194, NP_064553, AAF82800, Q9P286, BAA11844, AAC47094, AAB95646. RNAi-inducing entities can be used to induce isoform-specific suppression of PAK expression. RNAi-inducing entities can be used to induce suppression of all PAK isoforms.

A nucleic acid inhibitor of PAK can comprise an antisense molecule that binds to a translational start site, transcriptional start site, and/or splice junction. Antisense approaches involve the design of oligonucleotides (*e.g*. DNA and/or RNA) that are complementary to PAK mRNA. Antisense oligonucleotides typically bind to PAK mRNA and/or prevent translation. Alternatively or additionally, an antisense oligonucleotide may bind to DNA of a PAK gene, such as, for example, a regulatory element. An antisense oligonucleotide may or may not exhibit absolute complementarity. As used herein, a sequence "complementary" to a portion of a nucleic acid refers to a sequence having sufficient complementarity to be able to hybridize with the RNA and/or form a stable duplex. In the case of double-stranded antisense nucleic acids, a single strand of the duplex DNA may thus be tested and/or triplex formation may be assayed. The ability to hybridize will depend on the degree of complementarity and/or the length of the antisense nucleic acids. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of PAK mRNA (e.g., the 5' untranslated sequence up to and including the AUG initiation codon) may inhibit translation. Oligonucleotides that are complementary to the 3' untranslated sequences of PAK mRNA may inhibit translation (Wagner, 1994, Nature, 372:333). Therefore, oligonucleotides complementary to the 5' and/or 3' untranslated, non-coding regions of a PAK gene may be used in an antisense approach to inhibit translation of endogenous PAK mRNA. Oligonucleotides complementary to the 5' untranslated region of the mRNA may include the complement of the AUG start codon. Antisense oligonucleotides complementary to mRNA coding regions are generally less efficient inhibitors of translation but may be used in accordance with the invention. Whether designed to hybridize to any of the afore-mentioned regions of a PAK nucleic acid, an antisense nucleic acid described herein comprises at least about 6 nucleotides and may comprise about 6 to about 50 nucleotides. An antisense nucleic acid can comprise at least about 10 nucleotides, at least about 17 nucleotides, at least about 25 nucleotides, or at least about 50 nucleotides.

Ribozyme molecules designed to catalytically cleave PAK mRNA transcripts may be used to prevent translation of PAK mRNA and/or expression of PAK (see, e.g., PCT Publication WO 90/11364; and Sarver et al., 1990, Science, 247:1222). Ribozymes described herein may cleave mRNA at site specific recognition sequences in order to destroy PAK mRNAs. Alternatively, ribozymes may comprise hammerhead ribozymes, which cleave mRNAs at locations dictated by flanking regions that form complementary base pairs with the target mRNA. When hammerhead ribozymes are employed, the target mRNA may have the following sequence of two bases: 5'-UG-3'. The construction and/or production of hammerhead ribozymes is well known in the art and is described more fully in Haseloff et al. (1988, Nature, 334:585). There are numerous potential hammerhead ribozyme cleavage sites within the nucleotide sequence of human PAK cDNA. A ribozyme is engineered so that the cleavage recognition site is located near the 5' end of the PAK mRNA; i.e., to increase efficiency and/or minimize the intracellular accumulation of non-functional mRNA transcripts.

Ribozymes may comprise RNA endoribonucleases (hereinafter "Cech-type ribozymes"), such as the one which occurs naturally in *Tetrahymena thermophila* (known as the IVS, and/or L-19 IVS RNA) and which has been extensively described by Thomas Cech and collaborators (Zaug et al., 1984, Science, 224:574; Zaug et al., 1986, Science, 231:470; Zaug et al., 1986,. Nature, 324:429; PCT Publication WO 88/04300; and Been et al., 1986, Cell, 47:207).
The Cech-type ribozymes have an eight base pair active site which hybridizes to a target RNA sequence whereafter cleavage of the target RNA takes place.

Alternatively or additionally, endogenous PAK gene expression may be reduced by targeting deoxyribonucleotide sequences complementary to the regulatory region of the PAK gene (*i.e*., PAK promoter, enhancers, *etc*.) to form triple helical structures that prevent transcription of the PAK gene (see generally, Helene, 1991, Anticancer Drug Des., 6:569; Helene et al., 1992, Ann, N.Y. Acad. Sci, 660:27; and Maher, 1992, Bioassays, 14:807).

Nucleic acid molecules to be used in triple helix formation for the inhibition of transcription may be single-stranded and/or composed of deoxyribonucleotides. The base composition of these oligonucleotides may promote triple helix formation via Hoogsteen base pairing rules, which generally require sizable stretches of purines or pyrimidines to be present on one strand of a duplex. Nucleotide sequences may be pyrimidine-based, which will result in TAT and/or CGC triplets across the three associated strands of the resulting triple helix. Pyrimidine-rich molecules provide base complementarity to a purine-rich region of a single strand of the duplex in a parallel orientation to that strand. In some embodiments, nucleic acid molecules may be chosen that are purine-rich, for example, containing a stretch of G residues. These molecules will typically form a triple helix with a DNA duplex that is rich in GC pairs, in which the majority of the purine residues are located on a single stand of the targeted duplex, resulting in CGC triplets across the three strands in the triplex.

Alternatively or additionally, the potential sequences that can be targeted for triple helix formation may be increased by creating a so called "switchback" nucleic acid molecule. Switchback molecules are synthesized in an alternating 5' - 3', 3' - 5' manner, such that they base pair with first one strand of a duplex and then the other, eliminating the necessity for a sizable stretch of either purines or pyrimidines to be present on one strand of a duplex.

As discussed above, nucleic acid inhibitors of PAK can target PAK directly. However, it will be understood that nucleic acid inhibitors of PAK may target PAK directly, but instead, might target downstream effectors of PAK, upstream activators of PAK, and/or natural binding partners of PAK. For example, a PAK inhibitor can be an RNAi-inducing entity that targets one or more of the following: Myosin light chain kinase (MLCK), regulatory Myosin light chain (R-MLC), Myosins I heavy chain, myosin II heavy chain, Myosin VI, Caldesmon, Desmin, Op18/stathmin, Merlin, Filamin A, LIM kinase (LIMK), Ras, Raf, Mek, p47^{phox}, BAD, caspase 3, estrogen and/or progesterone receptors, RhoGEF, GEF-H1, NET1, Gαz, phosphoglycerate mutase-B, RhoGDI, prolactin, p41^{Arc}, Aurora-A (Bokoch et al., 2003, Annu. Rev. Biochem., 72:743; and Hofmann et al., 2004, J. Cell Sci., 117:4343), CIB; sphingolipids; G-proteinβ and/or γ subunits; PIX/COOL; GIT/PKL; Nef; Paxillin; NESH; SH3-containing proteins (e.g. Nick and/or Grb2); kinases (e.g. Akt, PDK1, PI 3-kinase/p85, Cdk5, Cdc2, Src kinases, Abl, and/or protein kinase A (PKA)); phosphatases (e.g. phosphatase PP2A, POPX1, and/or POPX2); PDK1, PDK2, PI3K, and/or NMDARs.

Nucleic acid inhibitors of PAK may target FMRP. A PAK inhibitor can be an RNAi-inducing entity that targets *FMRI* expression. The following sequences may be used to design RNAi-inducing entities that target *FMR1* expression in accordance with the guidelines described herein: GenBank Accession Numbers AF305881, L29074, U25165, U31501; and/or nucleic acids encoding the proteins of GenBank Accession Numbers AAG22045, AAB 18829, AAC50155, AAC50292.

A PAK inhibitor can be an RNAi-inducing entity, antisense oligonucleotide, ribozyme, and/or triple-helix inducing agent that targets one or more genes that have been shown to positively regulate expression of PAK and/or FMRP. For example, a PAK inhibitor can be an RNAi-inducing entity that targets transcription factors, translation factors, RNA processing factors, protein stabilizing factors, etc. that are involved in positive regulation of PAK and/or FMRP expression.

A candidate nucleic acid (e.g. RNAi-inducing agent, antisense oligonucleotide, ribozyme, and/or triple-helix inducing agent) can comprise an α-anomeric oligonucleotide. An α-anomeric oligonucleotide forms specific double-stranded hybrids with complementary RNA in which, contrary to the usual β-units, the strands run parallel to each other (Gautier et al., 1987, Nuc. Acids Res., 15:6625). The oligonucleotide is a 2'-O-methylribonucleotide (Inoue et al., 1987, Nuc. Acids Res., 15:6131) and/or a chimeric RNA-DNA analogue (Inoue et al., 1987, FEBS Lett, 215:327).

*In vitro* studies can be first performed to quantitate the ability of a candidate nucleic acid (e.g. RNAi-inducing agent, antisense oligonucleotide, ribozyme, and/or triple-helix inducing agent) to inhibit PAK gene expression. These studies may utilize controls that distinguish between gene inhibition due to the candidate nucleic acid and nonspecific biological effects of nucleic acids. These studies may compare levels of the target RNA and/or protein with that of an internal control RNA and/or protein. Results obtained using the candidate nucleic acid can be compared with those obtained using a control nucleic acid. A control nucleic acid can be of approximately the same length as the candidate nucleic acid, and/or the nucleotide sequence of the candidate nucleic acid differs from the control nucleic acid no more than is necessary to prevent specific hybridization to the target sequence.

A candidate nucleic acid (*e.g*. RNAi-inducing agent, antisense oligonucleotide, ribozyme, and/or triple-helix inducing agent) may be delivered to cells which express PAK *in vivo* and/or *in vitro.* A number of methods have been developed for delivering nucleic acids to cells. For example, nucleic acids may be injected directly into the tissue site. Alternatively or additionally, nucleic acids, designed to target desired cells (*e.g*., linked to peptides and/or antibodies that specifically bind receptors and/or antigens expressed on a target cell surface), may be administered systemically.

Since it may be difficult to achieve intracellular concentrations of a candidate nucleic acid (*e.g*. RNAi-inducing agent, antisense oligonucleotide, ribozyme, and/or triple-helix inducing agent) sufficient to inhibit gene expression of PAK, a recombinant DNA construct may be used in which the candidate nucleic acid is placed under the control of a strong pol III and/or pol II promoter. The use of such a construct to transfect, for example, target cells in a patient, may result in the transcription of sufficient amounts of single-stranded RNAs that will form complementary base pairs with endogenous PAK transcripts and thereby prevent translation of PAK mRNA. For example, a vector may be introduced *in vivo* such that it is taken up by a target cell and/or directs the transcription of a candidate nucleic acid. Such a vector may remain episomal and/or become chromosomally integrated, as long as it can be transcribed to produce the desired candidate nucleic acid. Such vectors may be constructed by recombinant DNA technology methods standard in the art. Vectors may be plasmid, viral, and/or others known in the art, used for replication and/or expression in mammalian cells. Expression of the sequence encoding the candidate nucleic acid may be by any promoter known in the art to act in mammalian (*e.g*. human) neuronal cells. Such promoters may be inducible and/or constitutive. Such promoters include, but are not limited to, the SV40 early promoter region (Bernoist et al., 1981, Nature, 290:304),
the promoter contained in the 31 long terminal repeat of Rous sarcoma virus (Yamamoto et al., 1980, Cell, 22:787), the herpes thymidine kinase promoter (Wagner et al., 1981, Proc. Natl. Acad. Sci., USA, 78:1441),
the promoter and/or regulatory sequences of *FMRI* gene, *etc.* Any type of plasmid, cosmid, YAC, and/or viral vector may be used to prepare the recombinant DNA construct which can be introduced directly into a tissue site.

A nucleic acid PAK inhibitor may comprise DNA, RNA, chimeric mixtures, derivatives, characteristic portions, and/or modified versions thereof. Nucleic acids of the present invention may be single-stranded and/or double-stranded. A nucleic acid may be modified at the base moiety, sugar moiety, and/or phosphate backbone, for example, to improve stability of the molecule, hybridization, etc. A nucleic acid may include other appended groups such agents facilitating transport across the cell membrane (see, e.g., Letsinger et al., 1989, Proc. Natl. Acad. Sci, USA, 86:6553; Lemaitre et al., 1987, Proc. Natl. Acad. Sci, USA, 84:648; and PCT Publication WO 88/09810).

Nucleic acid PAK inhibitors of the present invention (including RNAi-inducing agents, antisense oligonucleotides, ribozymes, triple-helix inducing agents, etc.) may be prepared according to any available technique including, but not limited to chemical synthesis, enzymatic synthesis, enzymatic or chemical cleavage of a longer precursor, etc. Methods of synthesizing RNAs are known in the art (see, e.g., Gait, MI. (ed.) Oligonucleotide synthesis: a practical approach, Oxford [Oxfordshire], Washington, DC: IRL Press, 1984; and Herdewijn, P. (ed.) Oligonucleotide synthesis: methods and applications, Methods in molecular biology, v. 288 (Clifton, NJ.) Totowa, NJ.: Humana Press, 2005).

Nucleic acid inhibitors of PAK may comprise naturally occurring nucleosides, modified nucleosides, naturally occurring nucleosides with hydrocarbon linkers (e.g., an alkylene) or a polyether linker (e.g., a PEG linker) inserted between one or more nucleosides, modified nucleosides with hydrocarbon or PEG linkers inserted between one or more nucleosides, or a combination of thereof. Nucleotides or modified nucleotides of a nucleic acid can be replaced with a hydrocarbon linker or a polyether linker provided that the binding affinity, selectivity, and/or other functional characteristics of the nucleic acid are not substantially reduced by the substitution.

It will be appreciated by those of ordinary skill in the art that nucleic acids in accordance with the present invention may comprise nucleotides entirely of the types found in naturally occurring nucleic acids, or may instead include one or more nucleotide analogs or have a structure that otherwise differs from that of a naturally occurring nucleic acid. U.S. Patents 6,403,779; 6,399,754; 6,225,460; 6,127,533; 6,031,086; 6,005,087; 5,977,089; and references therein disclose a wide variety of specific nucleotide analogs and modifications that may be used. See Crooke, S. (ed.) Antisense Drug Technology Principles, Strategies, and Applications (1st ed), Marcel Dekker; ISBN: 0824705661; 1st edition (2001) and references therein. For example, 2'-modifications include halo, alkoxy and allyloxy groups. In some embodiments, the 2'-OH group is replaced by a group selected from H, OR, R, halo, SH, SR₁, NH₂, NH_{R}, NR₂ or CN, wherein R is C₁-C₆ alkyl, alkenyl, or alkynyl, and halo is F, Cl, Br or I. Examples of modified linkages include phosphorothioate and 5'-N-phosphoramidite linkages.

Nucleic acids comprising a variety of different nucleotide analogs, modified backbones, or non-naturally occurring internucleoside linkages can be utilized in accordance with the present invention. Nucleic acids of the present invention may include natural nucleosides (i.e., adenosine, thymidine, guanosine, cytidine, uridine, deoxyadenosine, deoxythymidine, deoxyguanosine, and deoxycytidine) or modified nucleosides. Examples of modified nucleotides include base modified nucleoside (*e.g*., aracytidine, inosine, isoguanosine, nebularine, pseudouridine, 2,6-diaminopurine, 2-aminopurine, 2-thiothymidine, 3-deaza-5-azacytidine, 2'-deoxyuridine, 3-nitorpyrrole, 4-methylindole, 4-thiouridine, 4-thiothymidine, 2-aminoadenosine, 2-thiothymidine, 2-thiouridine, 5-bromocytidine, 5-iodouridine, inosine, 6-azauridine, 6-chloropurine, 7-deazaadenosine, 7-deazaguanosine, 8-azaadenosine, 8-azidoadenosine, benzimidazole, M1-methyladenosine, pyrrolo-pyrimidine, 2-amino-6-chloropurine, 3-methyl adenosine, 5-propynylcytidine, 5-propynyluridine, 5-bromouridine, 5-fluorouridine, 5-methylcytidine, 7-deazaadenosine, 7-deazaguanosine, 8-oxoadenosine, 8-oxoguanosine, O(6)-methylguanine, and 2-thiocytidine), chemically or biologically modified bases (*e.g.,* methylated bases), modified sugars (*e.g*., 2'-fluororibose, 2'-aminoribose, 2'-azidoribose, 2'-O-methylribose, L-enantiomeric nucleosides arabinose, and hexose), modified phosphate groups (*e.g*., phosphorothioates and 5'-N-phosphoramidite linkages), and combinations thereof. Natural and modified nucleotide monomers for the chemical synthesis of nucleic acids are readily available. In some cases, nucleic acids comprising such modifications display improved properties relative to nucleic acids consisting only of naturally occurring nucleotides. In some embodiments, nucleic acid modifications described herein are utilized to reduce and/or prevent digestion by nucleases (*e*.*g*. exonucleases, endonucleases, *etc*.). For example, the structure of a nucleic acid may be stabilized by including nucleotide analogs at the 3' end of one or both strands order to reduce digestion.

Modified nucleic acids need not be uniformly modified along the entire length of the molecule. Different nucleotide modifications and/or backbone structures may exist at various positions in the nucleic acid. One of ordinary skill in the art will appreciate that the nucleotide analogs or other modification(s) may be located at any position(s) of a nucleic acid such that the function of the nucleic acid is not substantially affected. The modified region may be at the 5'-end and/or the 3'-end of one or both strands. For example, modified nucleic acids in which approximately 1 to approximately 5 residues at the 5' and/or 3' end of either of both strands are nucleotide analogs and/or have a backbone modification have been employed. The modification may be a 5' or 3' terminal modification. One or both nucleic acid strands may comprise at least 50% unmodified nucleotides, at least 80% unmodified nucleotides, at least 90% unmodified nucleotides, or 100% unmodified nucleotides.

Nucleic acids in accordance with the present invention may, for example, comprise a modification to a sugar, nucleoside, or internucleoside linkage such as those described in U.S. Patent Publications 2003/0175950, 2004/0192626, 2004/0092470, 2005/0020525, and 2005/0032733. The present invention encompasses the use of any nucleic acid having any one or more of the modification described therein. For example, a number of terminal conjugates, *e.g*., lipids such as cholesterol, lithocholic acid, aluric acid, or long alkyl branched chains have been reported to improve cellular uptake. Analogs and modifications may be tested, *e.g*., using any appropriate assay known in the art. Nucleic acids in accordance with the present invention may comprise one or more non-natural nucleoside linkages. One or more internal nucleotides at the 3'-end, 5'-end, or both 3'- and 5'-ends of the nucleic acid are inverted to yield linkages such as a 3' - 3' linkage or a 5' - 5' linkage.

Nucleic acids comprising modified internucleoside linkages may be synthesized using reagents and/or methods that are well known in the art. For example, methods for synthesizing nucleic acids containing phosphonate phosphorothioate, phosphorodithioate, phosphoramidate methoxyethyl phosphoramidate, formacetal, thioformacetal, diisopropylsilyl, acetamidate, carbamate, dimethylene-sulfide (-CH₂-S-CH₂), diinethylene-sulfoxide (-CH₂-SO-CH₂), dimethylene-sulfone (-CH₂-SO₂-CH₂), 2'-O-alkyl, and/or 2'-deoxy 2'-fluoro phosphorothioate internucleoside linkages are well known in the art (Uhlmann et al., 1990, Chem. Rev., 90:543; Schneider et al., 1990, Tetrahedron Lett., 31:335; and references therein).

A nucleic acid may comprise phosphodiester linkages and/or modified linkages such as phosphotriester, phosphoramidate, siloxane, carbonate, carboxymethylester, acetamidate, carbamate, thioether, bridged phosphoramidate, bridged methylene phosphonate, bridged phosphoramidate, bridged phosphoramidate, bridged methylene phosphonate, phosphorothioate, methylphosphonate, phosphorodithioate, bridged phosphorothioate and/or sulfone linkages, and/or combinations of such linkages.

It will further be understood that, where a heterologous polypeptide is to be expressed in a host cell, it will often be desirable to utilize nucleic acid sequences encoding the polypeptide that have been adjusted to accommodate codon preferences of the host cell and/or to link the encoding sequences with regulatory elements active in the host cell. Nucleic acids of the present invention may be chosen for having codons, which may or may not be preferred for a particular expression system. For example, the nucleic acid may be one in which at least one codon, or in which at least 1%, at least 5%, at least 10%, or at least 20% of the codons have been altered such that the sequence is optimized for expression in the host cell.

Nucleic acid variants may be naturally occurring, such as allelic variants (same locus), homologs (different locus), and/or orthologs (different organism) and/or may be non naturally occurring. Non-naturally occurring variants may be made by mutagenesis techniques, including those applied to polynucleotides, cells, and/or organisms. Variants may contain nucleotide substitutions, deletions, inversions and insertions. Variation may occur in the coding and/or non-coding regions. Variations may produce conservative and/or non-conservative amino acid substitutions (as compared in the encoded product).

It is not intended that the present invention be limited by the nature of the nucleic acid employed. Nucleic acids in accordance with the present invention may not be synthetic and can be naturally-occurring entities that have been isolated from their natural environments. A nucleic acid may be a naturally-occurring and/or non- naturally-occurring (e.g. chemically-synthesized, artificial, man-made, etc.) nucleic acid.

Nucleic acids can be derived and/or obtained from natural sources (*e.g*. viral, fungal, bacterial, animal and/or plant sources). Alternatively or additionally, nucleic acids of the present invention may be prepared by any conventional means typically used to prepare nucleic acids in large quantity. For example, DNAs and/or RNAs may be chemically synthesized using commercially available reagents and/or synthesizers by methods that are well-known in the art (see, *e.g*., Gait, Oligonucleotide Synthesis: A Practical Approach, IRL Press, Oxford, England, 1985). RNAs may be produce in high yield via *in vitro* transcription using any of a variety of plasmids known in the art that are suitable for *in vitro* transcription, such as pSP72 (Promega Corporation, Madison, WI), pBluescript (Stratagene, La Jolla, CA), etc.

Nucleic acids may be purified by any suitable means, as are well known in the art. For example, the nucleic acids may be purified by reverse phase and/or ion exchange HPLC, size exclusion chromatography, and/or gel electrophoresis. Of course, the skilled artisan will recognize that the method of purification will depend in part on the size of the nucleic acid to be purified.

### Small Molecule PAK Inhibitors

In some embodiments, PAK inhibitors may comprise small molecules. For example, U.S. Patent Application 2004/007504 describes heterobicyclic pyrazole derivatives that inhibit PAK4 or PAK5 and may be used to treat Alzheimer's disease. Heterobicyclic pyrazole derivatives may be tested for use in treatment of FXS and/or other neurodevelopmental disorders.

In some embodiments, Emodin is a small molecule that can be used in the treatment of FXS and/or other neurodevelopmental disorders (see, *e.g.* Example 12). Emodin (also known as 1,3,8-Tri-hydroxy-6-methyl-anthra-quinone; 6-Methyl-1,3,8-tri-hydroxy-anthra-quinone; Emodol; and Frangula-emodin) is a member of a large family of naturally occurring anthraquinones and an active ingredient in Chinese herbal medicine. Emodin inhibits *HER-2*/*neu* tyrosine kinase activity (Zhang et al., 1999, Clin. Cancer Res., 5:343) and therefore has anti-cancer effects in *HER-2*/*neu-*overexpressing breast cancer cells (Jayasuriya et al., 1992, J. Nat. Prod., 55:696).
More recently, it has been demonstrated that 40 µM Emodin inhibits human cancer cell migration by interfering with the formation of an active Cdc42/Rac1 and PAK complex (Huang et al., 2005, Cell. Mol. Life Sci., 62:1167).

In some embodiments, OSU-03012 (Figures 6 and 7) is a small molecule that can be used in the treatment of FXS and/or other neurodevelopmental disorders (see, e.g. Example 12). OSU-03012 (Zhu et al., 2004, Cancer Res., 64:4309;
also known as 2-amino-N-{4-[5-(2-phenanthrenyl)-3-(trifluoromethyl)-1H-pyrazol-1-yl]-phenyl} acetamide) is derived from celecoxib. Celecoxib, sold by Pfizer under the brand name CELEBREX^{®}, is a nonsteroidal anti-inflammatory drug that works through the inhibition cyclooxygenase-2 (COX-2). Celecoxib can be used for the treatment of many conditions including but not limited to osteoarthritis, rheumatoid arthritis, analgesia, familial adenomatous polyposis, and cancer. Unlike celecoxib, its analog OSU-03012 is not a COX-2 inhibitor, but instead inhibits 3-phosphoinositide-dependent protein kinase-1 (PDK-1) and PAK (Porchia et al., 2007, Mol. Pharmacol., 72:1124)*.*
OSU-03012 directly inhibits PAK kinase activity. While not wishing to be bound by any one theory, inhibition probably occurs via competitive inhibition of ATP binding, with IC50 value of about 500 nM - 1 µM (Brader and Eccles, 2004, Tumori., 90:2).
OSU-03013 is another derivative of celecoxib that is structurally similar to OSU-03012. Although OSU-03013 has been shown to inhibit PAK activity, it is toxic *in vivo* and, therefore, not desirable for use as a therapeutic agent (Brader and Eccles, 2004, Tumori., 90:2).

### Activities of PAK Inhibitors

In some embodiments, PAK inhibitors target PAK directly. In some embodiments, PAK modulators target PAK indirectly. For example, PAK modulators might target downstream effectors of PAK, upstream effectors of PAK, and/or natural binding partners of PAK.

*PAKParticipates in Multiple Signaling Pathways*

PAK is known to participate in a variety of signaling pathways. To give but one example, Figure 8 (Klann and Dever, 2004, Nat. Rev. Neurosci., 5:931) shows how PAK fits within the context of the extracellular signal-regulated kinase (ERK) and phosphoinositide-3 kinase (PI3K) signaling pathways. PAK falls downstream of PDK1, PDK2, and PI3K. PAK falls upstream of Ras, Raf, MEK, ERK, and Mnk.

Although not pictured in Figure 8, PAK falls upstream of Myosin light chain kinase (MLCK), regulatory Myosin light chain (R-MLC), Myosins I heavy chain, myosin II heavy chain, Myosin VI, Caldesmon, Desmin, Op18/stathmin, Merlin, Filamin A, LIM kinase (LIMK), p47^{phox}, BAD, caspase 3, estrogen and/or progesterone receptors, RhoGEF, GEF-H1, NET1, Gαz, phosphoglycerate mutase-B, RhoGDI, prolactin, p41^{Arc}, and/or Aurora-A (Bokoch et al., 2003, Annu. Rev. Biochem., 72:743; and Hofmann et al., 2004, J. Cell Sci., 117:4343). The following factors bind to PAK in cells and may fall downstream of PAK: CIB; sphingolipids; G-protein β and/or γ subunits; PIX/COOL; GIT/PKL; Nef; Paxillin; NESH; SH3-containing proteins (*e.g*. Nck and/or Grb2); kinases (*e.g*. Akt, PDK1, PI 3-kinase/p85, Cdk5, Cdc2, Src kinases, Ab1, and/or protein kinase A (PKA)); and/or phosphatases (*e.g*. phosphatase PP2A, POPX1, and/or POPX2). Any of these factors may be targeted by PAK Modulators

The present invention encompasses the recognition that PAK modulators may indirectly modulate ERK activity. For example, considering that PAK functions upstream of ERK, aberrant activation of PAK (Figure 9) could cause in the constitutive activation of ERK pathway kinases. Down-regulation of negative regulators of the ERK pathway, such as mitogen-activated protein (MAP) kinase phosphatases (MKPs) and Sprouty proteins, would also result in the constitutive activation of ERK (see, *e.g.*, Kohno and Pouyssegur, 2006, Annals Med., 38:200).

As previously shown, *FMR1* KO mice exhibit enhanced basal ERK phosphorylation (and presumably activity) (Hou et al., 2006, Neuron, 51:41)
while ERK is phosphorylated and activated by PAK at least in non-neuronal cells (Eblen et al. 2002, Mol Cell Biol, 22:6023).
ERK is involved in regulation of spine morphology, synaptic plasticity and behaviors (Selcher et al., 2001, Learn Mem, 8:11; and Kelleher et al., 2004, Cell, 116:467),
therefore, it is possible that PAK inhibition returns the levels of phospho-ERK in *FMR1* KO mice to wild-type levels, thereby reversing phenotypes in *FMR1* KO mice.

As shown in Figure 9, the ERK signaling cascade, starting from Ras and going downstream, comprises Ras, Raf, MEK, and ERK. PAK has been shown to phosphorylate and activate both MEK-1 (Frost et al., 1997, EMBO J., 16:6426)
and Raf-1 (King et al., 1998, Nature, 396: 80; and Chaudhary et al., 2000, Curr. Biol., 10:551).

Thus, the present invention encompasses the recognition that inhibiting any member of the ERK signaling pathway (*e.g*. ERK, MEK, Ras, Raf) can be useful for treatment of FXS and/or other neurodevelopmental disorders. In one instance of the present disclosure A treating FXS and/or other neurodevelopmental disorders comprising administering a therapeutically effective amount of an erk pathway inhibitor (or pharmaceutical composition comprising an ERK pathway inhibitor) to a patient susceptible to, suffering from, and/or exhibiting one or more symptoms of FXS and/or other neurodevelopmental disorder. The general description of PAK modulators can be applied to ERK pathway inhibitors as well. To give but one example, ERK pathway inhibitors may be proteins, nucleic acids, small molecules, glycoproteins, proteoglycans, lipids, and/or carbohydrates, as described herein.

The signaling activity of Ras is dependent upon its association with the inner face of the plasma membrane, and this association is dependent upon a post-translational modification that places a farnesyl group on a cysteine residue near the C-terminus of Ras. This modification is catalyzed by farnesyltransferase (FTase). Accordingly, FTase inhibitors have been developed as anti-Ras compounds. In one instance, ERK pathway modulators may be FTase inhibitors. Exemplary FTase inhibitors include, but are not limited to, FTI-276, FTI-2148, L-739,750, and BZA-2B (Sebti and Der, 2006, Nat. Rev. Cancer, 3:945; Zhu et al., 2003, Curr. Opin. Investig. Drugs, 4:1428);
AZD-3409 (AstraZeneca; Lavelle, 1998, Exp. Opin. Invest. Drugs, 7:1015; Williams, 1998, Curr. Opin. Ther. Pat., 8:553; Singh and Lingham, 2002, Curr. Opin. Drug Discov. Develop., 5:225; Wilson et al., 2004, Eur. J. Cancer. Suppl., 2:Abstract 354; Smethurst et al., 2004, Eur. J. Cancer Suppl., 2:Abstract 376; Kelly et al., 2005, Proc. Amer. Assoc. Cancer Res., 46:Abstract 5962);
Tipifarnib (R-115777, Zarnestra^{™}; End et al., 2001, Cancer Res., 61:131*;* Cunningham et al., 2002, Proc. Amer. Soc. Clin. Oncol., 21:Abstract 502; Lancet et al., 2004, Blood, 104:Abstract 874; and Adjei et al., 2001, Proc. Amer. Soc. Clin. Oncol., 20:Abstract 320); and/or lonafarnib (Schering-Plough; Sch-66336, Sarasar^{™}; Yang et al., 2005, Proc. Amer. Soc. Clin. Oncol., 24:Abstract 5565; Long et al., 2005, Proc. Amer. Assoc. Cancer Res., 46:Abstract 5968; and Oh et al., 2005, Proc. Amer. Assoc. Cancer Res., 46:Abstract 5967).

ERK pathway modulators may be antisense inhibitors of Raf (see, *e.g.,* Smith et al., 2006, Curr. Topics Med. Chem., 6:1071; and Sridhar et al., 2005, Mol. Cancer Ther., 4:677; and references therein).
For example, the Raf antisense ISIS-5132 (ISIS Pharmaceuticals/Novartis) was designed as a 20-mer phosphorothioate oligonucleotide to inhibit translation of the Raf-1 RNA message into protein (Lau et al., 1998, Oncogene, 16:1899; Koller et al., 2000, Tr. Pharm. Sci., 21:142; Monia et al., 1996, Nature Med. 2:668; and Monia et al., 1996, Proc. Natl. Acad. Sci., USA 93:15481). To overcome degradation and improve intracellular delivery of ISIS-5132, a liposomal formulation (*i.e*. LErafAON) is under investigation.

ERK pathway modulators include Raf kinase destabilizers (see, e.g., Sridhar et al., 2005, Mol. Cancer Ther., 4:677 and references therein). Oxindoles include, but are not limited to, geldanamycin and MCP1.

ERK pathway modulators include small molecule inhibitors of MEK (see, e.g., Kohno and Pouyssegur, 2006, Annals Med., 38:200). Small molecule inhibitors of MEK include, but are not limited to, PD98059, U0126, PD184352 (CI-1040), PD0325901, and/or ARRY-14886.

ERK pathway modulators may be ureas (see, e.g., Smith et al., 2006, Curr. Topics Med. Chem., 6: 1071 and references therein).
Ureas may be derivitized in any way that makes them suitable as ERK pathway modulators in accordance with the present invention. In one instance, ureas may be, for example, biaryl ureas, diphenyl ureas, heteroaryl aryl ureas, quinolinyl ureas, isoquinolinyl ureas, pyridinyl ureas, *etc.* Exemplary ureas that may be utilized as ERK pathway modulators include, but are not limited to, 3-thienyl urea 7, isoxazole, pyrazole, and/or BAY 43-9006. BAY 43-9006 (also known as sorafenib), a novel biaryl urea, inhibits Raf-1 kinase activity *in vitro.* The crystal structure of the Raf/BAY 43-9006 complex revealed that the inhibitor binds in the adenosine triphosphate (ATP) pocket and interacts with residues of the kinase activation loop of Raf proteins. This interaction prevents the activation loop and the catalytic residues from adopting a conformation that is competent to bind and phosphorylate substrates (Wan et al., 2004, Cell, 116:855). Ureas that can be utilized as ERK pathway modulators may be urea derivatives (e.g. benzylic ureas, pyridopyrimidinones, heteroaryl-substituted diaryl ureas, annelated ureas, etc.).

Ureas include urea bioisosteres (see, e.g., Smith et al., 2006, Curr. Topics Med. Chem., 6: 1071 and references therein). Urea bioisosteres include, but are not limited to, compounds in which methylene and/or carbonyl moieties have been inserted into the urea functionality; glycinamides in which a methylene moiety has been introduced into the urea; oxamides having a second carbonyl group added to the urea; malonamides having both a carbonyl and a methylene moiety inserted into the urea functionality; diacyl hydrazines; 2-amino-benzimidazole derivatives; and/or pyrrolecarboxamides.

ERK pathway modulators include bis-aryl imidazoles and related structures (see, e.g., Smith et al., 2006, Curr. Topics Med. Chem., 6:1071 and references therein). In some embodiments, bis-aryl imidazoles include, but are not limited to, SB-203580, L-779,450, SB-590885, pyridyl-naphthyl-imidazoles, and/or derivatives thereof.

ERK pathway modulators include benzamides (see, e.g., Smith et al., 2006, Curr. Topics Med. Chem., 6:1071 and references therein). Benzamides include, but are not limited to, ZM-336372, imatinib, AMN-107, and/or derivatives thereof.

ERK pathway modulators include oxindoles (see, e.g., Smith et al., 2006, Curr. Topics Med. Chem., 6:1071 and references therein). In some embodiments, oxindoles include, but are not limited to, GW-5074.

ERK pathway modulators include PTK-787, thienopyrimidines, styrene (see, e.g., Smith et al., 2006, Curr. Topics Med. Chem., 6:1071 and references therein; incorporated herein by reference).

Alternatively or additionally, PAK is activated by PI3K signaling via PDK, and PI3K signaling is involved in protein synthesis (Hou and Klann, 2004, J. Neurosci., 24:6352). Thus, the present invention encompasses the recognition that, a PAK modulator may be an inhibitor of the PI3K/PDK pathway.

Biological Activities of PAK Inhibitors

Substances that are known to modulate serine/threonine kinase activity may accordingly modulate PAK kinase activity, including but not limited to Staurosporin, PD098059, Genistein, tyrphostin B42, HA1077, K252a, H-7: (1(5-isoquinoline-sulfonyl)-2-methylpiperazine), CEP-1347, etc., including analogs, derivatives, and/or mimetics thereof, that retain the ability to modulate PAK activity.

list Table 3 Kumar and Table 2 Eswaran

PAK inhibitors such as the ones described in Eswaren et al. (2007, Structure, 15:201) and Kumar et al. (2006, Nat. Rev. Cancer, 6:459) are used to treat FXS and/or other neurodevelopmental disorders. See, for example, Table 1 (adapted from Eswaren *et al*.) and Table 2 (adapted from Kumar *et al*.):

**Table 1. Exemplary PAK Modulators**

| | | **Tm Shift (°C)** | | | **% Activity at 10 µM** | | |
|---|---|---|---|---|---|---|---|
| **Compound Name** | **Chemical Structure** | **PAK4** | **PAK5** | **PAK6** | **PAK4** | **PAK5** | **PAK6** |
| Cdk1 Inhibitor | 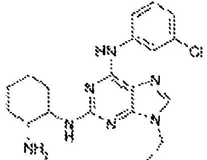 | 7.0 ± 1.5 | 7.1 ± 0.3 | 7.0 ± 0.3 | 56 | 12 | 23 |
| Cdk1/2 Inhibitor III | 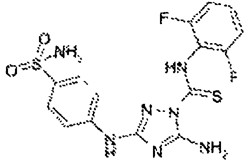 | 6.5 ± 0.2 | 5,6 ± 0.3 | 5.6 ± 0.8 | 62 | 7.0 | 18 |
| Purvalanol A | 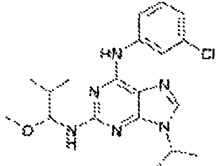 | 5.0 ± 0.3 | 4.5 ± 0.2 | 5.4 ± 0.5 | 20 | 24 | 48 |
| K252a | 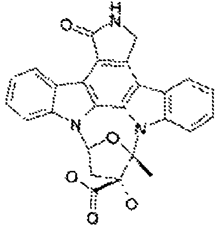 | 4.5 ± 0.3 | 5.9 ± 0.3 | 8,6 ± 1.0 | 16 | 22 | 16 |
| Staurospurine | 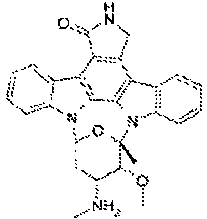 | 13.1 ± 1.5 | 12.5 ± 0.3 | 16.6 ± 0.5 | 0 | 0 | 0 |
| SU11652 | 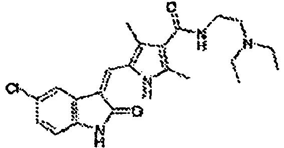 | 6.4 ± 1.2 | 5.3 ± 0.2 | 5.3 ± 0.3 | 34 | 43 | 75 |

**Table 2. Exemplary PAK1 Inhibitors**

| **Inhibitor** | **Mode of Action** | **References** |
|---|---|---|
| hPIP | Binds to regulatory domain and blocks kinase activity | Xia et al., 2001, Proc. Natl Acad. Sci., USA, 98:6174 |
| Merlin | Binds to PBD and inhibits recruitment to focal adhesions | Kissil et al., 2003, Mol. Cell, 12:841 |
| Nischarin | Binds to kinase domain and inhibits kinase activity | Alahari et al., 2004, EMBO J., 23:2777 |
| P35/CDK5 | Phosphorylates Pak and inhibits kinase activity | Nikolic et al., 1998, Nature, 395:194; and Rashid et al., 2001, J. Biol. Chem., 276:49043 |
| CDC2 | Phosphorylates xPAK2 and inhibits kinase activity | Cau et al, 2000, J. Biol. Chem., 275:2367 |
| p 110C | Binds to part of kinase domain and inhibits kinase activity | Chen et al., 2003, J. Biol. Chem., 278:20029 |
| POPX1, POPX2 | Dephosphorylation of T422 in kinase activation loop | Koh et al., 2002, Curr. Biol., 12:317 |
| CRIPak | Binds to regulatory domain and blocks kinase activity | Talukder et al, 2006, Oncogene, 25:1311 |
| PAK1 aa 89 -143 peptide | Competitive Inhibitor | Zhao et al., 1998, Mol. Cell. Biol., 18:2153 |
| CEP-1347 | Small molecule - ATP antagonist | Nheu et al., 2002, Cancer J., 8:328 |
| GL-2003 | ERK inhibitor that blocks Pak activation | Hirokawa et al., 2006, Cancer Lett. |

| | | |
|---|---|---|
| CDC2, cell division cycle 2; CDK5, cyclin-dependent kinase 5; CRIPak, cysteine-rich inhibitor of PAKI; ERK, extracellular signal regulated kinase; hPIP, human PAK/PLC interacting protein 1; PBD, p21-binding domain | | |

PAK inhibitors may affect the ability of PAK to interact with its natural binding partners, including but not limited to FMRP. In certain embodiments, such binding blocks the interaction between PAK and its natural binding partners (for example, the interaction between PAK and FMRP). In some embodiments, such binding promotes the interaction between PAK and its natural binding partners. However, a PAK inhibitor need not necessarily bind directly to a catalytic and/or binding site, and may bind, for example, to an adjacent site, such as an adjacent site in the PAK polypeptide. A PAK inhibitor may even bind to another substance (for example, a protein, lipid, carbohydrate, etc. which is complexed with the enzyme), so long as its binding modulates PAK activity.

The present invention encompasses the discovery that the integrity of the FMRP KH domains facilitates FMRP's interaction with PAK. PAK inhibitors may affect the integrity of one or more KH domains of FMRP and modulate its ability to bind to PAK.

A PAK inhibitor can bind to a natural binding partner of PAK and inhibits and/or promotes the interaction of PAK with its natural binding partner. In another aspect, a PAK inhibitor binds to PAK and inhibits and/or promotes the interaction of a natural binding partner of PAK with PAK. Some modulators that regulate PAK function in this manner have been described in the art. For example, U.S. Patent Application 2004/0208880 describes compounds that inhibit PAK1 function by modulating its interaction with dynein light chain 1/protein inhibitors of nitric oxide synthase (DLC1/PIN). U.S. Patent Application 2006/0172360 describes compounds that inhibit PAK4 by modulating its interaction with MKK7. U.S. Patent Application 2004/0091907 describes compounds that inhibit PAK4 by modulating its interaction with GEF/H1 (used to treat cancer). U.S. Patent Application 2002/0106690 describes inhibitors that function by altering the interaction between PAK and the beta subunit of G-protein coupled receptors. U.S. Patent Application 2006/0088897 describes substances that modulate PAK by altering the interaction between PAK and SH3 domain-containing proteins. U.S. Patents 6,013,500 and 6,667,168 describe agents that bind to the GTP -binding domain of PAK4 and prevent PAK4 from binding to GTP -binding proteins and agents that bind the PAK4 cdc42-binding domain and prevent PAK4 from interacting with cdc42.

A PAK inhibitor may bind to and/or compete for one or more sites on a relevant molecule, for example, a catalytic site and/or a binding site of PAK. The PAK inhibitor can interfere with and/or inhibit the binding of FMRP to PAK. The PAK inhibitor can compete for an FMRP-binding region of PAK. The PAK inhibitor can compete for a PAK- binding region of FMRP.

Where inhibitors of an enzyme such as PAK are concerned, an inhibitor may include a substrate of PAK kinase and/or characteristic portion thereof which is capable of binding to PAK. Alternatively or additionally, whole or portions of a substrate isolated from a biological source (e.g. purified from tissues and/or cells) and/or by chemical synthesis may be used to compete with the substrate for binding sites on the enzyme. Alternatively or additionally, an antibody capable of binding to PAK may be used. A PAK inhibitor may include a peptide or other small molecule which is capable of modulating the binding interaction.

PAK inhibitors include substances which bind to and/or block the kinase domain of PAK and/or the p21-binding domain of PAK, and/or the autophosphorylation sites of PAK. PAK inhibitors can be short peptides comprising sequences of PAK that dominant-negative activity (Kiosses et al., 2002, Circ. Res., 90:697). Such peptides may not block PAK kinase activity *per se,* but may displace PAK from sites of action within the cell, which may indirectly inhibit PAK activity.

PAK inhibitors may function to alter the ability of PAK to phosphorylate its substrates. For example, U.S. Patent 6,383,734 describes agents that inhibit PAK function by inhibiting the ability of PAK to phosphorylate Raf. U.S. Patents 6,013,500 and 6,667,168 describe agents that block the ATP -binding domain of PAK4 in order to inhibit the kinase function of PAK4.

PAK inhibitors may function by altering the activity and/or expression of PAK activators. For example, U.S. Patent 6,046,224 describes agents that inhibit PAK function by blocking 12(S)HETE receptors. 12(S)HETE stimulates PAK activity, so blocking 12(S)HETE function indirectly inhibits PAK function.

Inventive PAK inhibitors may comprise phosphatases that inhibit PAK function by removing phosphate groups from targets that are phosphorylated by PAK kinase. Alternatively or additionally, inventive PAK inhibitors may comprise kinases that indirectly activate PAK function by phosphorylating targets that are. phosphorylated by PAK kinase.

PAK inhibitors may comprise the autoinhibitory domain of PAK, which may block and/or reduce PAK activity. The candidate substance may be an autoinhibitory region of PAK protein, such as a peptide comprising at least 25, 50, 75, 100, 125, or 150 contiguous amino acids of PAK. The protein can include at least 25, 50, 75, 100, 125, 150, 200, or 300 of the N- terminal amino acids of PAK protein. PAK inhibitors may comprise a dominant-negative PAK protein (e.g., a mutant and/or a characteristic portion of PAK protein).

PAK inhibitors can function to modulate the expression, stability, and/or cellular levels of PAK. For example, U.S. Patent Applications 2004/0102326 (PAK1), 2002/0142325 (PAK2), 2004/0009935 (PAK2), and 2005/0191672 (PAK4) describe nucleotide inhibitors of PAK expression that target PAK mRNA for degradation.

Alternatively or additionally, inventive PAK inhibitors affect PAK levels by increasing and/or decreasing transcription and/or translation of PAK, PAK substrates, and/or natural binding partners of PAK. PAK inhibitors may affect RNA and/or protein half-life, for example, by directly affecting mRNA and/or protein stability. Inventive PAK inhibitors can cause the mRNA and/or protein to be more and/or less accessible and/or susceptible to nucleases, proteases, and/or the proteasome.

Inventive PAK inhibitors can affect the processing of mRNAs encoding PAK, PAK substrates, and/or natural binding partners of PAK. For example, PAK modulators may function at the level of pre-mRNA splicing, 5' end formation (e.g. capping), 3' end processing (e.g. cleavage and/or polyadenylation), nuclear export, and/or association with the translational machinery and/or ribosomes in the cytoplasm.

Inventive PAK inhibitors can affect translational control and/or post-translational modification of PAK, PAK substrates, and/or natural binding partners of PAK. For example, PAK inhibitors may function at the level of translation initiation, elongation, termination, and/or recycling. In some embodiments, PAK modulators may function at the step of protein folding into secondary, tertiary, and/or quaternary structures. Alternatively or additionally, PAK modulators may function at the level of intracellular transport (e.g. ER to Golgi transport, intra-Golgi transport, Golgi to plasma membrane transport, and/or secretion from the cell). PAK inhibitors may function at the level of post-translational modification (e.g. cleavage of signal sequences and/or the addition of entities such as methyl groups, phosphates, glycan moieties, etc.).

A PAK inhibitor can be purified and/or unpurified synthetic organic molecule and/or naturally occurring organic molecule.

Inventive PAK inhibitors may cause the level of PAK mRNA and/or protein, an activity of PAK protein, the half-life of PAK mRNA and/or protein, the binding of PAK mRNA and/or protein to its natural binding partners, and/or the level and/or activity of a substance that phosphorylates a PAK kinase to decrease by at least about 5%, at least about 10%, at least about 20%, at least about 30%, at least about 40%, at least about 50%, at least about 60%, at least about 80%, at least about 90%, at least about 95%, or substantially 100%.

PAK inhibitors may be used alone and/or in conjunction with other substances which affect PAK activity. Additional examples of PAK inhibitors will be apparent to the skilled person.

### Identification and/or characterization of PAK inhibitors,

The present invention provides methods of identifying PAK inhibitors useful for treating neurodevelopmental disorders. Inventive methods screen for novel PAK inhibitors by identifying substances that improve and/or treat the symptoms of FXS and/or other neurodevelopmental disorders. In some embodiments, inventive methods identify novel PAK inhibitors by identifying substances that affect PAK's ability to interact with its natural binding partners (e.g. FMRP). In some embodiments, inventive methods identify novel PAK inhibitors by identifying substances that inhibit PAK kinase activity. In some embodiments, inventive methods identify PAK inhibitors by identifying substances that inhibit PAK expression and/or levels.

The inventive methods can identify substances that are known to have a particular function, but were not previously known to function as PAK modulators. The inventive methods can identify substances that have been identified and/or synthesized, but have not been attributed any particular function. The inventive methods can identify novel substances that have never been identified and/or synthesized before.

As used herein, the term "test substance" refers to (1) a PAK protein, a nucleic acid encoding PAK, and/or homolog, portion, variant, mutant, and/or derivative thereof; (2) a natural binding partner of PAK, a nucleic acid encoding a natural binding partner of PAK, and/or a homolog, portion, variant, mutant, and/or derivative thereof; (3) an FMRP protein, a nucleic acid encoding FMRP, and/or a homolog, portion, variant, mutant, and/or derivative thereof; and/or (4) a substrate of PAK kinase, a nucleic acid encoding a substrate of PAK, and/or a homolog, portion, variant, mutant, and/or derivative thereof. In some embodiments, a test substance is a protein and/or characteristic portion thereof comprising a FMRP-binding portion of PAK. In some embodiments, a test substance is a protein and/or characterstic portion thereof comprising a PAK-binding portion of FMRP.

The efficacy of a candidate substance may be assessed by generating dose response curves from data obtained using various concentrations of the candidate substance. Moreover, a control assay may be performed to provide a baseline for comparison. In the control assay, the assay is performed in the absence of a candidate substance.

The inventive PAK inhibitors inhibit PAK activity by at least about 10%, at least about 20%, at least about 30%, at least about 40%, at least about 50%, at least about 60%, at least about 70%, at least about 80%, at least about 90%, or greater than about 90% as compared with the activity observed under otherwise identical conditions lacking a candidate substance.

It will, of course, be understood that all screening methods of the present invention are useful in themselves notwithstanding the fact that effective PAK inhibitors may not be found. The invention provides methods for screening for candidate PAK inhibitors, not solely methods of finding them.

### Screening

Screening for PAK inhibitors can be employed. High throughput screening for PAK inhibitors can be employed. Such screening can identify substances that bind to PAK. Typically, large numbers of candidate substances are immobilized on a solid substrate. Immobilized candidate substances are contacted with PAK and washed. Bound PAK is then detected by methods well known in the art.

Using high throughput assays, it is possible to screen up to several thousand candidate substances in a single day. Each well of a microtiter plate can be used to run a separate assay against a selected candidate substance, or, if concentration and/or incubation time effects are to be observed, every 5 - 10 wells can test a single candidate substance. Thus, a single standard microtiter plate can assay up to 96 candidate substances. If 1536 well plates are used, then a single plate can assay up to 1536 candidate substances. It is possible to assay many plates per day; assay screens for up to about 6,000, about 20,000, about 50,000, or more than about 100,000 different candidate substances are possible using high throughput systems in accordance with the present invention.

For solid state reactions, a candidate substance may be bound to the solid state component, directly or indirectly, via covalent and/or non covalent linkage *e.g*., via a tag. A tag may comprise any of a variety of components. In general, a substance which binds the tag (a tag binder) is fixed to a solid support, and the tagged candidate substance is attached to the solid support by interaction of the tag and/or the tag binder.

A number of tags and/or tag binders may be used, based upon known molecular interactions well described in the literature. For example, where a tag has a natural binder, for example, biotin, protein A, and/or protein G, it may be used in conjunction with appropriate tag binders (avidin, streptavidin, neutravidin, the Fc region of an immunoglobulin, *etc*.). Antibodies to molecules with natural binders such as biotin and/or appropriate tag binders are widely available (Sigma Immunochemicals, St. Louis, MO).

Similarly, any haptenic and/or antigenic compound may be used in combination with an appropriate antibody to form a tag/tag binder pair. Thousands of specific antibodies are commercially available and many additional antibodies are described in the literature. For example, in one common configuration, the tag is a first antibody and the tag binder is a second antibody which recognizes the first antibody. In addition to antibody-antigen interactions, receptor-ligand interactions are appropriate as tag and/or tag-binder pairs, including but not limited to transferrin, c-kit, viral receptor ligands, cytokine receptors, chemokine receptors, interleukin receptors, immunoglobulin receptors and/or antibodies, the cadherin family, the integrin family, the selectin family, *etc.* (see, *e.g*., Pigott et al., The Adhesion Molecule Facts Book I, 1993). Similarly, toxins and/or venoms; viral epitopes; hormones (*e.g*. opiates, steroids, *etc*.); intracellular receptors (*e.g*. which mediate the effects of various small ligands, including steroids, thyroid hormone, retinoids, vitamin D, and/or peptides); drugs; lectins; carbohydrates; nucleic acids (linear and/or cyclic polymer configurafions); proteins; phospholipids; and/or antibodies may interact with various cell receptors.

Synthetic polymers, such as polyurethanes, polyesters, polycarbonates, polyureas, polyamides, polyethyleneimines, polyarylene sulfides, polysiloxanes, polyimides, and/or polyacetates may form appropriate tags and/or tag binders. Many other tag/tag binder pairs are useful in assay systems described herein, as would be apparent to one skilled in the art.

Common linkers such as peptides, polyethers, and the like may serve as tags and may include polypeptide sequences, such as poly-Gly sequences of between about 5 and 200 amino acids. Such flexible linkers are known to persons of skill in the art. For example, poly(ethelyne glycol) linkers are available from Shearwater Polymers, Inc. (Huntsville, AL). These linkers optionally have amide linkages, sulfhydryl linkages, and/or heterofunctional linkages.

Tag binders are fixed to solid substrates using any of a variety of methods currently available. Solid substrates are commonly derivatized and/or functionalized by exposing all and/or a portion of the substrate to a chemical reagent which fixes a chemical group to the surface which is reactive with a portion of the tag binder. For example, groups which are suitable for attachment to a longer chain portion include amines, hydroxyl, thiol, and/or carboxyl groups. Aminoalkylsilanes and/or hydroxyalkylsilanes may be used to functionalize a variety of surfaces, such as glass surfaces. The construction of such solid phase biopolymer arrays is well described in the literature (see, *e.g*., Merrifield, 1963, J. Am. Chem. Soc. , 85:2149, describing solid phase synthesis of, *e.g.,* peptides; Geysen et al., 1987, J. Immun. Meth., 102:259, describing synthesis of solid phase components on pins; Frank et al., 1988, Tetrahedron, 44:6031, describing synthesis of various peptide sequences on cellulose disks; Fodor et al., 1991, Science, 251:767; Sheldon et al., 1993, Clinical Chemistry, 39(4):718; and Kozal et al., 1996, Nature Medicine, 2:753; all describing arrays of biopolymers fixed to solid substrates).
Non-chemical approaches for fixing tag binders to substrates include other common methods, such as heat, cross-linking by ultraviolet radiation, and the like.

### In Vitro Assays

*In vitro* assays can often be run quickly and/or in large numbers, thereby increasing the amount of information obtainable in a short period of time. A variety of vessels may be used to run the assays, including test tubes, plates, dishes, microtiter plates, and/or other surfaces such as dipsticks and/or beads. Some *in vitro* and *in cyto* assays have been described, for example, in PCT Publication WO 06/029337).

The present invention provides *in vitro* methods for screening for PAK inhibitors. For example, methods may comprise steps of: (1) providing a test substance (e.g. PAK protein, PAK gene, and/or characteristic portion thereof); (2) providing at least one candidate substance; and (3) measuring and/or detecting the influence of the candidate substance(s) on the test substance.

In some embodiments, a test substance (*e.g*. PAK protein, PAK gene, and/or characteristic portion thereof) is provided and brought directly and/or indirectly into contact with a candidate substance (*e.g*. in the form of a library). Then, the influence of the candidate substance on the test substance is detected and/or measured. Thereafter, suitable PAK inhibitors may be isolated and/or analyzed. For the screening of libraries, the use of high-throughput assays are contemplated and described herein.

*In vitro* assays can comprise binding assays. Binding of a candidate substance to a test substance (*e.g*. PAK protein, PAK gene, and/or characteristic portion thereof) may, in and of itself, be inhibitory, due to steric, allosteric, and/or charge-charge interactions. The test substance may be free in solution, fixed to a support, and/or expressed in and/or on the surface of a cell. The test substance and/or the candidate substance may be labeled, thereby permitting detection of binding. The test substance is frequently the labeled species, decreasing the chance that the labeling will interfere with and/or enhance binding. Competitive binding formats may be performed in which one of the substances is labeled, and one may measure the amount of free label versus bound label to determine the effects on binding.

Binding assays can involve, for example, exposing a test substance to a candidate substance and detecting binding between the test substance and the candidate substance. A binding assay may be conducted *in vitro* (*e.g.* in a test tube, comprising substantially only the components mentioned; in cell-free extracts; and/or in substantially purified components). Alternatively or additionally, binding assays may be conducted *in cyto* and/or *in vivo* (*e.g.* within a cell, tissue, organ, and/or organism; described in further detail below).

At least one candidate substance can be contacted with a test substance (e.g. PAK protein, PAK gene, and/or characteristic portion thereof) and an effect detected. A candidate substance can be contacted with PAK protein, and binding to PAK protein is tested. An assay may involve contacting a candidate substance with a characteristic portion of PAK protein, including but not limited to a FMRP-binding portion of PAK. Binding of the candidate substance to the PAK peptide is detected. It will be appreciated that fragments, portions, homologs, variants, and/or derivatives of PAK may be employed, provided that they comprise FMRP binding activity.

Assays may involve providing a test substance (e.g. immobilized on a solid support), and a non-immobilized candidate substance. The extent to which the test substance and candidate substance bind to one another is determined. Alternatively, the candidate substance may be immobilized and the test substance non-immobilized. Such assays may be used to identify candidate substances capable of binding to PAK and/or fragments, portions, homologs, variants, and/or derivatives thereof.

An antibody that recognizes the test substance (e.g. an α-PAK antibody) can be immobilized to a solid support (e.g. Protein-A beads). The antibody is contacted with the test substance, which binds to the immobilized antibody. The resulting complex is then brought into contact with the candidate substance (purified protein, cellular extract, combinatorial library, *etc*.). If the candidate substance interacts with the test substance, the candidate substance will become indirectly immobilized to the solid support. Presence of the candidate substance on the solid support can be assayed by any standard technique known in the art (including, but not limited to, western blotting). This type of assay is known in the art as an "immunoprecipitation" assay.

A test substance (e.g. PAK protein, PAK gene, and/or characteristic portion thereof) can be immobilized on a solid support (e.g. agarose beads). The test substance can be expressed as a GST-fusion protein in bacteria, yeast, insect cells, and/or higher eukaryotic cell line and/or purified from crude cell extracts using glutathione-agarose beads. As a control, binding of the candidate substance, which is not a GST-fusion protein, to the immobilized PAK protein is determined in the absence of PAK protein. The binding of the candidate substance to the immobilized PAK protein is then determined. This type of assay is known in the art as a "GST pulldown" assay. Alternatively or additionally, the candidate substance may be immobilized and the test substance non-immobilized.

It is possible to perform this type of assay using different affinity purification systems for immobilizing one of the components, for example Ni-NTA agarose- and/or histidine-tagged components.

Binding of a test substance to the candidate substance may be determined by a variety of methods well-known in the art. For example, the non-immobilized component may be labeled (with for example, a radioactive label, an epitope tag, and/or an enzyme-antibody conjugate). Alternatively or additionally, binding may be determined by immunological detection techniques. For example, the reaction mixture may be subjected to Western blotting and the blot probed with an antibody that detects the non-immobilized component. Alternatively or additionally, enzyme linked immunosorbent assay (ELISA) may be utilized to assay for binding.

Screening methods of the present invention can comprise: (1) obtaining a candidate substance; (2) contacting the candidate substance with PAK (and/or characteristic portion thereof) and FMRP; and (3) detecting inhibition and/or activation of binding between PAK and FMRP in the presence and/or absence of the candidate substance.

Screening methods of the present invention can comprise: (1) obtaining a candidate substance; and (2) contacting the candidate substance with a preformed PAK (and/or characteristic portion thereof)-FMRP complex; and (3) determining whether the candidate substance affects the PAK (and/or characteristic portion thereof)-FMRP complex.

In some embodiments, screening methods of the present invention comprise (1) obtaining a candidate substance; (2) contacting the candidate substance with PAK (and/or characteristic portion thereof) and a natural binding partner; and (3) detecting whether the candidate substance can compete with the binding interaction between PAK (and/or characteristic portion thereof) and the natural binding partner.

In some embodiments, a candidate substance is determined to be a PAK inhibitor if administering the candidate substance to PAK and the natural binding partner results in decreased binding between PAK and the natural binding partner. A candidate substance is determined to be a PAK inhibitor if administering the candidate substance to PAK and the natural binding partner results in an at least 2-fold decrease in binding between PAK and the natural binding partner. A candidate substance can be determined to be a PAK inhibitor if administering the candidate substance to PAK and the natural binding partner results in an at least 3-fold, at least 4-fold, at least 5- fold, at least 10-fold, at least 20-fold, at least 50-fold, at least 100-fold, at least 200-fold, at least 500-fold, at least 1000-fold, at least 10,000-fold, or greater than 10,000-fold decrease in binding between PAK and the natural binding partner. A candidate substance can be determined to be a PAK inhibitor if administering the candidate substance to PAK and the natural binding partner results in an at least 25%, 50%, 75%, 100%, 200%, 500%, 1000%, or greater than 1000% decrease in binding between PAK and the natural binding partner.

A candidate substance can be determined to be a PAK inhibitor if administering the candidate substance to PAK and the natural binding partner results in increased binding between PAK and the natural binding partner. A candidate substance can be determined to be a PAK inhibitor if administering the candidate substance to PAK and the natural binding partner results in an at least 2-fold increase in binding between PAK and the natural binding partner. A candidate substance can be determined to be a PAK inhibitor if administering the candidate substance to PAK and the natural binding partner results in an at least 3-fold, at least 4-fold, at least 5 fold, at least 10-fold, at least 20-fold, at least 50-fold, at least 100-fold, at least 200-fold, at least 500-fold, at least 1000-fold, at least 10,000-fold, or greater than 10,000-fold increase in binding between PAK and the natural binding partner. A candidate substance can be determined to be a PAK inhibitor if administering the candidate substance to PAK and the natural binding partner results in an at least 25%, 50%, 75%, 100%, 200%, 500%, 1000%, or greater than 1000% increase in binding between PAK and the natural binding partner.

The activity of PAK inhibitors of the present invention may be determined by, for example, assaying for kinase activity of PAK. In such assays PAK and/or a characteristic portion thereof produced by recombinant means as described above can be contacted with a substrate in the presence of a suitable phosphate donor (e.g. ATP) containing radiolabeled phosphate, and PAK-dependent incorporation of radiolabel into the substrate measured. By "substrate," one means any substance containing a suitable hydroxyl moiety that acts as an acceptor for the γ-phosphate group transferred from a donor molecule such as ATP in a reaction catalyzed by PAK. A substrate may be an endogenous substrate of PAK, i.e. a naturally-occurring substance that is phosphorylated in unmodified cells by naturally-occurring PAK and/or any other substance that is not normally phosphorylated by PAK in a physiological situation, by that may be phosphorylated by PAK in the reaction conditions employed. A substrate may be a protein or peptide, and the phosphorylation reaction may occur on a substrate serine and/or threonine residue. It is well-known to those skilled in the art that non-natural substrates can act as suitable substrates in kinase assays such as that described above.

It is well known to those skilled in the art that detection of kinase-dependent substrate phosphorylation can be effected by a number of means other than measurement of radiolabeled phosphate incorporation into the substrate. For example, incorporation of phosphate groups can affect physicochemical properties of the substrate, such as electrophoretic mobility, light absorbance, fluorescence and/or phosphorescence, chromatographic properties, *etc.* Such alterations of substrate physicochemical properties can be readily measured by one skilled in the art and used as an indicator of kinase activity.

Alternatively or additionally, it is well known that monoclonal or polyclonal antibodies can be generated which selectively recognize phosphorylated forms of the substrate, and thus the degree of binding of such antibodies to substrate subsequent to the kinase reaction may be used as an indirect method of determining kinase activity. Furthermore, it is known that many kinases, including PAK kinases, possess the capacity to phosphorylated residues on the same kinase molecule. Such phosphorylation reactions are termed autophosphorylation, and therefore measurement of incorporation of phosphate into PAK itself catalyzed by the same may be used to monitor PAK activity. Kinase assays such as those described above may be performed using purified, partially recombinant PAK, and/or PAK which is purified from cells that naturally express the protein using purification procedures such as those described above.

ELISA-based assays may be used to screen for novel PAK substrates. One such assay employs random biotinylated peptides that can be phosphorylated by a kinase, such as PAK. PAK-specific antibodies are immobilized in the wells of a microtiter dish, and samples comprising PAK protein may be diluted into a reaction buffer and subsequently added to plate wells. Reactions are initiated, for example, by the addition of the biotinylated peptide substrates to the PAK sample. Reactions are stopped by removing the mixtures, and then the plates are washed. After washing, streptavidin-horseradish peroxidase (HRP) is added. Thereafter, unbound streptavidin-HRP is removed, the peroxidase color reaction is initiated by addition of the peroxidase substrate, and the optical density is measured in a suitable densitometer.

A candidate substance suspected of modulating the binding between FMRP and PAK can be a candidate substance suspected of modulating the phosphorylation of FMRP by PAK. For example, the candidate substance may be a substance suspected of promoting the phosphorylation of FMRP by PAK. A candidate substance suspected of modulating the phosphorylation of FMRP by PAK can pertain to a candidate substance suspected of inhibiting the phosphorylation of FMRP by PAK.

Alternatively or additionally, PAK activity may be measured by assaying its kinase activity, its ability to interact with its natural binding partners, and/or by the detection of events that lead to and/or are consequent of PAK activity in intact cells, in cell lysates, and/or in systems in which signaling events are reconstituted *in vitro.* For example, it is known that PAK proteins bind to and are activated by GTP-binding proteins such as Rac and/or Cdc42. Thus detection of interaction of PAK with naturally-occurring activators such as Rac/Cdc42, and/or substrates may be used as an indicator of PAK activity.

A candidate substance can be determined to be a PAK inhibitor if administering the candidate substance to PAK and a substrate results in decreased ability of PAK to phosphorylate the substrate. A candidate substance can be determined to be a PAK inhibitor if administering the candidate substance to PAK and the substrate results in an at least 2-fold decrease in the ability of PAK to phosphorylate the substrate. A candidate substance can be determined to be a PAK inhibitor if administering the candidate substance to PAK and the substrate results in an at least 3- fold, at least 4-fold, at least 5-fold, at least 10-fold, at least 20-fold, at least 50-fold, at least 100-fold, at least 200-fold, at least 500-fold, at least 1000-fold, at least 10,000-fold, or greater than 10,000-fold decrease in the ability of PAK to phosphorylate the substrate.

### In Cyto Assays

Methods for screening for PAK modulators wherein a candidate substance is contacted with a cell are described herein. The cell can then be assayed for various parameters associated with PAK activity. For example, parameters associated with PAK activity include, but are not limited to, PAK's ability to interact with its natural binding partners (*e.g*. FMRP) and/or PAK's ability to phosphorylate its substrates.

Cells may be directly assayed for binding between FMRP and PAK. Immunohistochemical techniques, confocal techniques, and/or other techniques to assess binding are well known to those of skill in the art. In some embodiments, a cell is assayed for phosphorylation of FMRP by PAK. Various cell lines may be utilized for such screening assays, including cells specifically engineered for this purpose. Examples of cells used in the screening assays include neuronal cells and/or dendritic cells. The cell may be a stimulated cell, such as a cell stimulated with a growth factor. One of skill in the art would understand that the invention disclosed herein contemplates a wide variety of *in cyto* assays for measuring parameters that correlate with the activity of PAK.

Depending on the assay, cell and/or tissue culture may be required. A cell may be examined using any of a number of different physiologic assays, as discussed above for binding between FMRP and PAK. Alternatively or additionally, molecular analysis may be performed, including, but not limited to, western blotting to monitor protein expression and/or test for protein-protein interactions; northern blotting, differential display of RNA, and/or microarray analysis to monitor mRNA expression; kinase assays to monitor phosphorylation; mass spectrometry to monitor other chemical modifications; *etc.*

Methods for identifying substances that bind to PAK and, therefore, may modulate PAK activity are described herein. One *in cyto* method of identifying substances that bind to PAK is the two-hybrid system assay (Fields et al., 1994, Trends in Genetics, 10:286; and Colas et al., 1998, TIBTECH, 16:355).
In this assay, yeast cells express a first fusion protein comprising a test substance in accordance with the present invention (*e.g*. PAK protein, PAK gene, and/or a characteristic portion thereof) and a DNA-binding domain of a transcription factor such as Gal4 and/or LexA. The cells additionally contain a reporter gene whose promoter contains binding sites for the corresponding DNA-binding domain. By transforming the cells with a vector that expresses a second fusion protein comprising a candidate substance fused to an activation domain (*e.g*. from Gal4 and/or herpes simplex virus VP16) expression of the reporter gene may be increased if the candidate substance interacts with the test substance. Consequently this assay may be used for screening for substances that modulate an interaction between PAK and any number of candidate substances. In this way, it is possible rapidly to identify novel PAK modulators.

The assays can involve solid phase-bound PAK proteins and detecting their interactions with one or more candidate substances. Thus, a test substance (*e.g*. PAK protein and/or a characteristic portion thereof) may contain a detectable marker, such as a radioactive, fluorescent, and/or luminescent label. Furthermore, candidate substances can be coupled to substances which permit indirect detection (*e.g*. by means of employing an enzyme which uses a chromogenic substrate and/or by means of binding a detectable antibody). Changes in the conformation of PAK as the result of an interaction with a candidate substance may be detected, for example, by the change in the emission of the detectable marker. Alternatively or additionally, the solid phase-bound protein complexes may be analyzed by means of mass spectrometry.

The assays can involve monitoring activities of downstream effectors of PAK. For example, assays involving monitoring the activity of ERK pathway members and/or PDK pathway members. Kinase activity can be assayed using any method known in the art. To give but one example, kinase activity can be assayed by using phospho-specific antibodies, as the phosphorylation state of members of these pathways indicates whether the protein is active. For example, a Western blot can be conducted for phosphoERK1/2, and quantification of changes in pathway activity can be obtained by normalization to total BRK1/2 levels. Alternatively or additionally, the activity of downstream members of the ERK signaling pathway (e.g. translation initiation factors S6, eIF4E, 4EBP1, etc.) can also be determined with Western blots probed with phospho-specific antibodies against these proteins (Kelleher et ah, 2004, Cell, 116:467). PI3K pathways activity can be monitored through detection of phosphorylated PDK or downstream signaling proteins Akt and PDK1.

Screening assays may assay PAK activity by monitoring the downstream cellular effects of PAK activity. Such effects include, but are not limited to, the formation of peripheral actin microspikes and/or associated loss of stress fibers (Zhao et al., 1998, Mol. Cell Biol., 18:2153) and/or other cellular responses, such as growth, growth arrest, differentiation, and/or apoptosis.
Yeast cells can be used for this type of screening assay. For example, in a PAK yeast functional assay, when cultured on glucose containing medium, PAK yeast cells grow like normal yeast cells. Upon exposure to galactose, however, intracellular expression of PAK is induced, causing yeast cells to die. Substances that inhibit PAK activity are identified by their ability to prevent yeast cells from dying.

PAK levels can be determined by measuring levels of protein and/or mRNA. Levels of PAK protein and/or characteristic portions thereof are measured using immunoassays such as western blotting and/or ELISA using antibodies that selectively bind to. PAK. For measurement of mRNA, amplification (*e.g*., using polymerase chain reaction [PCR], ligase chain reaction [LCR], *etc*.) and/or hybridization assays (*e.g.,* northern hybridization, RNAse protection, dot blotting, *etc*.) may be used.
Levels of protein and/or mRNA can be detected using directly- and/or indirectly-labeled detection agents, *e.g*., fluorescently and/or radioactively labeled nucleic acids, radioactively and/or enzymatically labeled antibodies, *etc.* as described herein.

Alternatively or additionally, PAK expression may be measured using a reporter gene system. Such a system may be devised using a PAK protein promoter operably-linked to a reporter gene such as chloramphenicol acetyltransferase, firefly luciferase, bacterial luciferase, O-galactosidase, alkaline phosphatase, *etc.* Furthermore, PAK may be used as an indirect reporter via attachment to a second reporter such as red and/or green fluorescent protein (see, *e.g*., Mistili et al., 1997, Nature Biotech., 15:961).
The reporter construct is typically transfected into a cell. After treatment with a candidate substance, the amount of reporter gene transcription, translation, and/or activity is measured according to standard techniques known to those of skill in the art.

Methods to determine whether PAK can induce phosphorylation of FMRP *in vivo* are described herein. In such methods, cells are transfected with vectors expressing constitutively active-PAK and/or FMRP. Cell extracts are prepared at specified time points after transfection, and the degree of phosphorylation of FMRP is analyzed by western blot analysis.
*FMR1* KO mice and FXS patients show similar behavioral phenotypes and/or *similar abnormalities in synaptic morphology in the* brain. Their brains have more dendritic spines and/or a higher proportion of longer and/or thinner spines compared to normal individuals. Furthermore, they display abnormal synaptic function, such as enhanced long-term depression (LTD) mediated by metabotropic glutamate receptor in the hippocampus and/or impaired long-term potentiation (LTP) in the cortex. Therefore, the *FMR1* KO mouse serves as an accurate model of FXS, and the *FMR1* KO mouse exhibits several quantifiable phenotype. Such phenotypes include behavior, synaptic morphology, and/or synaptic function.

In some embodiments, screening methods of the present invention can measure behavioral symptoms. Such behavioral symptoms can include hyperactivity, stereotypy, perseverative behavior, anxiety, hypo-anxiety, seizure, impaired social behavior, and/or cognitive delay. In some embodiments, behavioral symptoms can be measured using an open field test In an open field test, a subject is allowed to run freely in an open arena (*e.g. VersaMax activity monitor chamber from Accuscan Instruments*) and certain behaviors are analyzed. These behaviors include, but are not limited to: (1) hyperactivity, determined by measuring the distance and/or length of time traveled by the subject; (2) stereotypy, determined by measuring the number of repetitive behaviors exhibited by the subject; (3) hypo-anxiety, determined by measuring the amount of time the subject remains in the center field relative to the time spent in the corners of the field; and/or (4) combinations of these.

In some embodiments, behavioral symptoms can be measured using a trace fear conditioning task. For example, a subject is placed in a training chamber (Chamber A), where a tone is sounded, followed by a blank time (also called trace), and then shock. The sequence is repeated several times to let the subject learn the association between tone and shock across the time gap. To examine whether the subject remembers this association, at various time point after conditioning, the subject is placed into a new chamber (Chamber B) with a different shape and smell from Chamber A and its response to the tone is monitored. *If the subject learns and remembers that tone is associated with shock, it will become* immobile (called "freezing"). Previous studies have shown that attention-distracting stimuli and/or lesion of prefrontal cortex and/or hippocampus can interfere with trace fear conditioning. Thus, the subject's attention and/or associative memory may be measured by comparing the degree of freezing both pre- and post-conditioning, often relative to a control subject.
Relevant behavior tests can include eight-arm maze test and/or sensitization test to amphetamine-induced stereotypy for stereotypy and perseverative behavior. Relevant behavior tests can include elevated plus maze, light-dark transition, and/or novelty suppressed feeding test for anxiety, audiogenic seizure, social interaction, and/or social learning for social behaviors. Relevant behavior tests can include Morris water maze (including the reversal version) and/or fear conditioning for learning and memory.

Behavioral symptoms can be measured using an audiogenic seizure (AGS) assay. Fragile X humans and mice are susceptible to seizures at early ages. Fragile X mice show a robust phenotype in an AGS assay. While no 19-21 day old (p19-21) wildtype mice typically have seizures in this task, the majority of fragile X mice do have seizures. AGS can be performed essentially as described (Yan et al., 2005, Neuropharmacol, 49: 1053). Briefly, mice are habituated to a behavioral chamber and then exposed to a high intensity siren of frequency peak 1800 Hz - 6300 Hz at an average sound pressure level above 120 dB at approximately 10 cm for 5 minutes. Behaviors of the mice can be monitored after administration of sound. Fragile X mice typically (1) run wildly, (2) have seizures, and/or (3) die. Wildtype mice typically do not exhibit these responses. An AGS phenotype is scored based on the animal's endpoint.

Behavioral symptoms can be measured using any of several social interaction tests. Home cage behavior and social interaction can be assessed with a variety of tests (Kwon et al., 2006, Neuron, 50:377; Spencer et al., 2005, Genes Brain Behav., 4:420; and Lijam et al., 1997, Cell, 90:895). For example, mice can be observed in their home cage by videorecording and scored for various nonsocial behaviors and/or social behaviors (e.g. grooming, mounting, tail pulling, and sniffing).

Direct social interaction can be assessed by exposing mice to a novel conspecific mouse and observing approaching and sniffing behaviors. Percent of time spent interacting can be recorded. This is typically repeated about 3 days later with the same mice. Control mice usually exhibit a decrease in social interaction the second time, indicating recognition of the familiar mouse and/or normal social learning. *FMR* KO mice typically do not exhibit a decrease in social interaction the second time, indicating impaired social learning, memory, and/or behavior. This test can be conducted in a novel or familiar environment, as significant differences between *FMR* KO and wild-type mice have been observed in social interaction assays depending on the degree of familiarity with the environment. Additional social behaviors can be monitored in this assay including active behaviors (*e.g*. aggressive attacks, lateral threats, and/or chasing) and passive behaviors (*e.g*. receiving sniffing from other mouse and/or not showing signs of submissive and/or defensive behavior).

Prior to an indirect social interaction test mice can be housed individually for 4 days. Indirect social interaction tasks typically take place in a cage divided in half by a clear perforated partition. The task is run in two different modes: one involves a familiar environment, by pre-exposure to the testing chamber, while the other involves a novel environment. Test mice can be exposed to novel or familiar mice.
Typically, *FMR* KO mice behave similarly to wild-type mice in a novel environment, but behave significantly differently in a familiar cage. Time spent at the partition can be recorded in 2 to 5 minute intervals for a 20 minute test. *FMR* KO mice tend to spend significantly less time interacting (i.e. at the partition) in the first few minutes and take longer to first approach the partition than wild-type mice. In contrast, *FMR* KO mice usually spend more time at the partition during the last time intervals than controls.

In some experiments, the indirect social interaction test is conducted in a chamber divided into three rooms. The central room - which is connected to two rooms independent from each other, one on the left and one on the right - is empty. The left room contains an empty cage (*i.e*. a novel object and/or inanimate target), while the right room contains a similar cage enclosing a novel mouse (i.e. a social target). This task involves a choice between spending time with a social target or an inanimate target, and therefore is called a social preference test. Percent of total time interacting with each object can be recorded. *FMR* KO mice may spend a significantly different amount of time (*e.g*. more or less) interacting with the social target than control mice.

A social dominance tube test can be conducted in a tube approximately 30 cm long and 3 cm - 4 cm in diameter. Mice are placed at opposite ends of the tube and released simultaneously. A mouse is pronounced the "winner" when his opponent backs out completely. *FMR1* KO mice win significantly fewer matches against unfamiliar wild-type mice than expected by chance. When a *FMR1* KO mouse competes against a wild-type non-cagemate, the wild-type mouse is the winner in approximately 73% of matches (Kwon et al, 2006, Neuron, 50:377; Spencer et al., 2005, Genes Brain Behav., 4:420; and Lijam et al., 1997, Cell, 90:895).
Modulation of PAK activity via administration of a PAK modulator may ameliorate this phenotype.

Since *FMR1* KO mice have been shown to display some abnormal social behaviors, the present invention encompasses the recognition that home cage social behavior, including nest building and sleeping behavior, may be altered in *FMR1* KO mice. Nesting patterns are evaluated by placing a cotton nestle into a cage of approximately two, three, or four mice of the same genotype (*e.g.* wild-type, *FMR1* KO, *etc*.) that receive identical drug treatments. After about 30 minutes to 1 hour, the nest can be removed and the height measured. Wild-type mice build nests with depths that average 20 mm to 50 mm Mice which display abnormal social behavior, such as *FMR1* KO mice, frequently build shallower nests (*e.g*. < 20 mm).

Sleeping positions of mice in their home cages can be recorded two to four times a day over five consecutive days. Wild-type mice sleep huddled in the well-formed, fluffy nests they build. Observations will determine whether *FMR1* KO mice sleep in scattered, random patterns, do not build full nests, or sleep on top of intact nestle material. The present invention encompasses the recognition that, if *FMR1* KO mice display abnormal phenotypes, amelioration of those phenotypes may occur following modulation of PAK activity.

Behavioral symptoms such as hippocampus-dependent spatial learning can be assessed using the classical Morris Water Maze test. FMR1 KO mice, just like the wild-type controls, learn to find the visible or hidden platform with decreasing latency scores over the course of the standard training protocol. However, some groups observe an abnormal phenotype in a reversal trial, a test in which the platform is transferred to the quadrant opposite the initial training quadrant. In particular, *FMR1* KO mice display increased escape latency and path length, suggesting that they have low response flexibility or high memory interference (see, *e.g*. D'Hooge et al., 1997, Neuroscience, 76:367).

Hippocampal synaptic plasticity can be assessed using a standard protocol for mGluR-LTD (see, *e.g*. Example 5 for a more detailed protocol). Hippocampal slices can be prepared from *FMR* KO mice and control littermates per standard procedures. All experiments are generally performed blind to genotype. Schaffer collaterals are stimulated and extracellular field potentials measured in *str. radiatum* of CA1. mGluR-dependent LTD is elicited both electrophysiologically (by pairs of stimuli delivered at 1 Hz for 15 minutes to 20 minutes; "PP-LFS") and pharmacologically (by bath application of 50 µM to 100 µM 3,4-dihydroxyphenylglycine (DHPG) for 5 minutes). The initial slope of the field potential is recorded as an indicator of synaptic strength. Hippocampal slices from *FMR1* KO mice show enhanced mGluR-LTD relative to control mice (see, *e.g*., Huber et al., 2002, Proc. Natl. Acad. Sci., USA, 99:7746; and Nosyreva and Huber, 2006, J. Neurophysiol., 95:3291).

Cortical long-term potentiation (LTP) is reduced in slices from *FMR1* KO mice (see Figure 14). Coronol brain slices containing temporal cortex are prepared from two- to three-month-old male littermates, and left to recover for at least 1 hour before recording in oxygenated (95% O₂ and 5% CO₂) warm (30 °C) artificial cerebrospinal fluid containing 124 mM NaCl, 5 mM KCl, 1.25 mM NaH₂PO₄, 1 mM MgCl₂, 2 mM CaCl₂, 26 mM NaHCO₃, 10 mM dextrose. Field potentials (FPs) in layer II/III evoked by layer IV stimulation are measured as previously described and responses are quantified as the amplitude of FP in cortex. LTP is induced by TBS, which consisted of eight brief bursts (each with 4 pulses at 100 Hz) of stimuli delivered every 200 msec. Genetic inhibition of PAK rescues the reduced cortical LTP in the *FMR1* KO mice, and the present invention encompasses the recognition that pharmacological inhibition may have the same effect (Hayashi et al., 2007, Proc. Natl. Acad. Sci., USA, 104:11489*).*

The methods can involve measurement of synaptic morphology. The number of dendritic spines can be counted. Spine number can be examined in Golgi-stained layer II/III pyramidal neurons of the temporal cortex. For example, serial brain sections can be obtained following the Golgi-Cox technique. Layer II/IIIpyramidal neurons in the temporal cortex are visualized by microscopy (e.g. under Olympus upright BX61 with motorized XY stage using Neurolucida/ stereology software [Microbrightfield]). On each primary apical dendritic branch, regularly-sized segments (*e.g*. ten consecutive 10-µm-long dendritic segments) are analyzed to quantify spine density (*e.g*. the number of spines per 10 µm long dendritic segment). In some embodiments, length and/or width of dendritic spines is measured. In some embodiments, spine length is measured from Golgi-stained neurons.

Dendritic spine head size can be examined by electron microscopic analysis of the length of the postsynaptic density (PSD).
The dendritic spine heads can be examined by conducting electron microscopic analyses of the proportion of larger, perforated synapses. For example, for electron microscopy; subjects are anaesthetized and perfused. Blocks of temporal cortex are embedded, from which sections (*e.g*. 1 µm thick) are cut and stained with toluidine blue (*e.g*. diluted to 1%) to guide the further trimming to isolate layer II/III of temporal cortex. Ultrathin sections (*e.g*. 90 nm) are then cut and stained with uracyl acetate and lead citrate. Randomly selected neuropil areas are photographed (*e.g*. at a 10,000X magnification with a JEOL 1200EX electron microscope) and image negatives are scanned and analyzed.

Neurons can be labeled by fluorescent proteins (directly or via indirect staining) in an animal and/or in culture established from an animal. Fluorescent microscopy can be used to visualize neurons and measure the number, length, and/or size of spines. The size of presynaptic terminals can be measured. The size of presynaptic terminals can be examined by conducting electron microscopic analysis of the number of synaptic vesicles and/or docked vesicles. Presynaptic terminals can be labeled by fluorescent proteins and fluorescent microscopy is used to visualize the presynaptic terminals and measure their size.

The methods can involve measuring synaptic function. Long-term depression (LTD) mediated by metabotropic glutamate receptor (mGluR) can be measured in the hippocampus. For example, hippocampal slices are prepared and allowed to recover before recording in oxygenated artificial cerebrospinal fluid (ACSF). Field potentials (FPs) in stratum radiatum of area CA1 are evoked by a current pulse to Schaffer collateral axons. Stable baseline responses are regularly collected by a stimulation intensity (10 µA - 30 µA) yielding 50% - 60% of the maximal response. mGluR-LTD can be induced by application ofmGluR agonist 3,5-dihydroxyphenylglycine (DHPG; *e.g*. at 100 µM) and/or by using paired-pulse low-frequency stimulation consisting of 900 pairs of stimuli delivered at 1 Hz in the presence of the N-methyl-D-aspartate receptor (NMDAR) antagonist D-(-)-2-amino-5-phosphono-pentanoic acid (D-APV; *e.g*. at 50 µM). DHPG is a chiral compound, and mGluR-LTD can be induced by application of RS-DHPG and S-DHPG, but typically not by application of R-DHPG.

Long-term potentiation (LTP) can be measured in the cortex. For example, coronal brain slices containing cortex are prepared and left to recover before recording in oxygenated ACSF. FPs in layer II/III evoked by layer IV stimulation are measured and responses are quantified as the amplitude of FP. Cortical LTP can be induced by theta-burst stimulation (TBS), which consists of brief bursts of stimuli delivered at regular intervals (eg. eight brief bursts, each with 4 pulses at 100 Hz, of stimuli delivered every 200 msec).

Synaptic currents mediated by α-amino-3-hydroxy-5-methylisoxazole-4- propionic acid receptors (AMPARs) and/or NMDARs can be measured in the cortex. For example, for measurement of AMPAR-mediated miniature excitatory postsynaptic current (mEPSC), tetrodotoxin, APV, and bicuculline are added in a bath of ACSF. Continuous traces (*e.g*. 30 msec - 60 msec) are collected at regular intervals (*e.g*. every 8 seconds) and filtered (*e.g*. at 2 KHz). Cells with series resistance above a certain threshhold level (*e.g*. ≥ 13 mΩ) are discarded. The measurement of NMDAR-EPSC/AMPAR-EPSC ratio is typically done in ACSF containing Mg²⁺, bicuculline, and glycine. NMDAR-dependent and AMPAR-dependent responses are discriminated based on their distinct kinetics and voltage dependence. Thus, NMDAR-mediated response is valued as the currents recorded at +40 mV and measured 100 msec after the response onset. The AMPAR-mediated response is taken from the peak amplitude response recorded at -80 mV.

The methods involving measuring the interaction between PAK and its natural binding partners are also described. The candidate substance is determined to treat, alleviate, ameliorate, relieve, delay onset of, inhibit progression of, reduce severity of, and/or reduce incidence of one or more symptoms or features of FXS and/or other neurodevelopmental disorder if the candidate substance modulates the interaction between PAK and its natural binding partners.
The candidate substance can be determined to treat, alleviate, ameliorate, relieve, delay onset of, inhibit progression of, reduce severity of, and/or reduce incidence of one or more symptoms or features of FXS and/or other neurodevelopmental disorder if the candidate substance modulates the interaction between PAK and FMRP. The interaction between PAK and its natural binding partners may be measured using standard methods, which are described herein and in Sambrook et al. (Molecular Cloning: A Laboratory Manual, 3rd ed., Cold Spring Harbor Laboratory Press, Cold Spring Harbor, N.Y., 2001).

Screening methods involving measuring PAK kinase activity in a cell, tissue, in the presence and/or absence of the candidate substance are also described. A candidate substance is determined to treat, alleviate,

Screening methods for determining whether a candidate substance is useful for treating, alleviating, ameliorating, relieving, delaying onset of, inhibiting progression of, reducing severity of, and/or reducing incidence of one or more symptoms or features of a FXS and/or other neurodevelopmental disorder in a mammal that involve measuring the phosphorylation level of FMRP in a cell, tissue, in the presence and/or absence of the candidate substance are described herein. The candidate substance is determined to treat, alleviate, ameliorate, relieve, delay onset of, inhibit progression of, reduce severity of, and/or reduce incidence of one or more symptoms or features of FXS and/or other neurodevelopmental disorder if the candidate substance decreases the phosphorylation level of FMRP

Screening methods of the present invention can involve measuring PAK mRNA and/or protein levels in a cell, tissue, in the presence and/or absence of the candidate substance. The candidate substance is determined to treat, alleviate, ameliorate, relieve, delay onset of, inhibit progression of, reduce severity of, and/or reduce incidence of one or more symptoms or features of FXS and/or other neurodevelopmental disorder if the substance modulates PAK mRNA and/or protein levels. PAK mRNA and/or protein levels may be measured using standard methods, which are described herein and in Sambrook et al. (Molecular Cloning: A Laboratory Manual, 3rd ed., Cold Spring Harbor Laboratory Press, Cold Spring Harbor, N.Y., 2001),

A strain of *C*. *elegans* can be utilized which is characterized by a phenotype of sterility and/or embryonic lethality and/or a defective gonad migration and which harbors an impaired and/or missing PAK function. The strain of *C*. *elegans* utilized could be obtainable and/or could be obtained by one or more of the methods for generating a *C*. *elegans* having said phenotypes. Such a model may be used, amongst other things, for characterizing the signaling pathways linked to PAK. Such a model may be used for identifying substances that interfere and/or interact with one or more proteins that are part of a signaling pathway linked to PAK. Such a model may be used for identification of a PAK modulator using any of the *in vivo* assays described herein.

### Methods of Use

The present invention provides PAK inhibitors for use in methods of treating FXS and/or other neurodevelopmental disorders (e.g. POF, FXTAS, various forms of mental retardation, and/or autism spectrum disorders (ASD)). Such methods involve inhibiting PAK activity. PAK activity may be inhibited by disrupting its ability to interact with FMRP and/or to phosphorylate FMRP.

The invention includes pharmaceutical compositions comprising at least one PAK inhibitor and at least one pharmaceutically acceptable excipient. A pharmaceutical composition comprising a candidate substance can be for administration to a cell, such as one in a patient suffering from and/or susceptible to FXS and/or other neurodevelopmental disorders.

The PAK inhibitors in one instance of the present disclosure,can be useful in the treatment of abnormal synaptic formation. Abnormal synaptic formation is caused by FXS and/or other neurodevelopmental disorders. Inventive PAK can be useful in the treatment of improperly- formed dendritic spines. Dendritic spines can be characterized as "improperly- formed" when an individual possesses abnormally high numbers of dendritic spines. Dendritic spines can be characterized as "improperly-formed" when an individual possesses spines that are abnormally long and/or thin. Improperly-formed dendritic spines are caused by FXS and/or other neurodevelopmental disorders.

Inventive PAK inhibitors can be used in the treatment of enhanced long-term depression (LTD) mediated by metabotropic glutamate receptor in the hippocampus and/or impaired long-term potentiation (LTP) in the cortex. Defective enhanced long-term depression (LTD) mediated by metabotropic glutamate receptor in the hippocampus and/or impaired long-term potentiation (LTP) in the cortex are caused by FXS and/or other neurodevelopmental disorders.

PAK inhibitors of the present invention can be useful in the treatment of one or more of the following symptoms: hyperactivity, stereotypy, anxiety, seizure, impaired social behavior, and/or cognitive delay caused by FXS and/or other neurodevelopmental disorders.

Methods of treating FXS and/or other neurodevelopmental disorders comprising steps of (1) providing a patient exhibiting symptoms of, suffering from, and/or susceptible to FXS and/or other neurodevelopmental disorders, and (2) administering a substance that inhibits the ability of PAK to interact with its natural binding partners are described.

Methods of treating FXS and/or other neurodevelopmental disorders comprising steps of (1) providing a patient exhibiting symptoms of, suffering from, and/or susceptible to FXS and/or other neurodevelopmental disorders, and (2) administering a substance that inhibits and/or disrupts the binding of PAK and FMRP are described.

Methods of treating FXS and/or other neurodevelopmental disorders comprising steps of (1) providing a patient exhibiting symptoms of, suffering from, and/or susceptible to FXS and/or other neurodevelopmental disorders, and (2) administering a substance that modulates the kinase activity of PAK are described.

Methods of treating FXS and/or other neurodevelopmental disorders comprising steps of (1) providing a patient exhibiting symptoms of, suffering from, and/or susceptible to FXS and/or other neurodevelopmental disorders, and (2) administering a substance that modulates ability of PAK to phosphorylate FMRP are described.

Methods of treating FXS and/or other neurodevelopmental disorders comprising steps of (1) providing a patient exhibiting symptoms of FXS and/or other neurodevelopmental disorders, and (2) administering a substance that modulates the levels of PAK mRNA and/or protein are described.

In some embodiments, inventive PAK inhibitors may be used to treat neurodevelopmental diseases, conditions, and/or disorders other than FXS, such as, mental retardation, autism spectrum disorders, premature ovarian failure and fragile X associated tremor ataxia. For example, some *FMR1* mutations do not cause FXS, but instead cause other disorders. "Alleles" of the *FMR1* gene are defined by the number of CGG repeats and/or by the methylation status of the *FMR1* locus. *FMR1* normally has between 7-52 CGG repeats (most often 30 repeats) located in the 5' untranslated region of exon 1. These sequences are normally unmethylated, but methylation can occur at each C of CG dinucleotide repeats, which silences expression of *FMR1.*

A "full mutation" is characterized by > 200 CGG repeats that are methylated. All males and 50% of females possessing the full mutation will develop FXS. In contrast, a premutation" is characterized by 55 - 200 CGG repeats that are not methylated. These individuals are not likely to develop FXS, but females exhibit increased twinning and/or an increased occurrence of premature ovarian failure (POF).

Furthermore, the premutation may lead to fragile X-associated tremor ataxia (FXTAS), especially in males. FXTAS is characterized by intention tremor, ataxia, cognitive decline, and/or brain atrophy and is thought to be related to RNA levels.

The premutation may lead to cognitive effects. For example, a premutation carrier's IQ may be within the average range, but is likely to be on lower end of the average range. There is evidence of socio-emotional effects, including increased shyness and/or social anxiety.

Thus, improper expression and/or activity of the *FMR1* gene may lead to diseases, disorders, and/or conditions other than FXS.

### Pharmaceutical compositions

The present invention provides PAK inhibitors useful in the treatment of FXS and/or other neurodevelopmental disorders. In some embodiments, the present invention provides pharmaceutical compositions comprising PAK inhibitors and at least one pharmaceutically acceptable excipient. The present invention provides pharmaceutical compositions comprising a therapeutically effective amount of inventive PAK inhibitor(s) appropriately formulated for administration to a subject suffering from and/or susceptible to FXS and/or other neurodevelopmental disorders. Such pharmaceutical compositions may optionally comprise one or more additional therapeutically-active substances.

The invention encompasses the preparation and/or use of pharmaceutical compositions comprising PAK inhibitors for use in treatment of FXS and/or other neurodevelopmental disorders as an active ingredient. Such a pharmaceutical composition may consist of the active ingredient alone, in a form suitable for administration to a subject, or the pharmaceutical composition may comprise the active ingredient and one or more pharmaceutically acceptable excipients, one or more additional ingredients, and/or a combination of these. The active ingredient may be present in the pharmaceutical composition in the form of a physiologically acceptable ester and/or salt, such as in combination with a physiologically acceptable cation and/or anion, as is well known in the art.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation. Patients to which administration of the pharmaceutical compositions of the invention is contemplated include, but are not limited to, humans and/or other primates; mammals, including commercially relevant mammals such as cattle, pigs, horses, sheep, cats, and/or dogs; and/or birds, including commercially relevant birds such as chickens, ducks, geese, and/or turkeys.

Pharmaceutical compositions as described herein may be prepared by any method known or hereafter developed in the art of pharmaceutics. In general, such preparatory methods include the step of bringing the active ingredient into association with one or more excipients and/or one or more other accessory ingredients, and then, if necessary and/or desirable, shaping and/or packaging the product into a desired single- or multi-dose unit.

A pharmaceutical composition of the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

The relative amounts of the active ingredient, the pharmaceutically acceptable excipient(s), and/or any additional ingredients in a pharmaceutical composition of the invention will vary, depending upon the identity, size, and/or condition of the subject treated and further depending upon the route by which the composition is to be administered. By way of example, the composition may comprise between 0.1% and 100% (w/w) active ingredient.

Pharmaceutical formulations of the present invention may additionally comprise a pharmaceutically acceptable excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro, (Lippincott, Williams & Wilkins, Baltimore, MD, 2006) discloses various excipients used in formulating pharmaceutical compositions and known techniques for the preparation thereof. Except insofar as any conventional excipient is incompatible with a substance or its derivatives, such as by producing any undesirable biological effect or otherwise interacting in a deleterious manner with any other component(s) of the pharmaceutical composition, its use is contemplated to be within the scope of this invention.

The pharmaceutically acceptable excipient can be at least 95%, 96%, 97%, 98%, 99%, or 100% pure. The excipient can be approved for use in humans and for veterinary use. The excipient can be approved by United States Food and Drug Administration. The excipient can be pharmaceutical grade. The excipient can meet the standards of the United States Pharmacopoeia (USP), the European Pharmacopoeia (EP), the British Pharmacopoeia, and/or the International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in the inventive formulations. Excipients such as cocoa butter and suppository waxes, coloring agents, coating agents, sweetening, flavoring, and perfuming agents can be present in the composition, according to the judgment of the formulator.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, *etc*., and combinations thereof

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked poly(vinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (VEEGUM), sodium lauryl sulfate, quaternary ammonium compounds, *etc*., and combinations thereof.

Exemplary surface active agents and/or emulsifiers include, but are not limited to, natural emulsifiers (e.g. acacia, agar, alginic acid, sodium alginate, tragacanth, chondrux, cholesterol, xanthan, pectin, gelatin, egg yolk, casein, wool fat, cholesterol, wax, and lecithin), colloidal clays (e.g. bentonite [aluminum silicate] and VEEGUM [magnesium aluminum silicate]), long chain amino acid derivatives, high molecular weight alcohols (*e.g*. stearyl alcohol, cetyl alcohol, oleyl alcohol, triacetin monostearate, ethylene glycol distearate, glyceryl monostearate, and propylene glycol monostearate, polyvinyl alcohol), carbomers (*e.g.* carboxy polymethylene, polyacrylic acid, acrylic acid polymer, and carboxyvinyl polymer), carrageenan, cellulosic derivatives (*e.g.* carboxymethylcellulose sodium, powdered cellulose, hydroxymethyl cellulose, hydroxypropyl cellulose, hydroxypropyl methylcellulose, methylcellulose), sorbitan fatty acid esters (*e.g*. polyoxyethylene sorbitan monolaurate [TWEEN^{®}20], polyoxyethylene sorbitan [TWEEN^{®}60], polyoxyethylene sorbitan monooleate [TWEEN^{®}80], sorbitan monopalmitate [SPAN^{®}40], sorbitan monostearate [SPAN^{®}60], sorbitan tristearate [SPAN^{®}65], glyceryl monooleate, sorbitan monooleate [SPAN^{®}80]), polyoxyethylene esters (*e.g*. polyoxyethylene monostearate [MYRJ^{®}45], polyoxyethylene hydrogenated castor oil, polyethoxylated castor oil, polyoxymethylene stearate, and SOLUTOL), sucrose fatty acid esters, polyethylene glycol fatty acid esters (*e.g*. CREMOPHOR), polyoxyethylene ethers, (*e.g.* polyoxyethylene lauryl ether [BRIJ^{®}30]), poly(vinyl-pyrrolidone), diethylene glycol monolaurate, triethanolamine oleate, sodium oleate, potassium oleate, ethyl oleate, oleic acid, ethyl laurate, sodium lauryl sulfate, PLURONIC®F 68, POLOXAMER 188, cetrimonium bromide, cetylpyridinium chloride, benzalkonium chloride, docusate sodium, *etc.* and/or combinations thereof.

Exemplary binding agents include, but are not limited to, starch (*e.g.* cornstarch and starch paste); gelatin; sugars *(e.g.* sucrose, glucose, dextrose, dextrin, molasses, lactose, lactitol, mannitol,); natural and synthetic gums (*e.g*. acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, poly(vinylpyrrolidone), magnesium aluminum silicate (VEEGUM), and larch arabogalactan); alginates; polyethylene oxide; polyethylene glycol; inorganic calcium salts; silicic acid; polymethacrylates; waxes; water; alcohol; *etc.*; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and sorbic acid. Exemplary alcohol preservatives include, but are not limited to, ethanol, polyethylene glycol, phenol, phenolic compounds, bisphenol, chlorobutanol, hydroxybenzoate, and phenylethyl alcohol. Exemplary acidic preservatives include, but are not limited to, vitamin A, vitamin C, vitamin E, beta-carotene, citric acid, acetic acid, dehydroacetic acid, ascorbic acid, sorbic acid, and phytic acid. Other preservatives include, but are not limited to, tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, GLYDANT Plus^{®}, PHENONIP^{®}, methylparaben, GERMALL^{®}115, GERMABEN^{®}II, NEOLONE^{™}, KATHON, and EUXYL. In certain embodiments, the preservative is an anti-oxidant. In other embodiments, the preservative is a chelating agent.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, *etc*., and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, *etc*., and combinations thereof.

Exemplary oils include, but are not limited to, almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and combinations thereof.

Liquid dosage forms for oral and parenteral administration include, but are not limited to, pharmaceutically acceptable emulsions, microemulsions, solutions, suspensions, syrups and elixirs. In addition to the active ingredient(s), liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, oral compositions can include adjuvants such as wetting agents, emulsifying and suspending agents, sweetening, flavoring, and perfuming agents. In certain embodiments for parenteral administration, an active ingredient can be mixed with solubilizing agents such as CREMOPHOR, alcohols, oils, modified oils, glycols, polysorbates, cyclodextrins, polymers, and combinations thereof.

Injectable preparations, for example, sterile injectable aqueous or oleaginous suspensions may be formulated according to the known art using suitable dispersing or wetting agents and suspending agents. A sterile injectable preparation may be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or di-glycerides. In addition, fatty acids such as oleic acid are used in the preparation of injectables.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Compositions for rectal or vaginal administration are typically suppositories which can be prepared by mixing the active ingredients of this invention with suitable nonirritating excipients such as cocoa butter, polyethylene glycol, or a suppository wax which are solid at ambient temperature but liquid at body temperature and therefore melt in the rectum or vaginal cavity and release the active ingredient.

Solid dosage forms for oral administration include capsules, tablets, pills, powders, and granules. In such solid dosage forms, the active ingredient is mixed with at least one inert, pharmaceutically acceptable excipient such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and/or (i) lubricants such as talc, calcium stearate, magnesium stearate, solid polyethylene glycols, sodium lauryl sulfate, and mixtures thereof. In the case of capsules, tablets and pills, the dosage form may comprise buffering agents.

Solid compositions of a similar type may be employed as fillers in soft and hard-filled gelatin capsules using such excipients as lactose or milk sugar as well as high molecular weight polyethylene glycols and the like. Solid dosage forms of tablets, dragees, capsules, pills, and granules can be prepared with coatings and shells such as enteric coatings and other coatings well known in the pharmaceutical formulating art. They may optionally comprise opacifying agents and can be of a composition that they release the active ingredient(s) only, or preferentially, in a certain part of the intestinal tract, optionally, in a delayed manner. Examples of embedding compositions which can be used include polymeric substances and waxes. Solid compositions of a similar type may be employed as fillers in soft and hard-filled gelatin capsules using such excipients as lactose or milk sugar as well as high molecular weight polethylene glycols and the like.

Active ingredients can be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active ingredient may be admixed with at least one inert diluent such as sucrose, lactose or starch. Such dosage forms may comprise, as is normal practice, additional substances other than inert diluents, e.g., tableting lubricants and other tableting aids such a magnesium stearate and microcrystalline cellulose. In the case of capsules, tablets and pills, dosage forms may comprise buffering agents. They may optionally comprise opacifying agents and can be of a composition that they release the active ingredient(s) only, or preferentially, in a certain part of the intestinal tract, optionally, in a delayed manner. Examples of embedding compositions which can be used include polymeric substances and waxes.

Dosage forms for topical and/or transdermal administration of a composition of this invention may include ointments, pastes, creams, lotions, gels, powders, solutions, sprays, inhalants and/or patches. Generally, an active ingredient is admixed under sterile conditions with a pharmaceutically acceptable excipient and/or any needed preservatives and/or buffers as may be required. Additionally, the present invention contemplates the use of transdermal patches, which often have the added advantage of providing controlled delivery of an active ingredient to the body. Such dosage forms may be prepared, for example, by dissolving and/or dispensing the active ingredient in the proper medium. Alternatively or additionally, the rate may be controlled by either providing a rate controlling membrane and/or by dispersing the active ingredient in a polymer matrix and/or gel.

Suitable devices for use in delivering intradermal pharmaceutical compositions described herein include short needle devices such as those described in U.S. Patents 4,886,499; 5,190,521; 5,328,483; 5,527,288; 4,270,537; 5,015,235; 5,141,496; and 5,417,662. Intradermal compositions may be administered by devices which limit the effective penetration length of a needle into the skin, such as those described in PCT publication WO 99/34850 and functional equivalents thereof. Jet injection devices which deliver liquid vaccines to the dermis via a liquid jet injector and/or via a needle which pierces the stratum corneum and produces a jet which reaches the dermis are suitable. Jet injection devices are described, for example, in U.S. Patents 5,480,381; 5,599,302; 5,334,144; 5,993,412; 5,649,912; 5.,569,189; 5,704,911; 5,383,851; 5,893,397; 5,466,220; 5,339,163; 5,312,335; 5,503,627; 5,064,413; 5,520,639; 4,596,556; 4,790,824; 4,941,880; 4,940,460; and PCT publications WO 97/37705 and WO 97/13537. Ballistic powder/particle delivery devices which use compressed gas to accelerate vaccine in powder form through the outer layers of the skin to the dermis are suitable. Alternatively or additionally, conventional syringes may be used in the classical mantoux method of intradermal administration.

Formulations suitable for topical administration include, but are not limited to, liquid and/or semi liquid preparations such as liniments, lotions, oil in water and/or water in oil emulsions such as creams, ointments and/or pastes, and/or solutions and/or suspensions. Topically-administrable formulations may, for example, comprise from about 1.0% to about 10% (w/w) active ingredient, although the concentration of the active ingredient may be as high as the solubility limit of the active ingredient in the solvent. Formulations for topical administration may further comprise one or more of the excipients and/or additional ingredients described herein.

A pharmaceutical composition of the invention may be prepared, packaged, and/or sold in a formulation suitable for pulmonary administration via the buccal cavity. Such a formulation may comprise dry particles which comprise the active ingredient and which have a diameter in the range from about 0.5 µm to about 7 µm or from about 1 µm to about 6 µm. Such compositions are conveniently in the form of dry powders for administration using a device comprising a dry powder reservoir to which a stream of propellant may be directed to disperse the powder and/or using a self propelling solvent/powder dispensing container such as a device comprising the active ingredient dissolved and/or suspended in a low-boiling propellant in a sealed container. Such powders comprise particles wherein at least 98% of the particles by weight have a diameter greater than 0.5 µm and at least 95% of the particles by number have a diameter less than 7 µm. Alternatively, at least 95% of the particles by weight have a diameter greater than 1 µm and at least 90% of the particles by number have a diameter less than 6 µm. Dry powder compositions may include a solid fine powder diluent such as sugar and are conveniently provided in a unit dose form.

Low boiling propellants generally include liquid propellants having a boiling point of below 65 °F at atmospheric pressure. Generally the propellant may constitute 50% to 99.9% (w/w) of the composition, and the active ingredient may constitute 0.1 % to 20% (w/w) of the composition. The propellant may further comprise additional ingredients such as a liquid non-ionic and/or solid anionic surfactant and/or a solid diluent (which may have a particle size of the same order as particles comprising the active ingredient).

Pharmaceutical compositions of the invention formulated for pulmonary delivery may provide the active ingredient in the form of droplets of a solution and/or suspension. Such formulations may be prepared, packaged, and/or sold as aqueous and/or dilute alcoholic solutions and/or suspensions, optionally sterile, comprising the active ingredient, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate. Droplets provided by this route of administration may have an average diameter in the range from about 0.1 µm to about 200 µm.

Formulations described herein as being useful for pulmonary delivery are useful for intranasal delivery of a pharmaceutical composition of the invention. Another formulation suitable for intranasal administration is a coarse powder comprising the active ingredient and having an average particle from about 0.2 µm to 500 µm. Such a formulation is administered in the manner in which snuff is taken, *i.e.* by rapid inhalation through the nasal passage from a container of the powder held close to the nares.

Formulations suitable for nasal administration may, for example, comprise from about as little as 0.1% (w/w) and as much as 100% (w/w) of the active ingredient, and may comprise one or more of the excipients and/or additional ingredients described herein. A pharmaceutical composition of the invention may be prepared, packaged, and/or sold in a formulation suitable for buccal administration. Such formulations may, for example, be in the form of tablets and/or lozenges made using conventional methods, and may contain, for example, 0.1% to 20% (w/w) active ingredient, the balance comprising an orally dissolvable and/or degradable composition and, optionally, one or more of the excipients and/or additional ingredients described herein. Alternately, formulations suitable for buccal administration may comprise a powder and/or an aerosolized and/or atomized solution and/or suspension comprising the active ingredient. Such powdered, aerosolized, and/or aerosolized formulations, when dispersed, may have an average particle and/or droplet size in the range from about 0.1 µm to about 200 µm, and may further comprise one or more of the excipients and/or additional ingredients described herein.

A pharmaceutical composition of the invention may be prepared, packaged, and/or sold in a formulation suitable for ophthalmic administration. Such formulations may, for example, be in the form of eye drops including, for example, a 0.1%/1.0% (w/w) solution and/or suspension of the active ingredient in an aqueous or oily liquid excipient. Such drops may further comprise buffering agents, salts, and/or one or more other of the excipients and/or additional ingredients described herein. Other opthalmically-administrable formulations which are useful include those which comprise the active ingredient in microcrystalline form and/or in a liposomal preparation. Such formulations may also be ear drops.

General considerations in the formulation and/or manufacture of pharmaceutical agents may be found, for example, in Remington: The Science and Practice of Pharmacy 21st ed., Lippincott Williams & Wilkins, 2005.

### Administration

. Thus, pharmaceutical compositions containing one or more PAK inhibitiors may be administered to one or more individuals suffering from, susceptible to, and/or exhibiting symptoms of FXS.

A therapeutically effective amount of a pharmaceutical composition comprising a PAK inhibitor can be delivered to a subject and/or organism prior to, simultaneously with, and/or after diagnosis with FXS and/or other neurodevelopmental disorder. A therapeutic amount of a pharmaceutical composition can be delivered to a patient and/or organism prior to, simultaneously with, and/or after onset of symptoms of FXS and/or other neurodevelopmental disorder. The amount of pharmaceutical composition can be sufficient to treat, alleviate, ameliorate, relieve, delay onset of, inhibit progression of, reduce severity of, and/or reduce incidence of one or more symptoms or features of FXS and/or other neurodevelopmental disorder.

Pharmaceutical compositions may be administered using any amount and any route of administration effective for treatment. The exact amount required will vary from subject to subject, depending on the species, age, and general condition of the subject, the severity of the infection, the particular composition, its mode of administration, its mode of activity, and the like. Compositions of the invention are typically formulated in dosage unit form for ease of administration and uniformity of dosage. It will be understood, however, that the total daily usage of the compositions of the present invention will be decided by the attending physician within the scope of sound medical judgment. The specific therapeutically effective dose level for any particular subject or organism will depend upon a variety of factors including the disorder being treated and the severity of the disorder, the activity of the specific active ingredient employed; the specific composition employed; the age, body weight, general health, sex and diet of the subject; the time of administration, route of administration, and rate of excretion of the specific active ingredient employed; the duration of the treatment; drugs used in combination or coincidental with the specific active ingredient employed; and like factors well known in the medical arts.

Pharmaceutical compositions may be administered by any route. Pharmaceutical compositions may be administered by a variety of routes, including oral, intravenous, intramuscular, intra-arterial, intramedullary, intrathecal, parenteral, subcutaneous, intraventricular, transdermal, interdermal, rectal, intravaginal, intraperitoneal, topical (e.g. by powders, ointments, creams, gels, and/or drops), transdermal, mucosal, nasal, buccal, enteral, sublingual; by intratracheal instillation, bronchial instillation, and/or inhalation; and/or as an oral spray, nasal spray, and/or aerosol. Inventive compositions may be administered intravenously. Inventive compositions may be administered orally. Inventive compositions may be administered using a continuous IV drip. Inventive compositions may be administered by retro-orbital injection.

Inventive compositions may be delivered using a pump. A pump to be used in accordance with the invention can be an external pump. A pump to be used in accordance with the invention can be a pump that is implanted within the body of a subject. A pump to be used in accordance with the invention can be a mechanical pump and/or an osmotic pump.

In general the most appropriate route of administration will depend upon a variety of factors including the nature of the composition (*e.g*., its stability in the environment of the gastrointestinal tract), the condition of the subject (*e.g*., whether the subject is able to tolerate oral administration), *etc*.

A composition may be administered in amounts ranging from about 0.001 mg/kg to about 100 mg/kg, from about 0.01 mg/kg to about 50 mg/kg, from about 0.1 mg/kg to about 40 mg/kg, from about 0.5 mg/kg to about 30 mg/kg, from about 0.01 mg/kg to about 10 mg/kg, from about 0.1 mg/kg to about 10 mg/kg, or from about 1 mg/kg to about 25 mg/kg, of subject body weight per day, one or more times a day, to obtain the desired therapeutic effect. The desired dosage may be delivered three times a day, two times a day, once a day, every other day, every third day, every week, every two weeks, every three weeks, or every four weeks. The desired dosage may be delivered using multiple administrations (e.g., two, three, four, five, six, seven, eight, nine, ten, eleven, twelve, thirteen, fourteen, or more administrations).

It will be appreciated that therapeutic agents and pharmaceutical compositions of the present invention can be employed in combination therapies.
For example, "therapeutic cocktails" comprising inventive compositions.
The particular combination of therapies (therapeutics or procedures) to employ in a combination regimen will take into account compatibility of the desired therapeutics and/or procedures and the desired therapeutic effect to be achieved. It will be appreciated that the therapies employed may achieve a desired effect for the same purpose. For example, a PAK inhibitor may be administered with another agent that is used to treat symptoms of FXS and/or other neurodevelopmental disorder. The therapies employed may achieve different effects (e.g., control of any adverse side effects).

Pharmaceutical compositions of the present invention may be administered either alone or in combination with one or more other therapeutic agents. By "in combination with," it is not intended to imply that the agents must be administered at the same time and/or formulated for delivery together, although these methods of delivery are within the scope of the invention. Compositions can be administered concurrently with, prior to, or subsequent to, one or more other desired therapeutics or medical procedures. In general, each agent will be administered at a dose and/or on a time schedule determined for that agent. Additionally, the invention encompasses the delivery of the inventive pharmaceutical compositions in combination with agents that may improve their bioavailability, reduce and/or modify their metabolism, inhibit their excretion, and/or modify their distribution within the body.

The particular combination of therapies (therapeutics and/or procedures) to employ in a combination regimen will take into account compatibility of the desired therapeutics and/or procedures and/or the desired therapeutic effect to be achieved. It will be appreciated that the therapies employed may achieve a desired effect for the same disorder (for example, an inventive agent may be administered concurrently with another therapeutic agent used to treat the same disorder), and/or they may achieve different effects (e.g., control of any adverse side effects). Compositions of the invention can be administered with a second therapeutic agent that is approved by the U.S.

### Food and Drug Administration.

In will further be appreciated that therapeutically active agents utilized in combination may be administered together in a single composition or administered separately in different compositions.

In general, it is expected that agents utilized in combination with be utilized at levels that do not exceed the levels at which they are utilized individually. In some embodiments, the levels utilized in combination will be lower than those utilized individually.

The pharmaceutical compositions of the present invention may be administered alone and/or in combination with other agents that are used to treat the symptoms of FXS. In some embodiments, such agents may treat seizures and/or mood instability, including but not limited to as Carbamazepine (TEGRETOL^{®}), Valproic Acid, Divalproex (DEPAKOTE^{®}), Lithium Carbonate, Gabapentin (NEURONTIN^{®}), Lamotrigine (LANUCTAL^{®}), Topiramate (TOPAMAX^{®}), Tiagabine (GABITRIL^{®}), Vigabatrin (SABRIL^{®}), Phenobarbital, Primidone (MYSOLINE^{®}), and/or Phenytoin (DILANTIN^{®}).

In some embodiments, such agents may be central nervous system stimulants, including but not limited to Methylphenidate (RITALIN^{®}), Dextroamphetamine (DEXEDRINE^{®}; ADDERALL^{®}), Ditropan (CONCERTA^{®}), L-acetylcarnitine, Venlafaxine (EFFEXOR^{®}), Nefazodone (SERZONE^{®}), Amantadine (SYMMETREL^{®}), Buproprion (WELLBUTRIN^{®}), Desipramine, Imipramine, and/or Buspirone (BUSPAR^{®}).

In some embodiments, such agents may be antihypertensive drugs, including but not limited to Clonidine (CATAPRES^{®}) and/or Guanfacine (TENEX^{®}).

In certain embodiments, such agents may include folic acid.

In some embodiments, such agents may be selected serotonin reuptake inhibitors, including but not limited to Fluoxetine (PROZAC^{®}), Sertraline (ZOLOFT^{®}), and/or Citalopram (CELEXA^{®}).

Such agents may be antipsychotics, including but not limited to Risperidone (RISPERIDAL^{®}), Olanzepine (ZYPREXA^{®}) and/or Quetiapine (SEROQUEL^{®}).

Such agents may be used to treat sleep disturbances, including but not limited to Desyrel (TRAZODONE^{®}) and/or Melatonin.

In will further be appreciated that therapeutically active agents utilized in combination may be administered together in a single composition or administered separately in different compositions.

Inventive PAK inhibitors may be administered in combination with one or more other PAK modulators.

### Kits

A variety of kits comprising one or more of PAK inhibitors are described herein. For example, kits comprising at least one PAK inhibitor and instructions for use. A kit may comprise multiple different PAK inhibitors. A kit may comprise any of a number of additional components or reagents in any combination.

A kit may include, for example, (i) a PAK inhibitor; (ii) instructions for administering the PAK inhibitor to a subject suffering from, susceptible to, and/or exhibiting symptoms of FXS and/or other neurodevelopmental disorder. whether it treats, alleviates, ameliorates, relieves, delays onset of, inhibits progression of, reduces severity of, and/or reduces incidence of one or more symptoms or features of FXS and/or other neurodevelopmental disorder in the mouse.

Kits typically include instructions for use of inventive PAK inhibitors. Instructions may, for example, comprise protocols and/or describe conditions for identification of PAK inhibitors, administration of PAK inhibitors to a subject in need thereof, etc. Kits generally include one or more vessels or containers so that some or all of the individual components and reagents may be separately housed. Kits may also include a means for enclosing individual containers in relatively close confinement for commercial sale, e.g., a plastic box, in which instructions, packaging materials such as styrofoam, etc., may be enclosed. An identifier, e.g., a bar code, radio frequency identification (ID) tag, etc., may be present in or on the kit or in or one or more of the vessels or containers included in the kit. An identifier can be used, e.g., to uniquely identify the kit for purposes of quality control, inventory control, tracking, movement between workstations, etc.

### Exemplification

The representative Examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein.

The following Examples contain important additional information, exemplification and guidance that can be adapted to the practice of this invention in its various embodiments and the equivalents thereof It will be appreciated, however, that these examples do not limit the invention.

Previous studies have consistently associated mental retardation with abnormalities in the number and size of synapses. In FXS patients and in *FMR1* KO mice, cortical neurons display more postsynaptic dendritic spines and a higher proportion of longer and thinner spines compared to normal individuals. The *FMR1* KO mice exhibit impaired cortical LTP compared to wild-type mice. Strikingly, the abnormalities in cortical synaptic morphology and plasticity of *FMR1* KO mice are opposite to those we observed in transgenic (*dnPAK*TG) mice in which activity of p21-activated kinase (PAK) is inhibited by its dominant negative form (dnPAK), specifically in the postnatal forebrain. In the *dnPAK* TG mice, we found that cortical neurons display fewer dendritic spines and a lower proportion of longer and thinner spines compared to wild type mice (Hayashi et al., 2004, Neuron, 42:773). These transgenic mice exhibit enhanced cortical LTP, in contrast to the impaired cortical LTP in *FMR1* KO mice. One hypothesis that may explain these results is that signaling pathways mediated by PAK and FMR1 may antagonize each other to regulate synaptic morphology and function, and this hypothesis was tested in the following examples.

### Example 1: PAK inhibition rescues spine morphological abnormalities in FMR1 KO mice.

### Materials and Methods

### Golgi Analysis

Following the Golgi-Cox technique (Ramon-Moliner, 1970, Contemporary Research Methods in Neuroanatomy, Springer, Berlin, Heidelberg, New York), 120 µm thick serial sections were obtained from brains of two-month-old male littermates. Slides containing these sections were coded before quantitative analysis, and tube code was broken only after the analysis was completed. Layer II/III pyramidal neurons in the temporal cortex were visualized under Olympus upright BX61 with motorized XY stage using Neurolucida/stereology software (Microbrightfield). On each primary apical dendritic branch, ten consecutive 10 µm-long dendritic segments were analyzed to quantify spine density. To ensure sampling consistency among Golgi analysis and electrophysiology experiments, analyses in the temporal cortex were all carried out in slices or sections corresponding to Figure 62 - 67 of the mouse brain atlas (Franklin et al., The mouse brain in stereotaxic coordinates, Academic, San Diego, CA, 1997).

### Electron Microscopy

Two-month-old male littermates were anaesthetized and perfused according to standard procedures. Blocks of temporal cortex were then embedded, from which 1 µm thick sections were cut and stained with 1% toluidine blue to guide the further trimming to isolate layer II/III of temporal cortex. 90 nm ultrathin sections were then cut and stained with uracyl acetate and lead citrate. Randomly selected neuropil areas were photographed at a 10,000X magnification with a JEOL 1200EX electron microscope. Image negatives were scanned at 1200 dpi and analyzed by OpenLab Program (Improvision). Excitatory synapses bearing spines were defined by the presence of a clear post-synaptic density (PSD) facing at least three presynaptic vesicles. Micrographs covering 500 µm² - 1000 µm² neuropil regions from each mouse were analyzed and used for quantitation. PSD length and percentage of perforated synapses were quantified from the same population of synapses. The measurements were all performed by an experimenter blind to the genotype.

### Results

Since spine abnormality is a pathological hallmark in FXS patients and *FMR1* KO mice at the cellular level, dendritic spine morphology was examined by measuring spine density in apical dendrites of Golgi-stained layer II/III pyramidal neurons of the temporal cortex of dMT mice as well as their littermates, *dnPAK* TG, *FMR1* KO, and wild-type mice. The number of spines per 10 µm of dendritic segments that run proximal to distal to the neuronal soma was quantified. In proximal dendritic segments, spine density was lower in *dnPAK TG* mice compared to wild-type mice while it was higher in *FMR1* KO mice compared to wild-type mice (Figures 10 and 11). In contrast, spine density in dMT mice was comparable to that in wild-type controls in all dendritic segments except segments 7 and 8 (Figure 11). When averaged over all segments, mean spine density in dMT mice was significantly lower than that in *FMR1* KO mice and significantly higher than that in *dnPAK* TG mice (Figure 12). These results indicate that PAK inhibition partially restores the abnormality of spine density in *FMR1* KO mice.

In addition to in increased spine density, cortical neurons from FXS patients and *FMR1* KO mice exhibit increased spine length (Hinton et al., 1991, Am. J. Med. Genet., 41:289; Comery et al., 1997, Proc. Natl. Acad. Sci., USA, 94:5401; Irwin et al., 2001, Am. J. Med. Genet., 98:161; and McKinney et al., 2005, Am. J. Med. Genet. B. Neuropsychiatr. Genet., 136:98). To investigate whether dnPAK can also restore this abnormality, spine length (the radial distance from tip of spine head to dendritic shaft) of Golgi-stained pyramidal neurons in the four genotypes was measured. In cumulative frequency plots, *FMR1* KO neurons exhibited a significant shift in the overall spine distribution towards spines of longer length compared to wild-type neurons, while *dnPAK* TG neurons exhibited the opposite shift to shorter spines (Figure 13). In contrast, spine length distribution of dMT neurons overlapped well with that of wild-type neurons (Figure 13), indicating that PAK inhibition is sufficient to restore the cortical spine length abnormality in *FMR1* KO mice.

### Example 2: PAK Inhibition Rescues Reduced Cortical LTP in FMR1 KO Mice.

### Materials and Methods

### Electrophysiology

From three-month-old male littermates, coronal brain slices containing temporal cortex were prepared and left to recover for at least 1 hour before recording in oxygenated (95% O₂ and 5% CO₂) warm (30 °C) artificial cerebrospinal fluid containing 124 mM NaCl, 5 mM KCl, 1.25 mM NaH₂PO₄, 1 mM MgCl₂, 2 mM CaCl₂, 26 mM NaHCO₃, and 10 mM dextrose. Field potentials (FPs) in layer II/III evoked by layer IV stimulation were measured as previously described (Hayashi et al., 2004, Neuron 42:773) and responses were quantified as the amplitude of FP in cortex. LTP was induced by TBS, which consisted of eight brief bursts (each with 4 pulses at 100 Hz) of stimuli delivered every 200 msec.\

### Results

Cortical long-term potentiation (LTP) has been shown to be reduced in *FMR1* KO mice while it is enhanced in *dnPAK* TG mice (Li et al., 2002, Mol. Cell. Neurosci., 19:138; Zhao et al., 2005, J. Neurosci., 25:7385; and Hayashi et al., 2004, Neuron, 42:773).
To assess the effect of PAK inhibition on the cortical synaptic transmission and plasticity in *FMR1* KO mice, extracellular field recordings in temporal cortex layer II/III synapses were carried out while stimulating layer IV. Basal synaptic transmission, as measured by field potential responses to a range of stimulus intensities, did not differ between the four genotypes (Figure 14A). However, as expected, administration of theta-burst stimulation (TBS) at 100 Hz produced LTP of a lower magnitude in *FMR1* KO mice than in wild-type mice and LTP of a higher magnitude in *dnPAK* TG than in wild-type mice (Figure 14B). In contrast, the magnitude of LTP was indistinguishable between dMT mice and wild-type controls at various times following the application of the stimulus (Figure 14B). This demonstrates that PAK inhibition rescues LTP defects in *FMR1* KO mice.

### Example 3: PAK inhibition rescues multiple behavioral defects in FMR1 KO mice.

### Materials and Methods

### Open Field Test

Two-month-old male littermates were subjected to the open field test according to standard procedures. Each mouse ran for 10 minutes in a VersaMax activity monitor chamber (Accuscan Instruments). Open field activity was detected by photobeam breaks and analyzed by the VersaMax software. Stereotypy is recorded when the mouse breaks the same beam (or set of beams) repeatedly. Stereotypy count is the number of beam breaks that occur during this period of sterotypic activity.

### Trace Fear Conditioning Task

Three-month-old male littermates were subjected to trace fear conditioning as previously described (Zhao et al., 2005, J. Neurosci., 25:7385). On day 1, mice were placed in the training chamber (Chamber A, Coulbourn Instruments) for 60 seconds before the onset of a 15-second white noise tone (conditioned stimulus or CS). 30 seconds later, mice received a 1-second shock (0.7 mA intensity; unconditioned stimulus or US). Thus, one trial is composed of tone (CS), 30 seconds blank time (also called "trace"), and then shock (US). Seven trials with an intertrial interval (ITI) of 210 seconds were performed to let the mice learn the association between tone and shock across a time gap. To examine whether mice remember this association, on day 2, mice were placed into a new chamber (Chamber B) with a different shape and smell from those in Chamber A. After 60 seconds, a 15-second tone was repeated seven times with an ITI of 210 seconds. Video images were digitized and the percentage of freezing time during each ITI was analyzed by Image FZ program (O'Hara & Co). Freezing was defined as the absence of all but respiratory movement for a 1-second period.

### Results

### PAK inhibition rescues multiple behavioral defects in FMR1 KO mice

To test if the partial rescue of spine morphology and the complete rescue of cortical LTP by PAK inhibition could ameliorate behavioral deficits present in *FMR1* KO mice, the mice of various genotypes were subjected to a series of behavioral tasks. In an open field test where mice are placed in a box and allowed to run freely for ten minutes, *FMR1* KO mice exhibited three abnormal behaviors compared to wild-type mice (Peier et al., 2000, Hum. Mol. Genet., 9:1145). (1) Hyperactivity: they traveled a longer distance and moved for a longer period of time (Figure 15); (2) Stereotypy: they exhibited a higher number of repetitive behaviors (Figure 15); and (3) Hypo-anxiety: they stayed in the center field for a longer period of time and in the corners of the field for a shorter period of time (Figure 15). In all three behaviors, dMT mice exhibited performance comparable to wild-type controls (Figure 15). This indicated that PAK inhibition in *FMR1* KO mice restores locomotion, repetitive behavior, and anxiety to wild-type levels.

To further examine whether PAK inhibition can rescue abnormal cortex-dependent behaviors, trace fear conditioning was performed, which is a test that depends on the integrity of the prefrontal cortex and is sensitive to attention-distracting stimuli (McEchron et al., 1998, Hippocampus, 8:638; and Han et al., 2003, Proc. Natl. Acad. Sci., USA, 100:13087). It was previously shown that *FMR1* KO mice are impaired in this form of conditioning, which may relate to the attention deficits in FXS patients (Zhao et al., 2005, J. Neurosci., 25:7385).
In this task, a conditioning trial was composed of a tone (as the conditioned stimulus or CS), then a 30-second time gap (also called "trace") and finally an electric shock (as the unconditioned stimulus or US). Seven trials were given to allow the mice to learn the association between the tone and the shock across the 30-second time gap. Mice that learn and remember this association will become immobile (or "freeze") in response to the tone, even when they are placed into a new chamber with a different shape and smell compared to the training chamber. During training, the four genotypes exhibited comparable amounts of freezing in all conditioning trials (Figure 16), suggesting normal memory acquisition. However, when placed in a new chamber 24 hours after training, both *FMR1* KO mice and *dnPAK* TG mice exhibited a significant reduction in tone-induced freezing compared to wild-type controls (Figure 16), indicating an impaired trace fear memory in these two genotypes. dMT mice also showed freezing deficits during the first several tone sessions (sessions 1 to 4) compared to wild-type controls (Figure 16), although the deficits during these sessions were, on average, less pronounced compared to *dnPAK* TG or *FMR1* KO mice (Figure 17). However, with additional tone sessions (sessions 5 to 7), freezing by dMT caught up to that of wild-type while its difference from *FMR1* KO mice almost reached statistical significance (p = 0.07, Figure 17). Thus, dMT mice are slow in expressing the memory and/or require a repetition of the recall cue (tone), but they can eventually (after 5 tone sessions) recall the memory at the level that is not significantly different from the wild-type level.

### Example 4: PAK 1 and FMRP physical/y interact

### Materials and Methods

### Animal Handling, Experimental Design, and Data Analysis

All strains of mice are of the C57B6 background. *FMR1* KO mice were obtained from Dr. Steven Warren. *dnPAK* TG mice were generated previously (Hayashi et al., 2004, Neuron, 42:773). Mouse maintenance and all experimental procedures were performed in compliance with National Institute of Health guidelines. All experiments were conducted in a blind fashion. Unless specified otherwise, data were analyzed with Statview software (SAS) using one-way ANOVA test followed by Fisher's protected least significance difference (PLSD) *post hoc* test. Values are presented as mean ± SEM.

### lmmunoprecipitation and Western Blotting

Mouse brains were homogenized in ice-cold homogenization buffer (0.32 M sucrose; 10 mM Tris-HCl, pH 7.4; 5 mM EDTA; Complete Protease Inhibitor Cocktail Tablets (Roche)) and centrifuged at 1,000 x g for 10 minutes at 4 °C. The supernatant was collected and centrifuged at 21,000 x g for 15 minutes at 4 °C. The pellet was resuspended in TE buffer (10 mM Tris-HCl pH 7.4; 5 mM EDTA) and one-ninth volume of cold DOC buffer (500 mM Tris-HCl, pH 9.0; 10% sodium deoxycholate) was added. The mixture was incubated in a 37 °C water bath for 30 minutes while shaking and mixed with one-ninth volume of Buffer T (1% Triton X-100; 1% sodium deoxycholate; 500 mM Tris-HCl, pH 9.0). The membrane extract was dialyzed against binding/dialysis buffer (50 mM Tris-HCl, pH 7.4; 0.1% Triton X-100) at 4 °C overnight.

For immunoprecipitation, the dialyzed membrane extract was pre-cleared with protein A-sepharose beads, then incubated with α-PAK1 (N-20 from Santa Cruz Biotech) or control rabbit serum (Sigma) in binding/dialysis buffer for 3 hours, and then incubated with protein A-sepharose beads overnight at 4 °C. To test binding specificity, α-PAK1 was also incubated with its corresponding blocking peptide (Santa Cruz Biotech) prior to incubation with the membrane extract The beads were washed three times with binding/dialysis buffer.

Proteins that bound to the beads were separated by SDS-PAGE, transferred to a nitrocellulose membrane, and subjected to western blot analysis. For PAK1 western blots, the membrane was blocked in 10% milk, then incubated with α-PAK1 antibody diluted at 1:1000, then incubated with α-rabbit horse radish peroxidase (HRP, Sigma) diluted at 1:1000, and then developed with enhanced chemiluminescence (ECL) Renaissance kit (New England Nuclear). For FMRP western blots, the membrane was processed with the Blast blotting amplification system (Perkin Elmer) with α-FMRP antibody (Chemicon) diluted at 1:1000, biotinylated α-mouse diluted at 1:1000, and streptavidin-HRP diluted at 1:1000.

### GST Pull-Downs

GEX6p-1 plasmid encoding GST was purchased from Pharmacia. GST-PAK1 plasmid was obtained from Dr. Joe Kissil (Kissil et al., 2003, Mol. Cell, 12:841).
Plasmids encoding FMRP and its mutants were obtained from Dr. Edouard Khandjian (Mazroui et al., 2003, Hum. Mol. Genet., 12:3087).
GST and GST-PAK1 proteins were expressed in BL21 *E. coli,* purified on glutathione sepharose 4B (GS4B) beads (Pharmacia), and dialyzed with PBS overnight. FMRP and its mutants were in vitro-translated with the TNT coupled reticulocyte lysate systems kit (Promega) and labeled with Transcend tRNA (Promega). GST or GST-PAK1 was incubated with FMRP or its mutants in binding buffer (50 mM Tris-HCl, pH 7.5; 120 mM NaCl; 10 mM MgCl_{2;} 5% glycerol; 1% Triton X-100) for 3 hours. GS4B beads were added and incubated for 1 hour. The beads were washed three times with the binding buffer. Proteins that bound to the beads were separated by SDS-PAGE, transferred to a nitrocellulose membrane, and subjected to western blot analysis. To detect in vitro-translated FMRP or its mutants, the membrane was blocked, incubated with streptavidin-HRP, washed, and developed with ECL Renaissance kit.

### Results

The morphological, electrophysiological and behavioral data presented in. Examples 1 - 3 demonstrate that PAK inhibition rescues (at least partially) multiple abnormalities in *FMR1* KO mice. To begin to understand the underlying mechanism, it was determined whether PAK1 and FMRP physically interact via immunoprecipitation followed by Western blot analysis. Since PAK1 and FMRP are both localized in synapses (Weiler et al., 1997, Proc. Natl. Acad Sci., USA, 94:5395; and Hayashi et al., 2004, Neuron, 42:773), synapse-enriched membrane extract was prepared from mouse brain and subjected the extract to immunoprecipitation with a PAK1 antibody (α-PAK1). Proteins that may co-precipitate through their direct or indirect interaction with PAK1 were separated by SDS-PAGE and subjected to Western blot analysis with an FMRP antibody. FMRP immuno-reactivity was observed in PAK1 immunoprecipitates but not in control serum immunoprecipitates (Figure 18). This interaction is specific because it did not occur when PAK1 antibody was pre-incubated with a blocking peptide, which competes with PAK 1 for binding to the PAK1 antibody, prior to immunoprecipitation (Figure 18). This result shows that the endogenous PAK1 and FMRP interact, directly or indirectly, in the brain.

To examine whether PAK 1 directly interacts with FMRP, a glutathione S-transferase (GST)-pull down assay was performed in which *in vitro*-translated FMRP was incubated with either GST or GST-tagged PAK1 (GST-PAK1). GST-PAK1, but not GST alone, bound to FMRP (Figures 19 and 20), suggesting a direct interaction between PAK1 and FMRP. FMRP contains a primary phosphorylation site at Ser 499 and three RNA-binding domains (KH1, KH2 and RGG) that are conserved among species (Figure 21; O'Donnell and Warren, 2002, Annu. Rev. Neurosci., 25:315; and Ceman et al., 2003, Hum. Mol. Genet., 12:3295). To map the PAK1-binding region on FMRP, a series of deletion or point mutants of FMRP were used in the GST-pull down assay. An FMRP mutant without the RGG box (ΔRGG) or phosphorylation domain containing Ser 499 (ΔS499) was still able to bind to PAK1, whereas an FMRP mutant without KH domains (ΔKH) or with a point mutation in the KH2 domain previously found in a human with severe FXS (1304N; Feng et al., 1997, Mol. Cell, 1:109) was unable to bind to PAK1 (Figures 20 and 21). These results show that PAK1 directly binds to FMRP and this interaction requires the integrity of the KH domains of FMRP.

### Example 5: LTD Mediated by mGluR in the Hippocampus

Long-term depression (LTD) mediated by metabotropic glutamate receptor (mGluR) can be measured in the hippocampus. Hippocampal slices are prepared in ice-cold dissection buffer (212 mM sucrose, 2.6 mM KCl, 1.25 mM NaH₂PO₄, 26 mM NaHCO₃, 5 mM MgCl₂, 0.5 mM CaCl₂, and 10 mM dextrose) and allowed to recover for 1 hours - 5 hours before recording in oxygenated (95% O₂ and 5% CO₂) 30 °C artificial cerebrospinal fluid (ACSF; 124 mM NaCl, 5 mM KCl, 1.25 mM NaH₂PO₄, 1 mM MgCl₂, 2 mM CaCl₂, 26 mM NaHCO₃, and 10 mM dextrose). Field potentials (FPs) in stratum radiatum of area CA1 are evoked by a 200 µsec current pulse to Schaffer collateral axons. Stable baseline responses are collected every 30 seconds by a stimulation intensity (10 µA - 30 µA) yielding 50% - 60% of the maximal response. mGluR-LTD is induced by a 5 minute application of mGluR agonist 3,5-dihydroxyphenylglycine (DHPG; at 100 µM) and/or by using paired-pulse low-frequency stimulation consisting of 900 pairs of stimuli delivered at 1 Hz in the presence of the N-methyl-D-aspartate receptor (NMDAR) antagonist D-(-)-2-amino-5-phosphono-pentanoic acid (D-APV; at 50 µM). DHPG is a chiral compound, and mGluR-LTD can be induced by application of RS-DHPG and S-DHPG, but typically not by application of R-DHPG. The initial slope of the field potential is recorded as an indicator of synaptic strength.

### Example 6: Synaptic Currents Mediated by AMPARs or NMDARs in the Cortex

Synaptic currents mediated by AMPA receptors (AMPARs) and/or NMDARs can be measured in the cortex. For measurement of AMPAR-mediated miniature excitatory postsynaptic current (mEPSC), 1 µM tetrodotoxin, 100 µM APV, and 10 µM bicuculline are added in a bath of ACSF. The cells are held at -80 mV, and recordings are done at 30 °C. Continuous 30 msec - 60 msec traces are collected at 8 second interval and filtered at 2 KHz. Cells with series resistance ≥13 more discarded. The measurement of NMDAR-EPSC/ AMPAR-EPSC ratio is done in ACSF containing 2 mM Mg²⁺, 10 µM bicuculline, and 50 µM glycine. NMDAR-dependent and AMPAR-dependent responses are discriminated based on their distinct kinetics and voltage dependence. Thus, NMDAR-mediated response is valued as the currents recorded at +40 mV and measured 100 msec after the response onset. The AMPAR-mediated response is taken from the peak amplitude response recorded at -80 mV.

### Example 7: Spine Morphology in dnPAK TG, FMR1 KO Mice

Spine morphology in *dnPAK TG, FMR1* KO mice ("dMT mice") is largely normal compared to wild type mice, indicating that signaling pathways mediated by PAK and FMRP antagonize each other to regulate spine morphogenesis. FMRP is known to bind to mRNA encoding Rac1, the upstream activator of PAK, and to antagonize the effect of Rac1 on dendritic development in *Drosophila.* If in the mouse, FMRP binds to and subsequently represses the translation *of Rac1* mRNA, *FMR1* removal would result in increased levels of Rac1 and enhanced Rac1/PAK-mediated signaling. Thus, in dMT mice, Rac1/PAK-mediated signaling would be reversed to wild type level by simultaneous *FMR1* removal and PAK inhibition, leading to normal actin dynamics and spine morphogenesis.

### Example 8: Analysis of FMRP in Translational Repression of Rac1, PAK1, PAK2, and/or PAK3 mRNA

In addition to an indirect signaling interaction between PAK and FMRP, possibly via FMRP's capability to bind to and repress the translation *of Rac1* mRNA, FMRP may directly bind to and repress the translation of mRNAs that encode PAKs, such as PAK1, PAK2, and/or PAK3. In this case, *FMR* removal would result in increased levels of total PAK, including active PAK. Thus, in dMT mice, the levels of active PAK would be reversed to wild type level.

### Example 9: Assaying Whether PAK Competes with RNA for Binding to FMRP KH Domains

PAK may bind to and negatively regulate FMRP's activity. Example 4 shows that the KH RNA binding domains of FMRP mediate the binding of FMRP to PAK1. Thus, PAK may compete with RNAs for binding to the KH domains of FMRP, thus relieving FMRP's activity to repress translation of these RNAs. To determine whether PAK may compete with RNAs for binding to the KH domains of FMRP, binding assays (such as immunoprecipitation and GST pulldown, described herein) may be performed in the presence of a substance that may compete with PAK for binding to the KH domains of FMRP.

In such assays, FMRP may be free in solution, fixed to a support, and/or expressed in and/or on the surface of a cell. The candidate RNAs and/or PAK may be labeled, thereby permitting detection of binding. Competitive binding formats may be performed in which one of the substances is labeled, and one may measure the amount of free label versus bound label to determine the effect of the competitor on binding. For example, PAK may be labeled and the candidate RNAs may be unlabeled in order to detect the ability of the candidate RNAs to compete with PAK for binding to FMRP.

### Example 10: Assaying Whether PAK Phosphorylates FMRP

PAK, a serine/threonine kinase, may phosphorylate FMRP. In the brain, FMRP is phosphorylated on Ser 499 and its phosphorylation status affects its association with RNA and polyribosomes. Additional putative kinase substrates in the mouse brain include Thr 454, Ser 496, Thr 501, Ser 503, Thr 517 (Ceman et al., 2003, Hum. Mol. Genet., 12:3295; and Mazroui et al., 2003, Hum. Mol. Genet., 12:3087).
Kinase assays may be performed in order to determine whether PAK phosphorylates FMRP. In such assays PAK and/or a characteristic portion thereof is contacted with FMRP and/or a characteristic portion thereof in the presence of a suitable phosphate donor, such as ATP, containing radiolabeled phosphate, and PAK-dependent incorporation of radiolabel into FMRP is measured.

PAK-dependent FMRP phosphorylation can be measured by monitoring physicochemical properties of FMRP that may occur as a result of incorporation of phosphate groups. Such properties may include electrophoretic mobility, light absorbance, fluorescence and/or phosphorescence, chromatographic properties, *etc.* Such alterations of substrate physicochemical properties can be readily measured by one skilled in the art and used as an indicator of kinase activity.

Alternatively or additionally, monoclonal or polyclonal antibodies may be generated which selectively recognize phosphorylated forms of FMRP, and thus the degree of binding of such antibodies to FMRP subsequent to the kinase reaction may be used as an indirect method of determining the ability of PAK to phosphorylate FMRP. Kinase assays may be performed using purified, partially recombinant PAK and/or FMRP, and/or PAK and/or FMRP which is purified from cells that naturally express the protein using standard purification procedures, such as those described herein.

### Example 11: PAK Modulators and Autism

FXS patients constitute ∼5% population of patients with autism, a complex multigenic disorder characterized by impaired communication, impaired social interactions and repetitive interests and behavior. Due to the difficulty in identifying susceptible chromosomal loci and genes, little is known on the causes and pathogenesis of autism. The association between FXS and autism suggests that these two disorders may share common genetic factors and underlying patho-physiological mechanisms. PAK modulators, therefore, may represent a novel treatment for certain types of autism. In support of this idea, the *PAK3* gene locates in chromosomal locus Xq22, a region linked to autism. It could, therefore, be determined whether *PAK3* and/or other *PAK* genes are mutated in autistic patients. To directly test the link between PAK modulation and autism, *dnPAK* TG mice could be crossed to available autism mouse models (Lijam et al., 1997, Cell, 90:895; Moretti et al., 2005, Hum. Mol. Genet., 14:205; and Kwon et al., 2006, Neuron, 50:377) to examine whether social behavior is reversed to wild type level in the double mutants.

### Example 12: Treatment of FMR KO Mice with Small Molecule PAK Inhibitors

### Preparation of Stock Solutions

Emodin (also known as 1,3,8-Tri-hydroxy-6-methyl-anthra-quinone; 6-Methyl-1,3,8-tri-hydroxy-anthra-quinone; Emodol; Frangula-emodin) is obtained from Sigma-Aldrich (# E7811). Emodin is an orange powder that is soluble in DMSO, ethanol, or 1 N dilute aqueous ammonia. A stock solution of 1 mg/ml - 50 mg/ml is made and stored at -20 °C.

OSU-03012 (also known as 2-amino-N-{4-[5-(2-phenanthrenyl)-3-(trifluoromethyl)-1H-pyrazol-1-yl]-phenyl} acetamide) is obtained from Cayman Chemicals (#10008005; Figures 6 and 7). OSU-03012 is a white solid that is soluble in organic solvents such as DMSO, ethanol, and DMF. A stock solution of 30 mg/ml - 100 mg/ml is made and stored at -20 °C.

### Administration of Small Molecule Therapeutics to FMR KO Mice

Drug effects are optimized by determining the ideal delivery method, dose, volume, and dosage schedule. The level of drug exposure and duration is compared in regions of the brain as well as in other organs and the blood following various drug administration methods.

Small molecule therapeutics, including, but not limited to, Emodin and/or OSU-03012, can be administered to *FMR* KO mice by injection, and injected mice can be evaluated for therapeutic efficacy. Studies are frequently performed using male mice, but heterozygous and/or homozygous female mice can also be used in accordance with the invention. Studies are frequently performed using adult mice, but prenatal, infant, and/or juvenile mice can also be utilized in accordance with the present invention.

Behavioral phenotypes vary slightly with mouse strain. In some embodiments, a C57BL6 genetic background can be used for small molecule PAK inhibitor studies. In some embodiments, a FVB/NJ (FVB) genetic background can be used for small molecule PAK inhibitor studies. In some embodiments, F1 hybrids produced by crossing a male FVB mouse to a female C57BL6 heterozygous *FMR1* KO can be used for small molecule PAK inhibitor studies. In some embodiments, F1 hybrids produced by crossing a male C57BL6 mouse to a female FVB heterozygous mouse can be used for small molecule PAK inhibitor studies.

In general, small molecule PAK modulators are frequently soluble in organic solvents, but not in aqueous solutions. A variety of different solvents are tested to achieve maximal therapeutic effects and minimal adverse side effects. For example, small molecule therapeutics (*e.g*. Emodin and/or OSU-03012) are formulated using water, saline (*e.g.* phosphate-buffered saline), 2-hydroxypropyl-beta-cyclodextrin, Tween, methyl cellulose, polyethylene glycol, Cremophor EL (Sigma), oils (*e.g*. vegetable oil), and/or Maalox. The small molecule agent may be formulated as a homogenous solution or colloidal dispersion.

A variety of amounts of Emodin and/or OSU-03012 are administered to *FMR* KO mice. For example, dosages of about 5 µl, about 10 µl, about 20 µl, about 50 µl, about 100 µl, about 500 µl, about 1000 µl, about 2000 µl, and/or about 3000 µl of Emodin and/or OSU-03012 can be administered per dose. Typically, dosages of about 50 µl to about 1.0 ml are utilized.

Emodin and/or OSU-03012 can be administered at concentrations of about 0.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 20 mg/kg, about 50 mg/kg, about 100 mg/kg, about 250 mg/kg, or about 500 mg/kg per dose (mg/kg corresponds to # mg Emodin and/or OSU-03012 per kg of animal subject).

A variety of dosage schedules for emodin and/or OSU-03012 are utilized. Emodin and/or OSU-03012 are administered one time only and/or are administered multiple times (e.g. at regular intervals). For example, Emodin and/or OSU-03012 can be administered one time per month, two times per month, one time per week, two times per week, three times per week, every other day, one time per day, two times per day, three times per day, and/or four times per day.

Emodin and/or OSU-03012 can be administered by oral, intravenous, intramuscular, intra-arterial, intramedullary, intrathecal, parenteral, subcutaneous, intraventricular, transdermal, interdermal, rectal, intravaginal, intraperitoneal, topical (e.g. by powders, ointments, creams, gels, and/or drops), transdermal, mucosal, nasal, buccal, enteral, and/or sublingual administration; by intratracheal instillation, bronchial instillation, and/or inhalation; and/or as an oral spray, nasal spray, and/or aerosol. In some embodiments, Emodin and/or OSU-03012 can be administered by a continuous IV drip. In some embodiments, Emodin and/or OSU-03012 can be administered by retro-orbital injection.

Time lapse between last dose and biochemical or behavioral analyses ranges between 15 minutes and 2 days. For example, time lapse between last dose and biochemical or behavioral analyses can be approximately 15 minutes, approximately 30 minutes, approximately 1 hour, approximately 2 hours, approximately 3 hours, approximately 4 hours, approximately 5 hours, approximately 6 hours, approximately 9 hours, approximately 12 hours, approximately 15 hours, approximately 18 hours, approximately 24 hours, approximately 30 hours, approximately 36 hours, approximately 42 hours, approximately 48 hours, approximately 60 hours, or approximately 72 hours.

### Exemplary Dosage Schedule #1

Adult male *FMR1* KO mice and littermates are administered either placebo or PAK inhibitor (1 mg/kg, 10 mg/kg, 30 mg/kg, 100 mg/kg, or 300 mg/kg) in a total volume of 200 µl via oral gavage. Mice are monitored for signs of toxicity or decline in health. At various time points (1 hour, 4 hours, 12 hours, or 24 hours) following the single drug administration, animals are sacrificed. At least two mice are used for each of the 48 conditions. In some experiments, between two and ten mice are used. In some experiments, more than 10 mice are used. In some experimental runs, only a subset of these conditions is performed. At the time of sacrifice, blood, urine, and organs are collected. Brains are dissected into subregions, including the hippocampus, cortex and cerebellum. Analysis of drug concentration and efficacy in various fluids and tissues is used to inform future studies by suggesting optimal drug dosing concentrations and schedules.

### Exemplary Dosage Schedule #2

Adult male *FMR1* KO mice and littermates are treated with PAK inhibitor (*e.g*. a small molecule such as Emodin and/or OSU-03012) for 3 days, 1 week, 2 weeks, or 4 weeks prior to behavior analysis. Mice of each genotype will be randomly divided into three dosing groups with at least two mice in each group: vehicle control and drug treated (10 mg/kg or 100 mg/kg; or another dose found to be optimal in the previously described pilot experiment). In some experiments, about five mice are used. In some experiments, about ten mice are used. In some experiments, more than 10 mice are used. All drugs are administered once per day in a total volume of 200 µl by oral gavage. In some experimental runs, only a subset of these conditions is performed. Behavior experiments - including open field, trace fear conditioning, and/or social interaction experiments - are conducted 12 - 18 hours after the last dosing (or at another time point found to be optimal in the previous dosing experiment). Mice are sacrificed immediately following final behavior assay and subregions of the brain, as well as other organs and blood, are collected and stored.

### Exemplary Dosage Schedule #3

Male *FMR1* KO pups and littermates to be used for audiogenic seizure (AGS) experiments are treated with PAK inhibitor (*e.g*. a small molecule such as Emodin and/or OSU-03012) for 1 day, 3 days, 5 days, or 7 days immediately prior to post natal day 20 (p20), at which time behavioral testing occurs. Therefore, PAK inhibitor treatments begin when the pups are p13, p15, p17, or p19. Subcutaneous injections of placebo or PAK inhibitor (1 mg/kg, 3 mg/kg, or 10 mg/kg) in a total volume of 50 µl/g body weight are given twice per day. For each of the 16 conditions (combinations of days of administration and drug dose), 6 - 8 *FMR1* KO pups and 2 control littermates are used. In some experimental runs, only a subset of these conditions is performed. Behavior experiments are conducted 4 8 hours after final injection.

One of ordinary skill in the art will readily recognize that these are exemplary, dosage schedules and are not meant to be limiting. A skilled artisan will recognize how the conditions of these exemplary dosage schedules may be modified in order to optimize dosage. Such optimizations are contained within the scope of the present invention.

### Post-Treatment Biochemical Analyses

After treatment, mice are subjected to a number of biochemical analyses in order to evaluate the efficacy of small molecule treatment. Typically, biological samples to be used in biochemical analyses are obtained post-mortem. However, biochemical analyses can be performed using biological samples obtained from living mice.

After the final administration of the compound, mice are subjected to a number of biochemical analyses in order to evaluate the bioavailability and efficacy of small molecule treatment. Mice are sacrificed at 1 hour, 2 hours, 6 hours, 12 hours, 24 hours, or 48 hours after final drug administration either by cervical dislocation or overdose of anesthesia (e.g. Avertin or Isoflurane), and blood is collected in heparinized tubes immediately thereafter by cardiac puncture. Blood plasma is separated by centrifugation (*e.g*. 20,000 x g), frozen, and stored at -80 °C until analysis. Cerebrospinal fluid (CSF) is collected from the ventricles, briefly centrifuged to pellet cell debris, frozen, and stored at - 80 °C until analysis. To determine route and rate of elimination of the compound, urine is collected from the bladder, briefly centrifuged to pellet cell debris, and the supernatant frozen and stored at 80 °C until analysis. Using dissection, tissues including liver, kidney, spleen, lymph node, heart, lung, muscle, skin, spinal cord and brain, which can be subdivided into multiple areas including but not limited to cerebral cortex, hippocampus, and cerebellum are collected. Emodin, OSU-03012, and their metabolites are extracted from tissues using methanol.

For determination of Emodin and metabolite concentrations, samples (*e.g*. blood plasma, CSF, urine, and methanol extracts from tissues) are subjected to High-Performance Liquid Chromatography (HPLC) using a C18 column as the solid phase and either 88%methanol:12% 0.1%phosphoric acid (water) or 40:60 0.1M phosphoric acid:methanol as the mobile phase. Emodin is detected by UV absorbance at 220 nm. Samples are spiked with a known amount of emodin prior to HPLC as an internal standard. The amount of emodin in the sample is determined by comparison to the known amount of internal standard, or with samples obtained from untreated mice spiked with known amount of emodin. The concentration of emodin is thus determined on a per mL basis (blood plasma /urine) or per mg basis (sample), based upon the amount of sample used for the procedure.

For determination of OSU-03012 and metabolite concentrations, samples (*e.g*. blood plasma, CSF, urine, and methanol extracts from tissues) are subjected to High-Performance Liquid Chromatography (HPLC) using a C18 column as the solid phase and a linear gradient from acetonitrile/0.025 M ammonium acetate, pH 4.5 (20:80) to acetonitrile/0.025 M ammonium acetate, pH 4.5 (60:40) as the mobile phase. OSU-03012 and metabolites are detected by UV absorbance at 240 nm or by fluorescence with excitation at 240 nm and emission at 380 nm. Samples are spiked with a known amount of OSU-03012 prior to HPLC as an internal standard. The amount of OSU-03012 in the sample is determined by comparison to the known amount of internal standard, or with samples obtained from untreated mice spiked with known amounts of OSU-03012. The concentration of OSU-03012 is thus determined on a per mL basis (blood plasma / urine) or per mg basis (sample), based upon the amount of sample used for the procedure.

Pharmacokinetic analysis includes plotting mean plasma, CSF, and/or urine concentrations of drug as a function of time, and calculating area under the curve (AUC) for different times post-injection. Half-life of PAK inhibitors is determined by fitting the concentration-time curve after the peak levels have been reached to a single exponential. Peak levels of small molecule PAK inhibitor in collected fluids are determined for each drug, dose, and time point combination.

Similarly, blood and urine can be collected and analyzed at set intervals throughout the dosage regimen. For example, the peri-orbital sinus can be used as a source of venous blood, and approximately 0.25 ml of blood can be safely collected in a capillary tube at weekly intervals under anesthesia. Similarly, tail vein venipuncture can be used to collect small blood volumes (typically a few drops) from restrained mice.

Post-mortem, various tissues are collected and analyzed for drug compounds and associated metabolites (as described for blood, CSF, and urine samples) and efficacy of PAK inhibition. Tissues collected include liver, kidney, spleen, lymph node, heart, lung, muscle, skin, spinal cord and brain, which can be subdivided into multiple areas including but not limited to cerebral cortex, hippocampus, and cerebellum. Efficacy of drug treatment in the brain is determined by homogenization of brain tissue, followed by gel electrophoresis and Western blotting to monitor activities of PAK and its downstream effectors through the use of phospho-specific antibodies.

These Western blots are performed as previously described (Hayashi et al., 2004, Neuron, 42:773; and Kelleher et al., 2004, Cell, 116:467).
Briefly, mouse cerebral cortex, hippocampus, and cerebellum are dissected and homogenized in cold (*e.g*. 4 °C) RIPA buffer (50 mM Tris-HCl pH 7.4, 150 mM NaCl, 1% NP-40, 0.5% sodium deoxycholate, 0 mM or 1mM SDS) with protease (*e.g*. some or all of Roche Complete®, Mini Complete®, Calbiochem Protease Inhibitor Cocktails I, II and III®), and phosphatase (*e.g*. some or all of Calbiochem Phosphatase Inhibitor Cocktails I, II and III®) inhibitors. Homogenates are centrifuged to remove debris and protein concentrations are determined (*e.g*. Biorad Bradford assay, Biorad Rc-Dc assay, or Molecular Probes NanoOrange protocols as per manufacturer's instructions). In some cases, further centrifugation through a sucrose gradient and treatment with detergents is conducted to fractionate the extracts, thereby allowing enrichment of membrane fractions, synaptosome fractions, and post-synaptic density fractions. These fractions are subjected to Western blot analysis using antibodies (*e.g*. obtainable from Cell Signaling) that recognize phospho-specific forms of PAK and downstream signaling proteins, including p-PAK1 Thr 423 antibody (recognizes phosphorylated forms of PAK1, PAK2, and PAK3), dually phosphorylated p-ERK1/2, p-S6 (S235/S236), p-eIF4E (S209), p-4E-BP1 (S65), and p-Akt (S473). Alternatively or additionally, these fractions are subjected to Western blot analysis using a p-vimentin antibody (S56; Medical & Biological Laboratories). Blots are stripped and reprobed with anti-sera against total PAK1 (Santa Cruz), PAK3 (Upstate), ERK1/2 (Cell Signaling), S6 (Cell Signaling), eIF4E (Cell Signaling), 4E-BP1 (Cell Signaling), and Akt (Cell Signaling). Results are quantified with software (*e.g*. NIH Image J or Li-Cor Odyssey).

Alternatively or additionally, the extent to which small molecule inhibitors like Emodin and OSU-03012 inhibit endogenous PAK catalytic activity in the mouse brain is determined using an *in vitro* kinase assay as described in Hayashi et al., 2004, Neuron, 42:773; and Zenke et al., 1999, J. Biol. Chem., 274:32565).
Briefly, catalytic activity of PAK can be stimulated by GTP-γS-Rac in total forebrain homogenates and measured by the amount of phosphorylated myelin basic protein (MBP).

For Western blots and *in vitro* PAK kinase assays, level of PAK activity (phospho-protein levels for all mentioned proteins above relative to no-drug condition or *in vitro* PAK catalytic activity level relative to no-drug condition) is monitored as a function of dose and dosage length.

Measurements of drug toxicity are conducted by monitoring weight loss, death, motor activity (*e.g*. open-field test), and/or coordination (*e.g*. rotorod) for all mentioned drug doses and throughout the dosage schedules.

In some embodiments, the optimum concentration of PAK to be used for behavior and electrophysiology experiments is approximately the smaller of the lowest amount of inhibitor required to achieve maximum inhibition of PAK activity (*e.g*. from Western Blot and/or kinase assay studies) or the lowest amount inhibitor that causes adverse toxic effect (*e.g*. weight loss, death, motor activity difference, coordination problems, *etc.*).

Typically, one group of mice is sacrificed and utilized in biochemical experiments (*e.g*. to determine the level of PAK kinase activity inhibition in the brain for various dosing schedules, times after final administration, *etc*.) A separate group of mice are used for behavior analyses. A mouse can be used for a single behavioral test or a battery of tests. If a single mouse is used for multiple behavioral tests, the tests are typically performed in the following order: open field, social-interaction, trace fear conditioning, and audiogenic seizure analysis.

### Post-Treatment Morphological Analyses

After treatment, mice are subjected to a number of histological and/or morphological analyses in order to evaluate the efficacy of small molecule treatment A few exemplary histological and/or morphological analyses are described below and are explained in further detail in Example 1.

Since dendritic spine abnormalities are a hallmark of loss of FMRP in both humans and mice, Golgi analysis is performed on *FMR* KO mice following PAK inhibitor administration using the Golgi-Cox technique. Dendritic spine density in areas of the cortex, such as pyramidal neurons of layer II/III of temporal cortex, is measured on each primary or secondary apical or basal dendritic branch in 10 µm long dendritic segments. Spine number per 10 µm segment and mean spine density averaged over segments are quantified and compared (as in Figures 11 and 12). *FMR* KO mice show increase spine density with respect to their wild-type littermates. This phenotype is rescued or partially rescued by PAK inhibition in the *FMR* KO mice.

The Golgi preparation is used to quantify and compare dendritic spine length. Increased spine length, and perhaps concurrent decreased synapse size, of Golgi stained pyramidal neurons is observed in *FMR* KO mice and may be rescued by inhibition of PAK activity (as in Figure 13). Spine length is measured as the radial distance from the tip of the spine head to the dendritic shaft (see, *e.g*. Hayashi et al., 2007, Proc. Natl. Acad. Sci., USA, 104:11489; and Ramon-Moliner, 1970, Contemporary Research Methods in Neuroanatomy, Springer, Berlin).

Dendritic spine abnormalities are also observed in green fluorescent protein (GFP) labeled neurons in the cortex, such as those in layer V neurons of the barrel cortex or cortical neurons in culture. Neurons are labeled with GFP through the use of either a viral vector injected into the cortex or a transgenic mouse that expresses this fluorescent protein in some cortical neurons. Fluorescent neurons are imaged in fixed brain sections or primary cultures using a two-photon laser scanning microscope. Spine length and density are quantified as described above. *FMR* KO neurons display increased spine length and increased spine density, as phenotype which may be rescued by inhibition of PAK kinase activity (see, *e.g*., Nimchinsky et al., 2001, J. Neurosci., 21:5139; and Livet et al., 2007, Nature, 450:56).

### Post-Treatment Analysis of LTP and LTD

In some embodiments, hippocampal synaptic plasticity is assessed using a standard protocol for mGluR-LTD (see, *e.g*. Example 5 for a more detailed protocol). Hippocampal slices are prepared from *FMR* KO mice and control littermates per standard procedures. All experiments are performed blind to genotype. Schaffer collaterals are stimulated and extracellular field potentials measured *in str. radiatum* of CA1. mGluR-dependent LTD is elicited both electrophysiologically (by pairs of stimuli delivered at 1 Hz for 15 minutes to 20 minutes; "PP-LFS") and pharmacologically (by bath application of 50 µM to 100 µM 3,4-dihydroxyphenylglycine (DHPG) for 5 minutes). The initial slope of the field potential is recorded as an indicator of synaptic strength. Hippocampal slices from *FMR1* KO mice show enhanced mGluR-LTD relative to control mice (see, *e.g*., Huber et al., 2002, Proc. Natl. Acad. Sci., USA, 99:7746; and Nosyreva and Huber, 2006, J. Neurophysiol., 95:3291).

Cortical long-term potentiation (LTP) is reduced in slices from *FMR1* KO mice (see Figure 14). Coronol brain slices containing temporal cortex are prepared from two- to three-month-old male littermates, and left to recover for at least 1 hour before recording in oxygenated (95% O₂ and 5% CO₂) warm (30 °C) artificial cerebrospinal fluid containing 124 mM NaCl, 5 mM KCl, 1.25 mM NaH₂PO₄, 1 mM MgCl₂, 2 mM CaCl₂, 26 mM NaHCO₃, 10 mM dextrose. Field potentials (FPs) in layer II/III evoked by layer IV stimulation are measured as previously described and responses are quantified as the amplitude of FP in cortex. LTP is induced by TBS, which consisted of eight brief bursts (each with 4 pulses at 100 Hz) of stimuli delivered every 200 msec. Genetic inhibition of PAK rescues the reduced cortical LTP in the *FMR1* KO mice, and the present invention encompasses the recognition that pharmacological inhibition may have the same effect (Hayashi et al., 2007, Proc. Natl. Acad. Sci., USA, 104:11489).

### Post-Treatment Behavioral Analyses

Fragile X mice and littermates treated with PAK inhibitors or placebo are subjected to various behavioral tasks to determine whether pharmacological inhibition of PAK can ameliorate symptoms of Fragile X Syndrome in the mouse model. The experimenter is blind to genotype and prior drug treatment.

### Open Field Test

As described in the specification, the open field test records parameters such as horizontal and vertical activity, time spent in the center, and stereotopy. Mice are allowed to run freely in an open arena (e.g. VersaMax activity monitor chamber from Accuscan Instruments). Mice are allowed to run about 5 minutes, about 10 minutes, about 15 minutes, about 20 minutes, about 30 minutes, or about 1 hour. After mice are allowed to run, behaviors are analyzed, including (1) hyperactivity, determined by measuring the distance and/or length of time traveled by the subject; (2) stereotypy, determined by measuring the number of repetitive behaviors exhibited by the subject; (3) hypo-anxiety, determined by measuring the amount of time the subject remains in the center field relative to the time spent in the corners of the field; and (4) combinations of these.

In certain experiments, open field activity can be detected by photobeam breaks and analyzed by VersaMax software. Activities measured are the amount of time the mouse spends in the center of the field, the number of times the mouse exhibits repetitive behaviors ("stereotypy"), and the total distance traveled by the mouse.

### Audiogenic Seizure (AGS) Assay

Fragile X humans and mice are susceptible to seizures at early ages. Fragile X mice show a robust phenotype in an audiogenic seizure (AGS) assay. While no 19 - 21 day old (p19-21) wildtype mice typically have seizures in this task, the majority of fragile X mice do have seizures.

AGS is performed essentially as described (Yan et al., 2005, Neuropharmacol., 49:1053). Briefly, mice are habituated to a behavioral chamber and then exposed to a high intensity siren of frequency peak 1800 Hz - 6300 Hz at an average sound pressure level above 120 dB at approximately 10 cm for 5 minutes. Behaviors of the mice are monitored after administration of sound. Fragile X mice typically (1) run wildly, (2) have seizures, and/or (3) die. Wildtype mice typically do not exhibit these responses. An AGS phenotype is scored based on the animal's endpoint.

### Trace Fear Conditioning

Mice are subjected to the trace fear conditioning task according to standard procedures. On day 1 ("conditioning"), mice are placed into a training chamber for ∼60 seconds before the onset of a ∼15-second white noise tone. Another -30 seconds later, mice received a ∼1-second shock (∼0.7 mA intensity). Thus, one trial is composed of tone, 30 seconds blank time (also called "trace"), and then shock. Five to ten trials with an intertrial interval (ITI) of 210 seconds are performed. To examine whether mice remember this association, on day 2 ("tone test"), mice are placed into a new chamber with a different shape and smell from the first chamber. After ∼60 seconds, a ∼15-second tone is repeated for five to ten times with an ITI of 210 seconds. Video images are digitized and the percentage of freezing time during each ITI is analyzed (*e.g*. by Image FZ program). Freezing is defined as the absence of all but respiratory movement for a 1-second period. See, *e.g.* Zhao et al. (2005, J. Neurosci., 25:7385).

### Social Interaction Tests

Home cage behavior and social interaction are assessed with a variety of tests (Kwon et al., 2006, Neuron, 50:377; Spencer et al., 2005, Genes Brain Behav., 4:420; and Lijam et al., 1997, Cell, 90:895). For example, mice can be observed in their home cage by videorecording and scored for various nonsocial behaviors and/or social behaviors (e.g. grooming, mounting, tail pulling, and sniffing).

Direct social interaction is assessed by exposing mice to a novel conspecific mouse and observing approaching and sniffing behaviors. Percent of time spent interacting is recorded. This is repeated about 3 days later with the same mice. Control mice exhibit a decrease in social interaction the second time, indicating recognition of the familiar mouse and/or normal social learning. *FMR* KO mice do not exhibit a decrease in social interaction the second time, indicating impaired social learning, memory, and/or behavior. This test can be conducted in a novel or familiar environment, as significant differences between *FMR* KO and wild-type mice have been observed in social interaction assays depending on the degree of familiarity with the environment. Additional social behaviors can be monitored in this assay including active behaviors (*e.g*. aggressive attacks, lateral threats, and/or chasing) and passive behaviors (*e.g*. receiving sniffing from other mouse and/or not showing signs of submissive and/or defensive behavior).

Prior to an indirect social interaction test mice are housed individually for 4 days. Indirect social interaction tasks typically take place in a cage divided in half by a clear perforated partition. The task is run in two different modes: one involves a familiar environment, by pre-exposure to the testing chamber, while the other involves a novel environment. Test mice are exposed to novel or familiar mice. Typically, *FMR* KO mice behave similarly to wild-type mice in a novel environment, but behave significantly differently in a familiar cage. Time spent at the partition is recorded in 2 to 5 minute intervals for a 20 minute test. *FMR* KO mice spend significantly less time interacting (i.e. at the partition) in the first few minutes and take longer to first approach the partition than wild-type mice. In contrast, *FMR* KO mice spend more time at the partition during the last time intervals than controls.

In some experiments, the indirect social interaction test is conducted in a chamber divided into three rooms. The central room - which is connected to two rooms independent from each other, one on the left and one on the right - is empty. The left room contains an empty cage (*i.e.* a novel object and/or inanimate target), while the right room contains a similar cage enclosing a novel mouse (i.e. a social target). This task involves a choice between spending time with a social target or an inanimate target, and therefore is called a social preference test. Percent of total time interacting with each object is recorded. *FMR* KO mice may spend a significantly different amount of time (*e.g*. more or less) interacting with the social target than control mice.

Social dominance tube test is conducted in a tube approximately 30 cm long and 3 cm - 4 cm in diameter. Mice are placed at opposite ends of the tube and released simultaneously. A mouse is pronounced the "winner" when his opponent backs out completely. *FMR1* KO mice win significantly fewer matches against unfamiliar wild-type mice than expected by chance. When a *FMR1* KO mouse competes against a wild-type non-cagemate, the wild-type mouse is the winner in approximately 73% of matches (Kwon et al., 2006, Neuron, 50:377; Spencer et al., 2005, Genes Brain Behav., 4:420; and Lijam et al., 1997, Cell, 90:895). Inhibition of PAK via administration of a small molecule inhibitor may ameliorate this phenotype.

Since *FMR1* KO mice have been shown to display some abnormal social behaviors, home cage social behavior, including nest building and sleeping behavior, may be altered in *FMR1* KO mice. Nesting patterns are evaluated by placing a cotton nestle into a cage of approximately two, three, or four mice of the same genotype (*e.g.* wild-type, *FMR1* KO, *etc.*) that receive identical drug treatments. After about 30 minutes to 1 hour, the nest can be removed and the height measured. Wild-type mice build nests with depths that average 20 mm to 50 mm. Mice which display abnormal social behavior, such as *FMR1* KO mice, frequently build shallower nests (*e.g*. < 20 mm).

In another assay, sleeping positions of mice in their home cages can be recorded two to four times a day over five consecutive days. Wild-type mice sleep huddled in the well-formed, fluffy nests they build. Observations will determine whether *FMR1* KO mice sleep in scattered, random patterns, do not build full nests, or sleep on top of intact nestle material. If *FMR1* KO mice display abnormal phenotypes, amelioration of those phenotypes may occur following PAK inhibition using small molecule inhibitors such as Emodin and/or OSU-03012.

### Eight-Arm Maze Test

FXS patients have cognitive deficits (*e.g*. short-term memory impairments) and/or perseverative language and movements. FXS patients often become anxious with any breach of their normal daily routine. The 8-arm radial maze is a task used to see if a similar phenotype is also present in the mouse model of the disease. The maze consists of a central arena surrounded by eight doors which guard the entrances to eight maze arms. Prominent distal cues surround the maze. A food pellet is hidden at the distal end of the arms and serves as a reward. In a version of the task specifically designed to test working memory, all 8 arms are baited with a food pellet. In each trial, the mouse must visit each of the 8 arms only once to consume the food reward. A second visit to an arm is considered a working memory error. Mice with poor short-term memory accrue more errors than wild-type mice. In the reference memory version of the task, only 4 arms are baited. In this case, the same 4 arms are baited each day during the training period (which typically lasts 10 - 16 days with 1 - 3 trials per day). An entry into an unbaited arm is considered a reference memory error. Just as in the previous version of the task, a second entry into any arm is considered a working memory error. Once the mice have learned the task, a reversal phase begins in which the previously unbaited arms now have the 4 food pellets and the previously baited arms contain no food reward. Mice with deficits in memory flexibility are unable to learn the new task and continue to visit the arms baited in the training phase. Errors are recorded and compared among *FMR* KO mice that receive PAK inhibitor, placebo, or no treatment.

### Morris Water Maze Test

Hippocampus-dependent spatial learning is assessed using the classical Morris Water Maze test. *FMR1* KO mice, just like the wild-type controls, learn to find the visible or hidden platform with decreasing latency scores over the course of the standard training protocol. However, some groups observe an abnormal phenotype in a reversal trial, a test in which the platform is transferred to the quadrant opposite the initial training quadrant. In particular, *FMR1* KO mice display increased escape latency and path length, suggesting that they have low response flexibility or high memory interference (see, *e.g.* D'Hooge et al., 1997, Neuroscience, 76:367*).*

### Equivalents and Scope

In the claims articles such as "a,", "an" and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context.

## Claims

1. At least one inhibitor of p21-activated kinase (PAK) for use in treating a neurodevelopmental disorder.

2. The inhibitor for the use of claim 1, wherein the neurodevelopmental disorder is selected from fragile X syndrome, mental retardation, autism spectrum disorders, premature ovarian failure or fragile X associated tremor ataxia.

3. The inhibitor for the use of claim 2, wherein the fragile X syndrome is associated with reduced long-term potentiation (LTP) in the cortex or enhanced long-term depression (LTD).

4. The inhibitor for the use of claim 3, wherein administration of the inhibitor to a subject results in decreased long-term depression (LTD) in the hippocampus or increased long-term potentiation (LTP) in the cortex.

5. The inhibitor for the use of claim 1 or claim 2, wherein the inhibitor is administered as an oral dose.

6. The inhibitor for the use of claim 1 or claim 2, wherein the inhibitor of PAK inhibits the interaction of PAK with FMRP

7. The inhibitor for the use of claim 1 or claim 2, wherein the inhibitor of PAK is Emodin, OSU-03012, staurosporin, PD098059, genistein tyrphostin B42, HA 1077, K252a, 1(5-isoquinoline-sulfonyl)-2- methylpiperazine (H-7), CEP- 1347, hPIP, Merlin, Nischarin, P35/CDK5, CDC2, p 11OC, POPX1, POPX2, CRIPak, PAK1 amino acids 89 - 143, GL-2003, SUl 1652, or Purvalanol A.

8. The inhibitor for the use of claim 1 or claim 2, wherein the inhibitor of PAK is a short interfering RNA (siRNA) or a short hairpin RNA (shRNA).

9. The inhibitor for the use of claim 1 or claim 2, wherein the inhibitor of PAK is an antibody.

10. The inhibitor for the use of claim 1 or claim 2, wherein the inhibitor of PAK is administered in combination with an additional therapeutic agent, for example an anti-seizure agent, a mood stabilizer, a central nervous system stimulant, an antihypertensive agent, folic acid, a selected serotonin reuptake inhibitor, or an antipsychotic agent.

11. A method of screening for a candidate substance useful for treating a neurodevelopmental disorder including fragile X syndrome, mental retardation, autism spectrum disorders, premature ovarian failure or fragile X associated tremor ataxia comprising steps of:
providing p21-activated kinase (PAK);
providing a natural binding partner of PAK or a phosphorylation substrate of PAK;
administering at least one candidate substance to PAK and the natural binding partner or the phosphorylation substrate; and
measuring the effect of the at least one candidate substance on the binding interaction,
measuring the ability of the at least one candidate substance to compete with the binding interaction between PAK and the natural binding partner or measuring the effect of the at least one candidate substance on ability of PAK to phosphorylate the substrate, and thereby identifying a substance that inhibits PAK and is useful for treating a neurodevelopmental disorder including fragile X syndrome, mental retardation, autism spectrum disorders, premature ovarian failure or fragile X associated tremor ataxia.

12. Use of at least one inhibitor of p21-activated kinase (PAK) in the manufacture of a medicament for treating a neurodevelopmental disorder.

13. The use of claim 12, wherein the neurodevelopmental disorder is selected from fragile X syndrome, mental retardation, autism spectrum disorders, premature ovarian failure or fragile X associated tremor ataxia.

## Patentansprüche

1. Mindestens ein Inhibitor der p21-aktivierten Kinase (PAK) für die Verwendung beim Behandeln einer neurologischen Entwicklungsstörung.

2. Inhibitor für die Verwendung nach Anspruch 1, wobei die neurologische Entwicklungsstörung aus Fragilem-X-Syndrom, geistiger Retardierung, Störungen aus dem Autismusspektrum, vorzeitiger Ovarialinsuffizienz oder Fragiles-X-assoziiertem Tremor-/Ataxiesyndrom ausgewählt ist.

3. Inhibitor für die Verwendung nach Anspruch 2, wobei das Fragile-X-Syndrom mit reduzierter Langzeit-Potenzierung (LTP) im Kortex oder verstärkter Langzeitdepression (LTD) assoziiert ist.

4. Inhibitor für die Verwendung nach Anspruch 3, wobei die Verabreichung des Inhibitors an ein Individuum zu verringerter Langzeitdepression (LTD) im Hippocampus oder zu einer erhöhten Langzeit-Potenzierung (LTP) im Kortex führt.

5. Inhibitor für die Verwendung nach Anspruch 1 oder Anspruch 2, wobei der Inhibitor als orale Dosis verabreicht wird.

6. Inhibitor für die Verwendung nach Anspruch 1 oder Anspruch 2, wobei der Inhibitor von PAK die Interaktion von PAK mit FMRP hemmt.

7. Inhibitor für die Verwendung nach Anspruch 1 oder Anspruch 2, wobei es sich bei dem Inhibitor von PAK um Emodin, OSU-03012, Staurosporin, PD098059, Genisteintyphostin B42, HA 1077, K252a, 1(5-Isochinolinsulfonyl)-2-methylpiperazin(H-7), CEP-1347, hPIP, Merlin, Nischarin, P35/CDK5, CDC2, p11OC, POPX1, POPX2, CRIPak, PAKI Aminosäure 89-143, GL-2003, SU1 1652 oder Purvalanol A handelt.

8. Inhibitor für die Verwendung nach Anspruch 1 oder Anspruch 2, wobei es sich bei dem Inhibitor von PAK um eine kurze interferierende RNA (siRNA) oder eine kurze Haarnadel-RNA (shRNA) handelt.

9. Inhibitor für die Verwendung nach Anspruch 1 oder Anspruch 2, wobei es sich bei dem Inhibitor von PAK um einen Antikörper handelt.

10. Inhibitor für die Verwendung nach Anspruch 1 oder Anspruch 2, wobei der Inhibitor von PAK in Kombination mit einem weiteren therapeutischen Agens verabreicht wird, beispielsweise einem Anti-Epileptikum, einem Stimmungsstabilisator, einem zentralnervösen Stimulans, einem Antihypertensivum, Folsäure, einem ausgewählten Serotonin-Wiederaufnahmehemmer oder einem Antipsychotikum.

11. Verfahren des Durchmusterns auf eine Kandidatensubstanz, die zum Behandeln einer neurologischen Entwicklungsstörung, einschließlich des Fragilen-X-Syndroms, geistiger Retardierung, Störungen aus dem Autismusspektrum, vorzeitiger Ovarialinsuffizienz oder des Fragiles-X-assoziierten Tremor-/Ataxiesyndroms, nützlich ist, umfassend die Schritte:
Vorsehen einer p21-aktivierten Kinase (PAK);
Vorsehen eines natürlichen Bindungspartners von PAK oder eines Phosphorylierungssubstrates von PAK;
Verabreichen mindestens einer Kandidatensubstanz an PAK und den natürlichen Bindungspartner oder das Phosphorylierungssubstrat und
Messen der Auswirkung der mindestens einen Kandidatensubstanz auf die Bindungswechselwirkung,
Messen der Fähigkeit der mindestens einen Kandidatensubstanz, mit der Bindungswechselwirkung zwischen PAK und dem natürlichen Bindungspartner zu konkurrieren, oder Messen der Auswirkung der mindestens einen Kandidatensubstanz auf die Fähigkeit von PAK, das Substrat zu phosphorylieren, und dadurch Identifizieren einer Substanz, die PAK hemmt und zum Behandeln einer neurologischen Entwicklungsstörung, einschließlich des Fragilen-X-Syndroms, geistiger Retardierung, Störungen aus dem Autismusspektrum, vorzeitiger Ovarialinsuffizienz oder des Fragiles-X-assoziierten Tremor-/Ataxiesyndroms, nützlich ist.

12. Verwendung mindestens eines Inhibitors der p21-aktivierten Kinase (PAK) bei der Herstellung eines Medikaments zum Behandeln einer neurologischen Entwicklungsstörung.

13. Verwendung nach Anspruch 12, wobei die neurologische Entwicklungsstörung aus Fragilem-X-Syndrom, geistiger Retardierung, Störungen aus dem Autismusspektrum, vorzeitiger Ovarialinsuffizienz oder Fragiles-X-assoziiertem Tremor-/Ataxiesyndrom ausgewählt ist.

## Revendications

1. Au moins un inhibiteur de la kinase activée par p21 (PAK) pour une utilisation dans le traitement d'un trouble neurodévelopemental.

2. Inhibiteur pour l'utilisation selon la revendication 1, dans lequel le trouble neurodévelopemental est choisi parmi le syndrome du X fragile, le retard mental, les troubles du spectre autistique, l'insuffisance ovarienne précoce ou les tremblements ataxiques associés au X fragile.

3. Inhibiteur pour l'utilisation selon la revendication 2, dans lequel le syndrome du X fragile est associé à une réduction de la potentialisation à long terme (PLT) dans le cortex ou à l'amélioration de la dépression à long terme (DLT).

4. Inhibiteur pour l'utilisation selon la revendication 3, dans lequel l'administration de l'inhibiteur à un sujet produit une diminution de la dépression à long terme (DLT) dans l'hippocampe ou une augmentation de la potentialisation à long terme (PLT) dans le cortex.

5. Inhibiteur pour l'utilisation selon la revendication 1 ou la revendication 2, dans lequel l'inhibiteur est administré en dose orale.

6. Inhibiteur pour l'utilisation selon la revendication 1 ou la revendication 2, dans lequel l'inhibiteur de la PAK inhibe l'interaction de la PAK avec la FMRP.

7. Inhibiteur pour l'utilisation selon la revendication 1 ou la revendication 2, dans lequel l'inhibiteur de la PAK est l'émodine, OSU-03012, la staurosporine, PD098059, la génistérine V, la tyrphostine B42, HA 1077, K252a, I(5-isoquinoléine-sulfonyl)-2-méthylpipérazine (H-7), CEP-1347, hPIP, la merline, la nischarine, P35/CDK5, CDC2, p11OC, POPX1, POPX2, CRIPak, les acides aminés 89 à 143 de la PAK1, GL-2003, SUI 1652 ou le purvalanol A.

8. Inhibiteur pour l'utilisation selon la revendication 1 ou la revendication 2, dans lequel l'inhibiteur de la PAK est un petit ARN interférent (siARN) ou un petit ARN en épingle à cheveux (ARNsh).

9. Inhibiteur pour l'utilisation selon la revendication 1 ou la revendication 2, dans lequel l'inhibiteur de la PAK est un anticorps.

10. Inhibiteur pour l'utilisation selon la revendication 1 ou la revendication 2, dans lequel l'inhibiteur de la PAK est administré en combinaison avec un agent thérapeutique supplémentaire, par exemple un agent anticonvulsivant, un régulateur de l'humeur, un stimulant du système nerveux central, un agent antihypertenseur, l'acide folique, un inhibiteur de recaptage de la sérotonine choisi, ou un agent antipsychotique.

11. Procédé de criblage d'une substance candidate utile pour le traitement d'un trouble neurodévelopemental comprenant le syndrome du X fragile, le retard mental, les troubles du spectre autistique, l'insuffisance ovarienne précoce ou les tremblements ataxiques associés au X fragile, comprenant les étapes consistant à :
fournir une kinase activée par p21 (PAK) ;
fournir un partenaire de liaison naturel de la PAK ou un substrat de phosphorylation de la PAK ;
administrer au moins une substance candidate à la PAK et le partenaire de liaison naturel ou le substrat de phosphorylation ; et
mesurer l'effet de la au moins une substance candidate sur l'interaction de liaison,
mesurer la capacité de la au moins une substance candidate pour rivaliser avec l'interaction de liaison entre la PAK et le partenaire de liaison naturel ou mesurer l'effet de la au moins une substance candidate sur la capacité de la PAK à phosphoryler le substrat, et identifier ainsi une substance qui inhibe la PAK et est utile pour le traitement d'un trouble neurodévelopemental comprenant le syndrome du X fragile, le retard mental, les troubles du spectre autistique, l'insuffisance ovarienne précoce ou les tremblements ataxiques associés au X fragile.

12. Utilisation d'au moins un inhibiteur de la kinase activée par p21 (PAK) dans la fabrication d'un médicament destiné au traitement d'un trouble neurodévelopemental.

13. Utilisation selon la revendication 12, dans laquelle le trouble neurodévelopemental est choisi parmi le syndrome du X fragile, le retard mental, les troubles du spectre autistique, l'insuffisance ovarienne précoce ou les tremblements ataxiques associés au X fragile.
